(19) Europäisches Patentamt / European Patent Office / Office européen des brevets

(11) **EP 4 051 247 B1**

(12) **EUROPEAN PATENT SPECIFICATION**

(45) Date of publication and mention
of the grant of the patent:
**01.11.2023 Bulletin 2023/44**

(21) Application number: **20800265.9**

(22) Date of filing: **22.10.2020**

(51) International Patent Classification (IPC):
*A61K 9/70* [(2006.01)]   *A61K 31/485* [(2006.01)]
*A61K 47/10* [(2017.01)]   *A61P 25/32* [(2006.01)]
*A61P 25/36* [(2006.01)]   *A61P 1/00* [(2006.01)]
*A61P 9/00* [(2006.01)]   *A61P 11/00* [(2006.01)]
*A61P 17/02* [(2006.01)]   *A61P 17/00* [(2006.01)]
*A61P 17/04* [(2006.01)]   *A61P 17/06* [(2006.01)]
*A61P 19/02* [(2006.01)]   *A61P 25/00* [(2006.01)]
*A61P 25/24* [(2006.01)]   *A61P 31/12* [(2006.01)]
*A61P 35/00* [(2006.01)]   *A61P 37/02* [(2006.01)]

(52) Cooperative Patent Classification (CPC):
**A61K 9/7084; A61K 31/485; A61K 47/10;**
**A61P 25/32; A61P 25/36;** A61P 1/00; A61P 9/00;
A61P 11/00; A61P 17/00; A61P 17/02; A61P 17/04;
A61P 17/06; A61P 19/02; A61P 25/00; A61P 25/24;

(Cont.)

(86) International application number:
**PCT/GB2020/052673**

(87) International publication number:
**WO 2021/084229 (06.05.2021 Gazette 2021/18)**

(54) **TRANSDERMAL PATCH**

TRANSDERMALES PFLASTER

TIMBRE TRANSDERMIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.10.2019 GB 201915627**

(43) Date of publication of application:
**07.09.2022 Bulletin 2022/36**

(73) Proprietor: **University of Sunderland**
**Sunderland, Tyne & Wear SR1 3SD (GB)**

(72) Inventors:
 • **DODOU, Kalliopi**
 **Sunderland Tyne and Wear SR1 3SD (GB)**
 • **DOOLEY, Philippa**
 **Sunderland Tyne Wear SR1 3SD (GB)**

(74) Representative: **HGF**
**HGF Limited**
**1 City Walk**
**Leeds LS11 9DX (GB)**

(56) References cited:
**WO-A2-2008/115371      WO-A2-2009/106831**

• **RACHEL SHET HUI WONG ET AL: "Effect of Drug
Loading Method and Drug Physicochemical
Properties on the Material and Drug Release
Properties of Poly (Ethylene Oxide) Hydrogels for
Transdermal Delivery", POLYMERS, vol. 9, no. 12,
19 July 2017 (2017-07-19), page 286, XP55765821,
CH ISSN: 2073-4360, DOI: 10.3390/polym9070286**

• **RACHEL WONG ET AL: "Effect of Crosslinking Agent Concentration on the Properties of Unmedicated Hydrogels", PHARMACEUTICS, vol. 7, no. 3, 9 September 2015 (2015-09-09), pages 305-319, XP055757623, DOI: 10.3390/pharmaceutics7030305**

(52) Cooperative Patent Classification (CPC): (Cont.)
A61P 31/12; A61P 35/00; A61P 37/02

## Description

This invention relates to a transdermal patch, and more particularly a transdermal patch wherein the patch comprises a cross-linked poly(ethylene oxide) hydrogel loaded with naltrexone. The invention also relates to methods for preparing naltrexone-loaded cross-linked poly(ethylene oxide) hydrogels. Also provided are transdermal patches for use as a medicament, for example in the treatment of a specified condition.

## BACKGROUND

Naltrexone is typically prescribed for opioid dependence or alcohol dependence, as it is a strong opioid antagonist. Doses of naltrexone used in such treatments are typically in the range of 50 to 100 mg per day, with activity believed to be due to opioid receptor antagonism. Low-dose naltrexone (LDN), typically comprising doses of 5 mg or less per day, may be useful in treating conditions such as fibromyalgia, multiple sclerosis, Crohn's disease, cancer, Hailey-Hailey disease, complex-regional pain syndrome (Toljan and Vrooman, Low-Dose Naltrexone (LDN) - Review of Therapeutic Utilization, Med. Sci., 2018, 6, 82).

LDN is usually provided as oral dosage form. Naltrexone has relatively poor oral bioavailability, so dosage forms for parenteral administration have been investigated. For example, several naltrexone creams for topical delivery have been reported, including 1% w/w naltrexone cream formulated for the pruritus treatment using excipients including water, paraffin, myristyl alcohol, cetyl alcohol, glyceryl monostearate, polysorbate 20, citric acid, methyl paraben, propyl paraben and hexamidine diisetionate (Bigliardi et al., 2007). Dodou et al (2015) also investigated ex vivo studies for the passive transdermal delivery of naltrexone from a cream having 1.1% w/w drug loading and containing the excipients: water, isopropyl myristate (IPM), sorbitan monooleate, polysorbate 80, and propylene glycol. The results showed good permeation *via* pig skin. In another study, 0.03% w/w naltrexone cream formulated for the treatment of diabetic wounds was made by mixing naltrexone with Neutrogena moisturising cream (McLaughlin et al., 2017). These formulations showed potential to improve the conditions which they were trialled for, however the conditions were for a topical purpose, therefore systemic treatment was not achieved.

Paudel *et al.* investigated the topical application of naltrexone using human skin and guinea pig skin models. While this demonstrated transdermal delivery of naltrexone, it would be desirable to obtain a higher level of dosing. Delivery of larger quantities of drugs such as naltrexone may be provided by active methods, such as microneedles or iontophoresis. There are a number of examples in the art of naltrexone delivery using microneedles (Ghosh et al, 2013; Milewski et al, 2012; Pillai et al., 2004) and using iontophoresis (Cordery al, 2019). However, both of these methods have their complications. With microneedles, there is a possibility that a patient may apply a microneedle patch incorrectly, which may lead to incomplete drug delivery. With iontophoresis, the delivery time appears to be much shorter than required, suggesting this method is more appropriate for immediate release rather than sustained release. Additionally, this method of drug delivery is not the most universal, as differences in skin characteristic can have a significant effect on the efficiency of drug delivery. There is accordingly a need for improved methods for delivering naltrexone.

An object of the invention is to provide dosage forms for delivery of LDN.

## BRIEF SUMMARY OF THE DISCLOSURE

In accordance with a first aspect of the present invention there is provided a transdermal patch comprising: a cross-linked poly(ethylene oxide) hydrogel and an occlusive adhesive tape, wherein the hydrogel further comprises naltrexone, or a pharmaceutically acceptable salt or solvate thereof and wherein the poly(ethylene oxide) hydrogel has a crosslink density of at least $4.5 \times 10^{-4}$ mol cm$^{-3}$ and not more than $16 \times 10^{-4}$ mol cm$^{-3}$.

A second aspect of the invention provides a method for preparing a cross-linked poly(ethylene oxide) hydrogel. The method comprises:

(a) mixing an aqueous solution comprising poly(ethylene oxide) and naltrexone or pharmaceutically acceptable salt or solvate thereof;

(b) adding a cross-linking agent to the mixture;

(c) drying the mixture to form a xerogel;

(d) irradiating the xerogel under UV radiation to create cross-linking; and

(e) swelling of the irradiated cross-linked xerogel to provide the cross-linked hydrogel.

In accordance with a third aspect of the invention, there is provided a transdermal patch obtainable or obtained by a method of the present invention.

In accordance with a fourth aspect of the invention, there is provided a transdermal patch of the present invention for use as a medicament. The transdermal patch for use in said treatment typically comprises an effective amount of naltrexone, or a pharmaceutically acceptable salt or solvate thereof.

In accordance with a fifth aspect of the invention, there is provided a transdermal patch of the present invention for use in the treatment of a condition. The condition may be selected from: opioid dependency, alcohol dependency, Crohn's disease/ulcerative colitis, chronic fatigue syndrome/myalgic encephalomyelitis, autism, pruritus, diabetic wounds, HIV/AIDS, fibromyalgia, multiple sclerosis, inflammatory bowel disease, Crohn's disease/ulcerative colitis, complex regional pain syndrome, Hailey-Hailey disease, psoriasis, Ehlers-Danlos syndrome, cancer, Gulf War illness, depression, chronic arthritis, and autoimmune disorder. The transdermal patch for use in said treatment typically comprises an effective amount of naltrexone, or a pharmaceutically acceptable salt or solvate thereof.

## BRIEF DESCRIPTION OF THE DRAWINGS

Embodiments of the invention are further described hereinafter with reference to the accompanying drawings, in which:

Figure 1 is a schematic, illustrating in cross-section the construction of an exemplary transdermal patch. The patch comprises layers including an occlusive adhesive tape 1, hydrogel 5, permeable membrane 10 and release liner 15.

Figure 2 provides Fourier transform infra-red (FTIR) spectra of (a) naltrexone base loaded hydrogel films and (b) naltrexone hydrochloride salt loaded films.

Figure 3 provides a calibration curve illustrating the effect of the level of loading on the enthalpy of fusion of naltrexone hydrochloride.

Figure 4 shows the permeation profile of naltrexone base from film swollen in water, via human skin.

Figure 5 shows the permeation profile of naltrexone base from film swollen in 25% v/v propylene glycol aqueous solution, via human skin.

Figure 6 shows the permeation profile of naltrexone hydrochloride from film swollen in water, via human skin.

Figure 7 shows the permeation profile of naltrexone hydrochloride from film swollen in 25% v/v propylene glycol aqueous solution, via human skin.

Figure 8 provides FTIR spectra of xerogels formed by drying hydrogel films from patches stored under ambient conditions, for the following patches: a) NTX base water, b) NTX HCl water, c) NTX base PG and d) NTX HCl PG.

Figure 9 provides FTIR spectra of xerogels formed by drying hydrogel films from patches stored under accelerated conditions, for the following patches: a) NTX base water, b) NTX HCl water, c) NTX base PG and d) NTX HCl PG.

Figure 10 provides FTIR spectra of CoTran membrane from patches stored under ambient conditions, for patches comprising the following hydrogel films: a) NTX base water, b) NTX HCl water, c) NTX base PG and d) NTX HCl PG.

Figure 11 provides FTIR spectra of CoTran membrane from patches stored under accelerated conditions, for patches comprising the following hydrogel films: a) NTX base water, b) NTX HCl water, c) NTX base PG and d) NTX HCl PG.

Figure 12 provides FTIR spectra of the adhesive side of Blenderm medical tape from patches stored under ambient conditions, for patches comprising the following hydrogel films: a) NTX base water, b) NTX HCl water, c) NTX base PG and d) NTX HCl PG.

Figure 13 provides FTIR spectra of the adhesive side of Blenderm medical tape from patches stored under accelerated conditions, for patches comprising the following hydrogel films: a) NTX base water, b) NTX HCl water, c) NTX base PG and d) NTX HCl PG.

Figure 14 provides FTIR spectra of the Scotchpak release liner from patches stored under ambient conditions, for patches comprising the following hydrogel films: a) NTX base water, b) NTX HCl water, c) NTX base PG and d) NTX HCl PG.

Figure 15 provides FTIR spectra of the Scotchpak release liner from patches stored under accelerated conditions, for patches comprising the following hydrogel films: a) NTX base water, b) NTX HCl water, c) NTX base PG and d) NTX HCl PG.

## DETAILED DESCRIPTION

The abbreviations used herein have their conventional meaning within the chemical and biological arts.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification.

For the avoidance of doubt, it is hereby stated that the information disclosed earlier in this specification under the heading "Background" is relevant to the invention and is to be read as part of the disclosure of the invention.

In case of conflict with publications, patent applications, patents, and other references mentioned herein, the present specification, including definitions, will control.

DEFINITIONS

The following explanations of terms and methods are provided to better describe the present disclosure and to guide those of ordinary skill in the art in the practice of the present disclosure.

The term "hydrogel" refers to a hydrophilic cross-linked polymer (e.g. cross-linked poly(ethylene oxide)) comprising water. A hydrogel may comprise at least about 20% w/w water. For example, a hydrogel may comprise at least about 30% w/w water, at least 50% w/w water or at least 70% w/w water. The hydrogels of the present disclosure also comprise naltrexone and optionally other substances, such as propylene glycol. In the context of the present disclosure, a hydrogel may also be referred to as a film, as hydrogels of the disclosure may be provided in the form of a relatively thin film, e.g. a film having a thickness of not more than 2 mm, e.g. a thickness of not more than 1 mm. A hydrogel film may have a thickness of from 0.1 to 1 mm.

The term "xerogel" refers to an open network formed by removal of all swelling agents (such as water) from a gel. A hydrogel of the present disclosure may be converted to a xerogel by drying. Similarly, a xerogel of the present disclosure may be converted to a hydrogel by swelling the xerogel in an aqueous solvent system (e.g. water, optionally comprising propylene glycol).

The term "pharmaceutically acceptable salts" is meant to include salts of the active compounds which are prepared with relatively nontoxic acids or bases, depending on the particular substituents found on the compounds described herein. When compounds contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge et al., "Pharmaceutical Salts", Journal of Pharmaceutical Science, 1977, 66, 1-19). Certain specific compounds of the present invention contain both basic and acidic functionalities that allow the compounds to be converted into either base or acid addition salts.

Thus, the compounds of the present disclosure may exist as salts with pharmaceutically acceptable acids. The present invention includes such salts. Examples of such salts include hydrochlorides, hydrobromides, sulfates, methanesulfonates, nitrates, maleates, acetates, citrates, fumarates, tartrates (e.g. (+)-tartrates, (-)-tartrates or mixtures thereof including racemic mixtures, succinates, benzoates and salts with amino acids such as glutamic acid. These salts may be prepared by methods known to those skilled in the art.

The neutral forms of the compounds are preferably regenerated by contacting the salt with a base or acid and isolating the parent compound in the conventional manner. The parent form of the compound differs from the various salt forms in certain physical properties, such as solubility in polar solvents.

Certain compounds used in compositions of the present invention can exist in unsolvated forms as well as solvated forms, including hydrated forms. In general, the solvated forms are equivalent to unsolvated forms and are encompassed within the scope of the present invention. Certain compounds of the present invention may exist in multiple crystalline or amorphous forms. In general, all physical forms are equivalent for the uses contemplated by the present invention and are intended to be within the scope of the present invention.

Certain compounds used in a patch of the present invention possess asymmetric carbon atoms (optical centers) or double bonds; the racemates, diastereomers, tautomers, geometric isomers and individual isomers are encompassed within the scope of the present invention and disclosure. The compounds do not include those which are known in the art to be too unstable to synthesize and/or isolate.

The compounds used in the patches of the present invention may also contain unnatural proportions of atomic isotopes at one or more of the atoms that constitute such compounds. For example, the compounds may be radiolabeled with radioactive isotopes, such as for example tritium ($^3$H), iodine-125 ($^{125}$I) or carbon-14 ($^{14}$C). All isotopic variations of the compounds, whether radioactive or not, are encompassed within the scope of the disclosure.

The term "naltrexone" as used herein refers to the compound 17-(Cyclopropylmethyl)-4,5α-epoxy-3,14-dihydroxymorphinan-6-one. Naltrexone may also be provided in the form of a pharmaceutically acceptable salt, such as naltrexone HCl, or a solvate thereof.

The term "low dose naltrexone" ("LDN") refers to a dosage of not more than about 5 mg per day of naltrexone, or a pharmaceutically acceptable salt or solvate thereof. In the context of a therapy, LDN may refer to a dosage of not more than about 5 mg/day of naltrexone, or a pharmaceutically acceptable salt or solvate thereof. For example, an LDN therapy may comprise a dose of about 0.05 to about 3 mg / day (e.g. about 0.075 to about 2.7 mg / day). Patches may be sized appropriately to provide LDN. As will be appreciated, patches disclosed herein may provide a controlled release dosage form, providing LDN therapy over 2, 3, 4, 5, 6 or more days. For example, the patches may provide LDN therapy for at least 2 or 3 days.

The phrase "effective amount" refers to an amount sufficient to attain the desired result. The phrase "therapeutically effective amount" means an amount sufficient to produce the desired therapeutic result. Generally, the therapeutic result is an objective or subjective improvement of a disease or condition, achieved by inducing or enhancing a physiological process, blocking or inhibiting a physiological process, or in general terms performing a biological function that helps in or contributes to the elimination or abatement of the disease or condition.

The invention concerns amongst other things the treatment of a disease. The term "treatment", and the therapies encompassed by this invention, include the following and combinations thereof: (1) hindering, e.g. delaying initiation and/or progression of, an event, state, disorder or condition, for example arresting, reducing or delaying the development of the event, state, disorder or condition, or a relapse thereof in case of maintenance treatment or secondary prophylaxis, or of at least one clinical or subclinical symptom thereof; (2) preventing or delaying the appearance of clinical symptoms of an event, state, disorder or condition developing in an animal (e.g. human) that may be afflicted with or predisposed to the state, disorder or condition but does not yet experience or display clinical or subclinical symptoms of the state, disorder or condition; and/or (3) relieving and/or curing an event, state, disorder or condition (*e.g.,* causing regression of the event, state, disorder or condition or at least one of its clinical or subclinical symptoms, curing a patient or putting a patient into remission). The benefit to a patient to be treated may be either statistically significant or at least perceptible to the patient or to the physician. It will be understood that a medicament will not necessarily produce a clinical effect in each patient to whom it is administered; thus, in any individual patient or even in a particular patient population, a treatment may fail or be successful only in part, and the meanings of the terms "treatment" and "prophylaxis" and of cognate terms are to be understood accordingly. The compositions and methods described herein are of use for therapy and/or prophylaxis of the mentioned conditions.

The term "prophylaxis" includes reference to treatment therapies for the purpose of preserving health or inhibiting or delaying the initiation and/or progression of an event, state, disorder or condition, for example for the purpose of reducing the chance of an event, state, disorder or condition occurring. The outcome of the prophylaxis may be, for example, preservation of health or delaying the initiation and/or progression of an event, state, disorder or condition. It will be recalled that, in any individual patient or even in a particular patient population, a treatment may fail, and this paragraph is to be understood accordingly.

## PATCHES

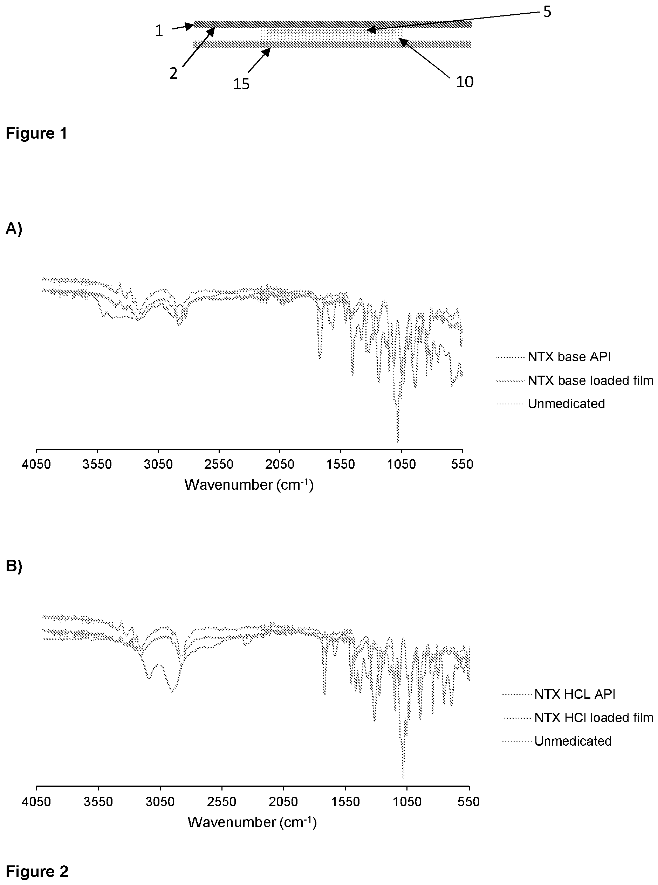

Patches of the invention are configured for transdermal administration of naltrexone, or a pharmaceutically acceptable salt or solvate thereof. An exemplary patch structure is illustrated in the cross-section provided by figure 1. The patch comprises layers including an occlusive adhesive tape 1, hydrogel 5, permeable membrane 10 and release liner 15. When the patch is to be placed on a subject, the release liner 15 is removed and the exposed portions of the adhesive surface 2 of the tape 1 may be adhered to the epidermis of the subject, providing an occlusive seal between the hydrogel 5 and epidermis of the subject. The permeable membrane 10 is useful, because it retains the hydrogel 5 in its intended position, both during storage and when the patch is placed on the subject. The release liner 15 provides advantages, as it ensures that the hydrogel 5 is retained in its intended position during storage and it also prevents or reduces release of reagents (such as naltrexone or solvent) from the hydrogel 5 prior to removal of the release liner 15. It should be noted that, while the permeable membrane 10 and release liner 15 may provide benefits, they represent optional features that are not required in all patches of the invention.

The occlusive adhesive tape 1 may be an impermeable plastic medical tape with an adhesive layer on one side. Examples of suitable tapes include occlusive plastic tapes such as 3M Blenderm™, 3M Scotchpak™, 3M CoTran™, Leukoflex® and Leukoplast® Sleek®. The hydrogel 5 typically comprises a film comprising at least: crosslinked polymer; naltrexone, or a salt or solvate thereof; and solvent. The hydrogel may also comprise other components, such as propylene glycol and excipients. The crosslinked polymer forms a matrix for the hydrogel. The hydrogel 5 has a smaller length and width than the tape 1, thereby providing exposed portions of the adhesive surface when the release liner is removed. The permeable membrane 10 typically has a length and with that is greater than the length and width of the hydrogel 5, but smaller than the length and width of the occlusive adhesive tape 1. This allows hydrogel 5 to be sandwiched between the occlusive adhesive tape 1 and permeable membrane 10, as illustrated in figure 1. This arrangement retains the hydrogel 5 in a relatively fixed position relative to the other parts of the patch. The permeable membrane may be fairly open, e.g. a gauze, or may be selected to have a lower level of permeability and thus modulate the rate at which naltrexone, or a salt or solvate thereof, is able to diffuse out of the patch. The release liner 15 may be sized to have approximately the same length and width as the occlusive adhesive tape 1, such that it covers the adhesive layer when assembled to the patch, as illustrated in figure 1.

An aspect of the invention provides a transdermal patch comprising: a cross-linked poly(ethylene oxide) hydrogel and an occlusive adhesive tape, wherein the hydrogel further comprises naltrexone, or a pharmaceutically acceptable salt or solvate thereof and wherein the poly(ethylene oxide) hydrogel has a crosslink density of at least $4.5 \times 10^{-4}$ mol cm$^{-3}$ and not more than $10 \times 10^{-4}$ mol cm$^{-3}$.

The hydrogel may have a crosslink density of at least $5 \times 10^{-4}$ mol cm$^{-3}$, e.g. of at least $5 \times 10^{-4}$ mol cm$^{-3}$. In embodiments, the hydrogel may have a crosslink density of not more than $8 \times 10^{-4}$ mol cm$^{-3}$, e.g. of not more than $7 \times 10^{-4}$ mol cm$^{-3}$. In embodiments, the hydrogel may have a crosslink density of at least $5 \times 10^{-4}$ mol cm$^{-3}$ and not more than $7 \times 10^{-4}$ mol cm$^{-3}$. Without wishing to be bound by any theory, it is believed that in the patches of the invention, the specified levels of crosslinking, as measured by crosslink density, provide a good level of mechanical performance (e.g. tensile strength) and dosing performance.

The hydrogel may comprise propylene glycol in an amount of about 15% to about 20% w/w of the hydrogel. The hydrogel may comprise propylene glycol solution in an amount of about 17% to about 19% w/w of the hydrogel. The presence of propylene glycol in the hydrogel may be advantageous, as it may act as a permeation enhancer, when the patch is applied to skin. Without wishing to be bound by any theory, propylene glycol may act as a permeation enhancer because it has a lower molecular weight than naltrexone, allowing propylene glycol to diffuse out of the hydrogel faster than drug, disrupting the stratum corneum and thus enabling the drug to penetrate the skin more easily (Williams and Barry, 2004).

Higher molecular weight poly(ethylene oxide) (PEO) (e.g. with viscosity average molecular weight of greater than 500,000) are desirable to form solid gels due to their higher viscosity (Clements, 1963) compared to PEO of lower molecular weights which tend to form viscous liquids. Without wishing to be bound by theory, it is thought that the semi-solid nature of the hydrogel films is highly desirable to achieve the mechanical strength required for skin application.

The poly(ethylene oxide) may have a viscosity average molecular weight (Mv) of at least about 600,000 g/mol and not more than about 8,000,000 g/mol. The poly(ethylene oxide) may have a viscosity average molecular weight of at least about 600,000 g/mol and not more than about 6,000,000 g/mol. The poly(ethylene oxide) may have a viscosity average molecular weight of at least about 600,000 g/mol and not more than about 5,000,000 g/mol. The poly(ethylene oxide) may have a viscosity average molecular weight of at least about 600,000 g/mol and not more than about 4,000,000 g/mol. The poly(ethylene oxide) may have a viscosity average molecular weight of at least about 600,000 g/mol and not more than about 3,000,000 g/mol. The poly(ethylene oxide) may have a viscosity average molecular weight of at least about 600,000 g/mol and not more than about 2,500,000 g/mol.

The poly(ethylene oxide) may have a viscosity average molecular weight (Mv) of at least about 700,000 g/mol and not more than about 6,000,000 g/mol. The poly(ethylene oxide) may have a viscosity average molecular weight of at least about 700,000 g/mol and not more than about 5,000,000 g/mol. The poly(ethylene oxide) may have a viscosity average molecular weight of at least about 700,000 g/mol and not more than about 4,000,000 g/mol. The poly(ethylene oxide) may have a viscosity average molecular weight of at least about 700,000 g/mol and not more than about 3,000,000 g/mol. The poly(ethylene oxide) may have a viscosity average molecular weight of at least about 700,000 g/mol and not more than about 2,500,000 g/mol. The polyethylene oxide) may have a viscosity average molecular weight of at least about 700,000 g/mol and not more than about 2,000,000 g/mol. The poly(ethylene oxide) may have a viscosity average molecular weight of at least about 700,000 g/mol and not more than about 1,500,000 g/mol. The poly(ethylene oxide) may have a viscosity average molecular weight of at least about 700,000 g/mol and not more than about 1,400,000 g/mol. The poly(ethylene oxide) may have a viscosity average molecular weight of at least about 700,000 g/mol and not more than about 1,300,000 g/mol. The poly(ethylene oxide) may have a viscosity average molecular weight of at least about 700,000 g/mol and not more than about 1,200,000 g/mol. The poly(ethylene oxide) may have a viscosity average molecular weight of at least about 700,000 g/mol and not more than about 1,100,000 g/mol. The poly(ethylene oxide) may have a viscosity average molecular weight of at least about 700,000 g/mol and not more than about 1,000,000 g/mol.

The poly(ethylene oxide) may have a viscosity average molecular weight of at least about 800,000 g/mol and not more than about 6,000,000 g/mol. The poly(ethylene oxide) may have a viscosity average molecular weight of at least about 800,000 g/mol and not more than about 5,000,000 g/mol. The poly(ethylene oxide) may have a viscosity average molecular weight of at least about 800,000 g/mol and not more than about 4,000,000 g/mol. The poly(ethylene oxide) may have a viscosity average molecular weight of at least about 800,000 g/mol and not more than about 3,000,000 g/mol. The poly(ethylene oxide) may have a viscosity average molecular weight of at least about 800,000 g/mol and not more than about 2,500,000 g/mol. The poly(ethylene oxide) may have a viscosity average molecular weight of at least about 800,000 g/mol and not more than about 2,000,000 g/mol. The poly(ethylene oxide) may have a viscosity average molecular weight of at least about 800,000 g/mol and not more than about 1,500,000 g/mol. The poly(ethylene oxide) may have a viscosity average molecular weight of at least about 800,000 g/mol and not more than about 1,400,000 g/mol. The poly(ethylene oxide) may have a viscosity average molecular weight of at least about 800,000 g/mol and not more than about 1,300,000 g/mol. The poly(ethylene oxide) may have a viscosity average molecular weight of at least about 800,000 g/mol and not more than about 1,200,000 g/mol. The poly(ethylene oxide) may have a viscosity average molecular weight of at least about 800,000 g/mol and not more than about 1,100,000 g/mol. The poly(ethylene oxide) may have a viscosity average molecular weight of at least about 800,000 g/mol and not more than about 1,000,000 g/mol.

The poly(ethylene oxide) may have a viscosity average molecular weight of at least about 900,000 g/mol and not more than about 6,000,000 g/mol. The poly(ethylene oxide) may have a viscosity average molecular weight of at least about 900,000 g/mol and not more than about 5,000,000 g/mol. The poly(ethylene oxide) may have a viscosity average molecular weight of at least about 900,000 g/mol and not more than about 4,000,000 g/mol. The poly(ethylene oxide) may have a viscosity average molecular weight of at least about 900,000 g/mol and not more than about 3,000,000 g/mol. The poly(ethylene oxide) may have a viscosity average molecular weight of at least about 900,000 g/mol and not more than about 2,500,000 g/mol. The poly(ethylene oxide) may have a viscosity average molecular weight of at least about 900,000 g/mol and not more than about 2,000,000 g/mol. The poly(ethylene oxide) may have a viscosity average molecular weight of at least about 900,000 g/mol and not more than about 1,500,000 g/mol. The poly(ethylene oxide) may have a viscosity average molecular weight of at least about 900,000 g/mol and not more than about 1,400,000 g/mol. The poly(ethylene oxide) may have a viscosity average molecular weight of at least about 900,000 g/mol and not more than about 1,300,000 g/mol. The poly(ethylene oxide) may have a viscosity average molecular weight of at least about 900,000 g/mol and not more than about 1,200,000 g/mol. The poly(ethylene oxide) may have a viscosity average molecular weight of at least about 900,000 g/mol and not more than about 1,100,000 g/mol. The poly(ethylene oxide) may have a viscosity average molecular weight of at least about 900,000 g/mol and not more than about 1,000,000 g/mol.

The poly(ethylene oxide) may have a viscosity average molecular weight of at least about 950,000 g/mol and not more than about 6,000,000 g/mol. The poly(ethylene oxide) may have a viscosity average molecular weight of at least about 950,000 g/mol and not more than about 5,000,000 g/mol. The poly(ethylene oxide) may have a viscosity average molecular weight of at least about 950,000 g/mol and not more than about 4,000,000 g/mol. The poly(ethylene oxide) may have a viscosity average molecular weight of at least about 950,000 g/mol and not more than about 3,000,000 g/mol. The poly(ethylene oxide) may have a viscosity average molecular weight of at least about 950,000 g/mol and not more than about 2,500,000 g/mol. The poly(ethylene oxide) may have a viscosity average molecular weight of at least about 950,000 g/mol and not more than about 2,000,000 g/mol. The poly(ethylene oxide) may have a viscosity average molecular weight of at least about 950,000 g/mol and not more than about 1,500,000 g/mol. The polyethylene oxide) may have a viscosity average molecular weight of at least about 950,000 g/mol and not more than about 1,400,000 g/mol. The poly(ethylene oxide) may have a viscosity average molecular weight of at least about 950,000 g/mol and not more than about 1,300,000 g/mol. The poly(ethylene oxide) may have a viscosity average molecular weight of at least about 950,000 g/mol and not more than about 1,200,000 g/mol. The poly(ethylene oxide) may have a viscosity average molecular weight of at least about 950,000 g/mol and not more than about 1,100,000 g/mol. The poly(ethylene oxide) may have a viscosity average molecular weight of at least about 950,000 g/mol and not more than about 1,000,000 g/mol.

The poly(ethylene oxide) may have a viscosity average molecular weight of at least about 1,000,000 g/mol and not more than about 5,000,000 g/mol. Preferably, the poly(ethylene oxide) has a viscosity average molecular weight of about 1,000,000 g/mol.

The cross-linked poly(ethylene oxide) may be formed by reacting poly(ethylene oxide) with a cross-linking agent. The ratio of poly(ethylene oxide) to cross-linking agent in the hydrogel film may be from about 15:1 to about 25:1 w/w. The ratio of poly(ethylene oxide) to cross-linking agent in the hydrogel film may be from about 17:1 to about 23:1 w/w. The ratio of poly(ethylene oxide) to cross-linking agent in the hydrogel film may be from about 18:1 to about 22:1 w/w. The ratio of poly(ethylene oxide) to cross-linking agent in the hydrogel film may be about 20:1 w/w. Preferably, the ratio of poly(ethylene oxide) to cross-linking agent in the hydrogel film is close to the level required to fully cross-link the polymer. This typically provides a hydrogel film with good mechanical properties and minimises the presence of unreacted cross-linking agent in the cross-linked hydrogel.

Where the crosslinked poly(ethylene oxide) comprises poly(ethylene oxide) with a relatively high viscosity average molecular weight (e.g. at least about 2,000,000 g/mol), the ratio of poly(ethylene oxide) to cross-linking agent may be lower, for example from about 10:1 w/w to about 20:1 w/w (e.g. from about 15:1 w/w to about 20:1 w/w). Where the crosslinked poly(ethylene oxide) comprises poly(ethylene oxide) with a relatively low viscosity average molecular weight (e.g. not more than about 900,000 g/mol), the ratio of poly(ethylene oxide) to cross-linking agent may be higher, for example from about 18:1 to about 28:1 (e.g. from about 18:1 w/w to about 25:1 w/w).

The crosslinking agent may be an acrylate monomer, such as an acrylate monomer comprising at least two ethynyl groups (e.g. an acrylate monomer comprising at least two acrylate groups). The crosslinking agent may be a di-, tri- or tetra-acrylate monomer. The crosslinking agent may be selected from: pentaerythritol tetraacrylate, pentaerythritol triacrylate, ethylene glycol diacrylate, di(ethylene glycol) diacrylate, tetra(ethylene glycol) diacrylate, ethylene glycol dimethacrylate, di(ethylene glycol) dimethacrylate, and tri(ethylene glycol) dimethacrylate. The crosslinking agent may be pentaerythritol tetraacrylate (PETRA).

Unreacted crosslinking agents, e.g. acrylate monomers, can potentially leach out of the hydrogels, as the crosslinking agents are typically low molecular weight compounds with relatively high diffusion coefficients. Therefore, by diffusion into the skin, they may cause side effects such as inflammation and skin sensitization. Accordingly, it is advantageous to ensure that there is no or minimal unreacted crosslinking agent in the cross-linked hydrogel. The amount of the residual crosslinking agent may be determined using conventional methods, for example as described for the crosslinking agent PETRA in Wong et al., Journal of Pharmaceutical Analysis, Vol. 6 (5), 2016, 307-312.

The naltrexone, or pharmaceutically acceptable salt or solvate thereof, may be present in an amount of at least about 3% w/w of the hydrogel. The naltrexone, or pharmaceutically acceptable salt or solvate thereof, may be present in an amount of at least about 7% w/w of the hydrogel.

The naltrexone may be in the form of naltrexone base. The naltrexone base may be present in an amount of from about 7% to about 15% w/w of the hydrogel. The naltrexone base may be present in an amount of from about 7% to about 12% w/w of the hydrogel.

The naltrexone may be in the form of pharmaceutically acceptable naltrexone salt. The naltrexone may be a pharmaceutically acceptable acid addition naltrexone salt. The naltrexone salt may be naltrexone hydrochloride, naltrexone hydrobromide, naltrexone glycolate, naltrexone lactate, naltrexone acetate, naltrexone fumarate, naltrexone citrate, or naltrexone tartrate. The naltrexone salt may be naltrexone hydrochloride, naltrexone glycolate, or naltrexone lactate. The naltrexone salt may be naltrexone hydrochloride. The naltrexone salt may be present in an amount of from about 7% to about 30% w/w of the hydrogel, e.g. from about 7% to about 20% w/w of the hydrogel. The naltrexone salt may be present in an amount of from about 7% to about 12% w/w of the hydrogel. The naltrexone hydrochloride may be present in an amount of from about 7% to about 30% w/w of the hydrogel, e.g. from about 7% to about 20% w/w of the hydrogel. The naltrexone hydrochloride may be present in an amount of from about 7% to about 12% w/w of the hydrogel.

The hydrogel may have a tensile strength of at least 0.50 MPa (e.g. at least 0.6 MPa). The occlusive adhesive tape may be impermeable. The occlusive adhesive tape may comprise an outer layer that is an impermeable polymeric layer and an inner layer that is an adhesive layer. The inner layer may be in contact with the hydrogel. The impermeable polymeric layer may comprise any suitable polymer, such as polyester, polyethylene and/or polyurethane. The occlusive adhesive tape may be 3M Blenderm™, 3M Scotchpak™, 3M CoTran™, Leukoflex®, or Leukoplast® Sleek®.

The transdermal patch further comprises a permeable membrane. When the transdermal patch comprises a permeable membrane, the cross-linked poly(ethylene oxide) hydrogel may be sandwiched between the occlusive adhesive tape and the permeable membrane. When the transdermal patch comprises a permeable membrane, the cross-linked poly(ethylene oxide) hydrogel may be sandwiched between the adhesive layer of the occlusive adhesive tape and the permeable membrane. The permeable membrane may comprise an ethylene vinyl acetate membrane. The permeable membrane may comprise a 3M™ CoTran™ Ethylene Vinyl Acetate Membrane. The permeable membrane may comprise a gauze.

The patch may further comprise a release liner. The release liner may be coated with fluoropolymer and/or fluorosilicone. The release liner may be coated with fluoropolymer. The release liner may be coated with fluorosilicone. The release liner may be a 3M™ Scotchpak™ 1022 release liner, a 3M™ Scotchpak™ 9744 release liner, a 3M™ Scotchpak™ 9755 fluoropolymer coated release liner, or a 3M™ Scotchpak™ 9709 release liner fluorosilicone coated polyester film. The release liner may be a 3M™ Scotchpak™ 9755 fluoropolymer coated release liner. The release liner may be configured such that, when the transdermal patch comprises the attached release liner, none of the adhesive of the occlusive adhesive tape is exposed.

The length and breadth of the hydrogel may be smaller than the length and breadth of the occlusive adhesive tape. This may allow the transdermal patch to be applied to a surface (e.g. skin) in a manner that allows that occlusive tape to isolate the hydrogel from the atmosphere. The permeable membrane, when present, may have a length and breadth that is greater than the length and breadth of the hydrogel, but smaller than the length and breadth of the occlusive adhesive tape. This may allow the hydrogel to be sandwiched between the occlusive adhesive tape and the permeable membrane, such that adhesion occlusive tape and permeable membrane assists in retaining the relative position of the hydrogel in the patch.

The hydrogel of the patch may be considered to have two faces, one face against the occlusive adhesive patch and the opposed face suitable for application to a surface (e.g. skin). The length and breadth of the hydrogel may be selected such that the surface area of a face (such as the opposed face) of the patch is at least about 0.2 cm$^2$ and not more than about 25 cm$^2$. For example, the surface area of a face of the patch may be at least about 0.4 cm$^2$ and not more than about 20 cm$^2$. The surface area of a face of the patch may be at least about 0.2 cm$^2$, at least about 0.4 cm$^2$, at least about 1 cm$^2$, at least about 2 cm$^2$, or at least about 5 cm$^2$. The surface area of a face of the patch may be not more than about 20 cm$^2$, not more than about 18 cm$^2$, or not more than about 15 cm$^2$.

The hydrogel may have a thickness of at least about 150 $\mu$m, for example a thickness of at least about 200 $\mu$m. The hydrogel may have a thickness of at least about 250 $\mu$m. The hydrogel may have a thickness of not more than about 2000 $\mu$m, for example a thickness of not more than about 1500 $\mu$m. The hydrogel may have a thickness of not more than about 1000 $\mu$m. The hydrogel may have a thickness of not more than about 800 $\mu$m. The hydrogel may have a thickness of not more than about 600 $\mu$m. The hydrogel may have a thickness of not more than about 500 $\mu$m. The hydrogel may have a thickness of not more than about 400 $\mu$m.

The hydrogel may have a thickness of at least about 100 $\mu$m and not more than about 1500 $\mu$m. The hydrogel may have a thickness of at least about 100 $\mu$m and not more than about 1000 $\mu$m. The hydrogel may have a thickness of at least about 100 $\mu$m and not more than about 800 $\mu$m. The hydrogel may have a thickness of at least about 100 $\mu$m and not more than about 600 $\mu$m. The hydrogel may have a thickness of at least about 100 $\mu$m and not more than about 600 $\mu$m. The hydrogel may have a thickness of at least about 100 $\mu$m and not more than about 500 $\mu$m. The hydrogel may have a thickness of at least about 150 $\mu$m and not more than about 1000 $\mu$m. The hydrogel may have a thickness of at least about 150 $\mu$m and not more than about 800 $\mu$m. The hydrogel may have a thickness of at least about 150 $\mu$m and not more than about 600 $\mu$m. The hydrogel may have a thickness of at least about 150 $\mu$m and not more than about 500 $\mu$m. The hydrogel may have a thickness of at least about 200 $\mu$m and not more than about 800 $\mu$m. The hydrogel may have a thickness of at least about 200 $\mu$m and not more than about 600 $\mu$m. The hydrogel may have a thickness of at least about 200 $\mu$m and not more than about 500 $\mu$m. The hydrogel may have a thickness of at least about 200 $\mu$m and not more than about 400 $\mu$m. The hydrogel may have a thickness of at least about 250 $\mu$m and not more than about 800 $\mu$m. The hydrogel may have a thickness of at least about 250 $\mu$m and not more than about 600 $\mu$m. The hydrogel may have a thickness of at least about 250 $\mu$m and not more than about 500 $\mu$m. The hydrogel may have a thickness of at least about 250 $\mu$m and not more than about 400 $\mu$m. The hydrogel may have a thickness of at least about 300 $\mu$m and not more than about 800 $\mu$m. The hydrogel may have a thickness of at least about 300 $\mu$m and not more than about 600 $\mu$m. The hydrogel may have a thickness of at least about 300 $\mu$m and not more than about 500 $\mu$m. The hydrogel may have a thickness of at least about 300 $\mu$m and not more than about 400 $\mu$m.

The transdermal patch may be packaged in a sealed pouch. The sealed pouch may be sterile.

The transdermal patch may have a shelf-life of at least 6 months, e.g. of at least 9 months. For example, a transdermal patch in a sealed pouch may have a shelf-life of at least 6 months, e.g. of at least 9 months.

Another example of the disclosure (and not of the invention) provides a cross-linked poly(ethylene oxide) hydrogel comprising naltrexone, or a pharmaceutically acceptable salt or solvate thereof and wherein the poly(ethylene oxide) hydrogel has a crosslink density of at least $4.5 \times 10^{-4}$ mol cm$^{-3}$ and not more than $10 \times 10^{-4}$ mol cm$^{-3}$.

The hydrogel may have a crosslink density of at least $5 \times 10^{-4}$ mol cm$^{-3}$, e.g. of at least $5 \times 10^{-4}$ mol cm$^{-3}$. In embodiments, the hydrogel may have a crosslink density of not more than $8 \times 10^{-4}$ mol cm$^{-3}$, e.g. of not more than $7 \times 10^{-4}$ mol cm$^{-3}$. In embodiments, the hydrogel may have a crosslink density of at least $5 \times 10^{-4}$ mol cm$^{-3}$ and not more than $7 \times 10^{-4}$ mol cm$^{-3}$. Without wishing to be bound by any theory, it is believed that in the patches of the invention, the specified levels of crosslinking, as measured by crosslink density, provide a good level of mechanical performance (e.g. tensile strength) and dosing performance.

The hydrogel may comprise propylene glycol in an amount of about 15% to about 20% w/w of the hydrogel. The hydrogel may comprise propylene glycol solution in an amount of about 17% to about 19% w/w of the hydrogel. The presence of propylene glycol in the hydrogel may be advantageous, as it may act as a permeation enhancer, when the patch is applied to skin. Without wishing to be bound by any theory, propylene glycol may act as a permeation enhancer because it has a lower molecular weight than naltrexone, allowing propylene glycol to diffuse out of the hydrogel faster than drug, disrupting the stratum corneum and thus enabling the drug to penetrate the skin more easily (Williams and Barry, 2004).

The cross-linked polyethylene oxide) may be formed by reacting polyethylene oxide) with a cross-linking agent. The ratio of polyethylene oxide) to cross-linking agent in the hydrogel film may be from about 15:1 to about 25:1 w/w. The ratio of polyethylene oxide) to cross-linking agent in the hydrogel film may be from about 17:1 to about 23:1 w/w. The ratio of polyethylene oxide) to cross-linking agent in the hydrogel film may be from about 18:1 to about 22:1 w/w. The ratio of polyethylene oxide) to cross-linking agent in the hydrogel film may be about 20:1 w/w.

The crosslinking agent may be an acrylate monomer, such as an acrylate monomer comprising at least two ethynyl groups (e.g. an acrylate monomer comprising at least two acrylate groups). The crosslinking agent may be a di-, tri- or tetra-acrylate monomer. The crosslinking agent may be selected from: pentaerythritol tetraacrylate, pentaerythritol triacrylate, ethylene glycol diacrylate, di(ethylene glycol) diacrylate, tetra(ethylene glycol) diacrylate, ethylene glycol dimethacrylate, di(ethylene glycol) dimethacrylate, and tri(ethylene glycol) dimethacrylate. The crosslinking agent may be pentaerythritol tetraacrylate.

The naltrexone, or pharmaceutically acceptable salt or solvate thereof, may be present in an amount of at least about 3% w/w of the hydrogel. The naltrexone, or pharmaceutically acceptable salt or solvate thereof, may be present in an amount of at least about 7% w/w of the hydrogel.

The naltrexone may be in the form of naltrexone base. The naltrexone base may be present in an amount of from about 7% to about 15% w/w of the hydrogel. The naltrexone base may be present in an amount of from about 7% to about 12% w/w of the hydrogel.

The naltrexone may be in the form of pharmaceutically acceptable naltrexone salt. The naltrexone may be a pharmaceutically acceptable acid addition naltrexone salt. The naltrexone salt may be naltrexone hydrochloride, naltrexone hydrobromide, naltrexone glycolate, naltrexone lactate, naltrexone acetate, naltrexone fumarate, naltrexone citrate, or naltrexone tartrate. The naltrexone salt may be naltrexone hydrochloride, naltrexone glycolate, or naltrexone lactate. The naltrexone salt may be naltrexone hydrochloride. The naltrexone salt may be present in an amount of from about 7% to about 30% w/w of the hydrogel, e.g. from about 7% to about 20% w/w of the hydrogel. The naltrexone salt may be present in an amount of from about 7% to about 12% w/w of the hydrogel. The naltrexone hydrochloride may be present in an amount of from about 7% to about 30% w/w of the hydrogel, e.g. from about 7% to about 20% w/w of the hydrogel. The naltrexone hydrochloride may be present in an amount of from about 7% to about 12% w/w of the hydrogel.

The hydrogel may have a tensile strength of at least 0.50 MPa (e.g. at least 0.6 MPa). The occlusive adhesive tape may be impermeable. The occlusive adhesive tape may comprise an outer layer that is an impermeable polymeric layer and an inner layer that is an adhesive layer. The inner layer may be in contact with the hydrogel. The impermeable polymeric layer may comprise any suitable polymer, such as polyester, polyethylene and/or polyurethane. The occlusive adhesive tape may be 3M Blenderm™, 3M Scotchpak™, 3M CoTran™, Leukoflex®, or Leukoplast® Sleek®.

The length and breadth of the hydrogel may be selected such that the surface area of a face (such as the opposed face) of the patch is at least about 0.2 cm² and not more than about 25 cm². For example, the surface area of a face of the patch may be at least about 0.4 cm² and not more than about 20 cm². The surface area of a face of the patch may be at least about 0.2 cm², at least about 0.4 cm², at least about 1 cm², at least about 2 cm², or at least about 5 cm². The surface area of a face of the patch may be not more than about 20 cm², not more than about 18 cm², or not more than about 15 cm².

The hydrogel may have a thickness of at least about 150 μm, for example a thickness of at least about 200 μm. The hydrogel may have a thickness of at least about 250 μm. The hydrogel may have a thickness of not more than about 2000 μm, for example a thickness of not more than about 1500 μm. The hydrogel may have a thickness of not more than about 1000 μm. The hydrogel may have a thickness of not more than about 800 μm. The hydrogel may have a thickness of not more than about 600 μm. The hydrogel may have a thickness of not more than about 500 μm. The hydrogel may have a thickness of not more than about 400 μm.

The hydrogel may have a thickness of at least about 100 μm and not more than about 1500 μm. The hydrogel may have a thickness of at least about 100 μm and not more than about 1000 μm. The hydrogel may have a thickness of at least about 100 μm and not more than about 800 μm. The hydrogel may have a thickness of at least about 100 μm and not more than about 600 μm. The hydrogel may have a thickness of at least about 100 μm and not more than about 600 μm. The hydrogel may have a thickness of at least about 100 μm and not more than about 500 μm. The hydrogel may have a thickness of at least about 150 μm and not more than about 1000 μm. The hydrogel may have a thickness of at least about 150 μm and not more than about 800 μm. The hydrogel may have a thickness of at least about 150 μm and not more than about 600 μm. The hydrogel may have a thickness of at least about 150 μm and not more than about 500 μm. The hydrogel may have a thickness of at least about 200 μm and not more than about 800 μm. The hydrogel may have a thickness of at least about 200 μm and not more than about 600 μm. The hydrogel may have a thickness of at least about 200 μm and not more than about 500 μm. The hydrogel may have a thickness of at least about 200 μm and not more than about 400 μm. The hydrogel may have a thickness of at least about 250 μm and not more than about 800 μm. The hydrogel may have a thickness of at least about 250 μm and not more than about 600 μm. The hydrogel may have a thickness of at least about 250 μm and not more than about 500 μm. The hydrogel may have a thickness of at least about 250 μm and not more than about 400 μm. The hydrogel may have a thickness of at least about 300 μm and not more than about 800 μm. The hydrogel may have a thickness of at least about 300 μm and not more than about 600 μm. The hydrogel may have a thickness of at least about 300 μm and not more than about 500 μm. The hydrogel may have a thickness of at least about 300 μm and not more than about 400 μm.

## METHODS FOR PREPARING HYDROGELS AND PATCHES

Provides herein are methods of preparing a hydrogel comprising cross-linked poly(ethylene oxide) and naltrexone. The hydrogel can then be affixed to an occlusive adhesive tape, or sandwiched between an occlusive adhesive tape and permeable membrane, to provide a patch.

An aspect of the invention provides a method for preparing a cross-linked poly(ethylene oxide) hydrogel. The method comprises:

(a) mixing an aqueous solution comprising poly(ethylene oxide) and naltrexone or pharmaceutically acceptable salt or solvate thereof;
(b) adding a cross-linking agent to the mixture;
(c) drying the mixture to form a xerogel;
(d) irradiating the xerogel under UV radiation to create cross-linking; and
(e) swelling of the irradiated cross-linked xerogel to provide the cross-linked hydrogel.

Step (e) may be performed in aqueous solution comprising about 5% to about 30% v/v propylene glycol. Said aqueous solution may comprise about 10 to about 30% v/v propylene glycol. Said aqueous solution may comprise about 15 to about 30% v/v propylene glycol. Said aqueous solution may comprise about 20 to about 30% v/v propylene glycol. Said aqueous solution may comprise about 10 to about 28% v/v propylene glycol. Said aqueous solution may comprise about 15 to about 28% v/v propylene glycol. Said aqueous solution may comprise about 20 to about 28% v/v propylene glycol. Said aqueous solution may comprise about 10 to about 27% v/v propylene glycol. Said aqueous solution may comprise about 15 to about 27% v/v propylene glycol. Said aqueous solution may comprise about 20 to about 27% v/v propylene glycol. Said aqueous solution may comprise about 10 to about 25% v/v propylene glycol. Said aqueous solution may comprise about 15 to about 25% v/v propylene glycol. Said aqueous solution may comprise about 20 to about 25% v/v propylene glycol. Said aqueous solution may comprise about 23 to about 27% v/v propylene glycol. Said aqueous solution may comprise about 25% v/v propylene glycol.

Step (d) may be performed for at least about 10 minutes. Step (d) may be performed for at least about 13 minutes. In embodiment, step (d) may be performed for not more than about 20 minutes. Step (d) may be performed for not more than about 17 minutes, e.g. not more than about 16 minutes. In embodiment, step (d) may be performed for between at least about 10 minutes and not more than about 20 minutes. In an embodiment, step (d) may be performed for between at least about 12 minutes and not more than about 17 minutes. Step (d) may be performed for between at least about 13 minutes and not more than about 16 minutes. Step (d) may be performed for about 15 minutes. Step (d) may be performed under an inert atmosphere, for example under nitrogen or argon. The UV radiation may be provided by a UV lamp, such as a mercury lamp having UV emission of about 248-579 nm.

The xerogel irradiated in step (d) may have two faces and the irradiating may be performed for about half of the time on one face and about half of the time on the other face. The xerogel may have a thickness of at least about 50 $\mu$m. The xerogel may have a thickness of at least about 100 $\mu$m. The xerogel may have a thickness of at least about 150 $\mu$m. The xerogel may have a thickness of not more than about 800 $\mu$m. The xerogel may have a thickness of not more than about 600 $\mu$m. The xerogel may have a thickness of not more than about 500 $\mu$m. The xerogel may have a thickness of not more than about 400 $\mu$m. The xerogel may have a thickness of not more than about 300 $\mu$m. The xerogel may have a thickness of at least about 50 $\mu$m and not more than about 800 $\mu$m. The xerogel may have a thickness of at least about 100 $\mu$m and not more than about 600 $\mu$m. The xerogel may have a thickness of at least about 100 $\mu$m and not more than about 500 $\mu$m. The xerogel may have a thickness of at least about 100 $\mu$m and not more than about 400 $\mu$m. The xerogel may have a thickness of at least about 150 $\mu$m and not more than about 300 $\mu$m. The xerogel may have a thickness of at least about 200 $\mu$m and not more than about 250 $\mu$m.

The poly(ethylene oxide) may be as further specified herein for hydrogel of the disclosure.

The ratio of poly(ethylene oxide) to cross-linking agent in step (b) is from about 15:1 w/w to about 25:1 w/w. The ratio of poly(ethylene oxide) to cross-linking agent in step (b) The ratio of poly(ethylene oxide) to cross-linking agent in the hydrogel film may be from about 17:1 to about 23:1 w/w. The ratio of poly(ethylene oxide) to cross-linking agent in the hydrogel film may be from about 18:1 to about 22:1 w/w. The ratio of poly(ethylene oxide) to cross-linking agent in the hydrogel film may be about 20:1 w/w.

The cross-linking agent of step (b) may be an acrylate monomer. The cross-linking agent of step (b) may be an acrylate monomer comprising at least two ethynyl groups (e.g. an acrylate monomer comprising at least two acrylate groups). The crosslinking agent of step (b) may be a di-, tri- or tetra-acrylate monomer. The cross-linking agent of step (b) may be selected from: pentaerythritol tetraacrylate, pentaerythritol triacrylate, ethylene glycol diacrylate, di(ethylene glycol) diacrylate, tetra(ethylene glycol) diacrylate, ethylene glycol dimethacrylate, di(ethylene glycol) dimethacrylate, and tri(ethylene glycol) dimethacrylate. The cross-linking agent of step (b) may be pentaerythritol tetraacrylate.

The drying of step (c) may be performed at a temperature of not more than about 50 °C, e.g. at a temperature of not more than about 40 °C. The drying of step (c) may be performed at a temperature between 15 °C and 50 °C. The drying of step (c) may be performed at a temperature between 20 °C and 40 °C. The drying of step (c) may comprise a two-stage process. The first stage may comprise drying under ambient conditions for at least 24 hours. The second stage may comprise drying under vacuum, for example in a vacuum oven at a temperature of about 30 °C to about 45 °C (e.g. at about 40°C).

The naltrexone of step (a) may be naltrexone base or a pharmaceutically acceptable naltrexone salt (e.g. naltrexone hydrochloride). The naltrexone may be a naltrexone base. The naltrexone may be a pharmaceutically acceptable naltrexone salt. The naltrexone may be a pharmaceutically acceptable acid addition naltrexone salt. The naltrexone salt may be naltrexone hydrochloride, naltrexone hydrobromide, naltrexone glycolate, naltrexone lactate, naltrexone acetate, naltrexone fumarate, naltrexone citrate, or naltrexone tartrate. The naltrexone salt may be naltrexone hydrochloride, naltrexone glycolate, or naltrexone lactate. The naltrexone salt may be naltrexone hydrochloride.

Where the naltrexone is naltrexone base, the aqueous solution of step (a) may further comprise ethanol. The aqueous solution may comprise ethanol in an amount of about 5% to about 30% v/v ethanol. For example, the aqueous solution may comprise ethanol in an amount of about 10% to about 28% v/v ethanol, e.g. about 15% to about 28% v/v ethanol. For example, the aqueous solution may comprise aqueous solution may comprise ethanol in an amount of about 20% to about 25% v/v ethanol, e.g. about 25% v/v ethanol. Where the naltrexone is naltrexone base, the cross-linked hydrogel may comprise naltrexone base in an amount of from about 7% to about 15% w/w of the hydrogel. The cross-linked hydrogel may comprise naltrexone base in an amount of from about 7% to about 12% w/w of the hydrogel.

Where the naltrexone is a pharmaceutically acceptable naltrexone salt, the cross-linked hydrogel may comprise the naltrexone salt in an amount of from about 7% to about 30% w/w of the hydrogel, e.g. from about 7% to about 20% w/w of the hydrogel. The naltrexone salt may be present in an amount of from about 7% to about 12% w/w of the hydrogel. The naltrexone hydrochloride may be present in an amount of from about 7% to about 30% w/w of the hydrogel, e.g. from about 7% to about 20% w/w of the hydrogel. The naltrexone hydrochloride may be present in an amount of from about 7% to about 12% w/w of the hydrogel.

The hydrogel may have a tensile strength of at least 0.50 MPa (e.g. at least 0.6 MPa).

The method may further comprise forming a transdermal patch by: (f) affixing the cross-linked hydrogel to an occlusive adhesive tape, or sandwiching the cross-linked hydrogel between an occlusive adhesive tape and a permeable membrane. Step (f) may further comprise attaching a release liner to an adhesive surface of the adhesive tape.

The occlusive adhesive tape may be impermeable. The occlusive adhesive tape may comprise an outer layer that is an impermeable polymeric layer and an inner layer that is an adhesive layer. The inner layer may be in contact with the hydrogel. The impermeable polymeric layer may comprise any suitable polymer, such as polyester, polyethylene and/or polyurethane. The occlusive adhesive tape may be 3M Blenderm™, 3M Scotchpak™, 3M CoTran™, Leukoflex®, or Leukoplast® Sleek®.

The permeable membrane may comprise an ethylene vinyl acetate membrane. The permeable membrane may comprise a 3M™ CoTran™ Ethylene Vinyl Acetate Membrane. The permeable membrane may comprise a gauze.

The release liner may be coated with fluoropolymer and/or fluorosilicone. The release liner may be coated with fluoropolymer. The release liner may be coated with fluorosilicone. The release liner may be a 3M™ Scotchpak™ 1022 release liner, a 3M™ Scotchpak™ 9744 release liner, a 3M™ Scotchpak™ 9755 fluoropolymer coated release liner, or a 3M™ Scotchpak™ 9709 release liner fluorosilicone coated polyester film. The release liner may be a 3M™ Scotchpak™ 9755 fluoropolymer coated release liner. The release liner may be configured such that, after the release liner is attached to the adhesive surface, none of the adhesive surface is exposed.

The length and breadth of the hydrogel may be smaller than the length and breadth of the occlusive adhesive tape. This may allow the transdermal patch to be applied to a surface (e.g. skin) in a manner that allows that occlusive tape to isolate the hydrogel from the atmosphere. In embodiments, the permeable membrane, when present, may have a length and breadth that is greater than the length and breadth of the hydrogel, but smaller than the length and breadth of the occlusive adhesive tape. This may allow the hydrogel to be sandwiched between the occlusive adhesive tape and the permeable membrane, such that adhesion occlusive tape and permeable membrane assists in retaining the relative position of the hydrogel in the patch.

The methods of forming the transdermal patch may further comprise: (g) sealing the transdermal patch in a pouch. Step (g) may further comprise sterilising the pouch during and/or after said sealing.

## USES

The patches of the invention represent a novel dosage form of naltrexone. The patches of the invention may be particularly suitable for LDN therapy, for example for the treatment of a condition selected from Crohn's disease/ulcerative colitis, chronic fatigue syndrome/myalgic encephalomyelitis, autism, pruritus, diabetic wounds, HIV/AIDS, fibromyalgia, multiple sclerosis, inflammatory bowel disease, Crohn's disease/ulcerative colitis, complex regional pain syndrome, Hailey-Hailey disease, psoriasis, Ehlers-Danlos syndrome, cancer, Gulf War illness, depression, chronic arthritis, an autoimmune disorder, and a respiratory inflammatory disorder.

Typical dosages of naltrexone for LDN therapy are of the order of 0.075 mg - 2.7 mg per day. Exemplary LDN patches of the invention are able to achieve this, for example over a period of three days, with patches having a hydrogel with a face having a surface area of at least about 0.4 cm$^2$ to not more than 18 cm$^2$.

An aspect of the invention provides a transdermal patch of the present invention for use as a medicament. The transdermal patch for use in said treatment typically comprises an effective amount of naltrexone, or a pharmaceutically acceptable salt or solvate thereof.

In accordance with an aspect of the invention, there is provided a transdermal patch of the present invention for use in the treatment of a condition. The condition may be selected from selected from: opioid dependency, alcohol dependency, Crohn's disease/ulcerative colitis, chronic fatigue syndrome/myalgic encephalomyelitis, autism, pruritus, diabetic wounds, HIV/AIDS, fibromyalgia, multiple sclerosis, inflammatory bowel disease, Crohn's disease/ulcerative colitis, complex regional pain syndrome, Hailey-Hailey disease, psoriasis, Ehlers-Danlos syndrome, cancer, Gulf War illness, depression, chronic arthritis, and autoimmune disorder. The transdermal patch for use in said treatment typically comprises an effective amount of naltrexone, or a pharmaceutically acceptable salt or solvate thereof.

Exemplary respiratory inflammatory disorders include those that may cause extreme inflammation of the respiratory system or acute respiratory infection, e.g., coronavirus disease 2019 (COVID-19), severe acute respiratory syndrome (SARS), acute respiratory distress syndrome (ARDS), Middle East respiratory syndrome (MERS or MERS-CoV), or other coronavirus infections.

Low-dose naltrexone has been used for treatment of pain and inflammation in multiple sclerosis, Crohn's disease, fibromyalgia and other pain conditions. Lower than standard doses of naltrexone have been found to limit cellular proliferation of T- and B-cells and block Toll-like receptor 4 (TLR4), providing pain relief and anti-inflammatory benefit. Naltrexone at doses below the normal therapeutic dose appears to reduce production of multiple cytokines including IL-6 in a steady pace. Transdermal patches of the disclosure may therefore be useful for immunomodulation.

In the treatment of COVID-19 infection, it has been suggested that interrupting the IL-6 pathway can slow or interrupt the progression of the disease from mild/moderate (viral response and early pulmonary effects) to severe (late pulmonary response and hyper-inflammation). Therefore, given the observed effect of naltrexone on the production of IL-6, low-dose naltrexone could be a viable treatment for COVID-19, as well as other respiratory inflammatory disorders. Furthermore, it may be that the low-dose naltrexone could be administered to COVID-19 patients transdermally, for example, using a patch of the present invention.

ASSAYS

The patches of the invention can be assessed in relation to a number of parameters using standard tests that would be known to the person skilled in the art. A number of suitable assays are described below.

**(A) Drug content analysis**

Drug content analysis is a measure of the concentration of drug loaded onto the hydrogel and also provides an indication of the homogeneity of the drug within the loaded hydrogel.

Actual drug loading of naltrexone in the cross-linked poly(ethylene oxide) hydrogels of the invention may be calculated by drug content analysis. A novel HPLC method was developed to quantify the concentration of drug in the loaded hydrogels.

HPLC Settings

HPLC is performed using a reverse-phase column packed with 5 $\mu$m diameter C18 particles. The column is maintained at 30 °C for all chromatographic separations. The detection wavelength is set to 282 nm, equivalent to the $\lambda_{max}$ of naltrexone base and naltrexone hydrochloride. The separation uses an isocratic method over a 10-minute run time. The mobile phase is mixed in a 65:35 ratio of aqueous to organic. The organic phase is HPLC grade pure acetonitrile. The aqueous phase is 0.1% trifluoroacetic acid (TFA) containing 0.065% w/v sodium 1-octane sulfonate monohydrate, adjusted to pH 3 using trimethylamine (TEA). The injection volume for all separations is set to 10 $\mu$L. The flow rate is maintained at 0.7 mL/min.

Actual drug loading is calculated by drug content analysis. Target drug loading is determined by calculating the theoretical drug loading based on the balance readings of the hydrogel.

Determination of drug remaining in the film formulation

Each post-release film formulation was dissolved in 5 ml 80% v/v acetonitrile for 16 hours at 30°C in an Elmasonic S 30 H sonicating water bath (Elma Schmidbauer, Singen, Germany). After 16 hours, the samples were filtered using 0.2 $\mu$m Whatman syringeless filters vials with nylon filter media and polypropylene housing (GE Healthcare UK Ltd., Little Chalfont, Buckinghamshire, UK) and injected in triplicate to the HPLC in order to determine the residual drug remaining in the film.

**(B) Swelling properties and cross-linking assay**

Swelling properties of the naltrexone loaded hydrogels may be assessed by measuring the equilibrium swelling and gel fraction. Analysing the swelling of the hydrogels also allows for the degree of cross-linking of the loaded hydrogels to be calculated, as a higher level of cross-linking results in a lower level of swelling for a solvent system. The degree of cross-linking also provides an indication of the relative strength of the loaded hydrogels.

Increased drug loading typically results in decreased levels of cross-linking. However, without wishing to be bound by theory, it is believed that the hydrogels of the present invention achieve sufficient cross-linking at higher levels of drug loading as a result of longer irradiation under UV. Thus, a sufficiently strong hydrogel having an increased drug content can be obtained in accordance with the present disclosure.

Swelling studies are conducted using four replicas of pre-weighed xerogel films (1 × 1 cm). The xerogel films are swollen to equilibrium in 500 mL of distilled water for 72 h at 25 °C. The equilibrated swollen films are removed from water, blotted with filter paper, and weighed. The equilibrium swelling of each sample is calculated using Equation 1; this calculation is an adaptation of the method described in Omidian *et al.* (1994).

$$\text{Equilibrium Swelling (\%)} = \frac{Ms-(MD-D)}{(MD-D)} \times 100 \qquad (1)$$

where:

$M_s$ = weight of hydrogel at equilibrium,
D = weight of loaded drug,
$M_D$ = weight of drug loaded xerogel.

Following equilibrium swelling, the thickness of the swollen hydrogel films is measured using a micrometer. The samples are dried in a vacuum oven until constant weight values have been attained. Based on these weight values, the gel fraction may be calculated using Equation 2; this calculation is an adaptation of the method described in Padmavathi and Chatterji (1996).

$$\text{Gel fraction (\%)} = \frac{M'}{(M_D-D)} \times 100 \qquad (2)$$

where:

M' = weight of xerogel after extraction of water,
D = weight of loaded drug,
$M_D$ = weight of drug-loaded xerogel.

The average molecular weight between crosslinks may be calculated using Equation 3, as provided in Caykara and Inam *et al.* (2004).

$$\frac{1}{M_c} = \frac{2}{M_n} - \frac{\left(\frac{v}{V_1}\right)\left[ln\left(1-V_{2,s}\right) + V_{2,s} + X_1\left(V_{2,s}\right)^2\right]}{\left[\left(V_{2,s}\right)^{\frac{1}{3}} - \frac{V_{2,s}}{2}\right]} \qquad (3)$$

where:

$M_n$ = average molecular weight of PEO = 1,000,000 / 1.8 = 555,555.5556
v = specific volume of PEO = 0.833 $cm^3$/g,
$V_1$ = molar volume of water = 18.1 $cm^3$/mol,
$V_{2,s}$ = polymer volume fraction = calculated from Equation 4,
$X_1$ = polymer-solvent interaction = 0.45

Mv, the viscosity average molecular weight, is related to Mn in the following manner, for typical PEO of the disclosure. The Mv is almost equal to the value of the Mw (weight average molecular weight) of the polymer. This means that dividing Mv by the polydispersity index (which may be about 1.8 for exemplary PEO) provides Mn.

The polymer volume fraction may be calculated using Equation 4, as provided in Omidian *et al.* (1994).

$$V_{2,s} = \left[ 1 + \frac{\rho_p}{\rho_w} \left( \frac{M_s}{M_D - D} - 1 \right) \right] \quad (4)$$

where:

$\rho_p$ = polymer density (PEO) = 1.2 $g/cm^3$,
$\rho_w$ = solvent density (water) = 1.0 $g/cm^3$,
$M_s$ = weight of hydrogel at equilibrium,
D = weight of loaded drug,
$M_D$ = weight of drug-loaded xerogel.

The crosslink density may be calculated using Equation 5, as provided in Xia *et al.* (2013).

$$\rho_{C=} (v. M_c)^{-1} \quad (5)$$

where:

v = specific volume of PEO = 0.833 $cm^3$/g,
$M_c$ = calculated from Equation 3.

The mesh size may be calculated using Equation 6, as provided in Zustiak and Leach (2011).

$$\varepsilon = (V_{2,s})^{-\frac{1}{3}} \left( \frac{2 C_n M_c}{M_r} \right)^{\frac{1}{2}} l \quad (6)$$

where:

$C_n$ = polymer characteristic ratio for PEO = 4.1,
$M_r$ = molecular weight of polymer repeating units for PEO = 44 g/mol,
*l* = average bond length for PEO = 1.54 Å
$V_{2,s}$ = polymer volume fraction = calculated from Equation 4.
$M_c$ = calculated from Equation 3.

The results may be compared with the unmedicated hydrogel properties to determine statistical differences caused by drug loading.

**(C) Skin permeation assays**

Permeation testing is a measure of transdermal penetration of the drug formulation from the hydrogel into the skin and provides an indication of the expected dose administered to a patient over a given time. Permeation studies may involve calculating peak flux, daily flux, lag time, percentage dose permeated in a given time and surface area required to deliver a given amount of drug.

Without wishing to be bound by theory, it is believed that the permeation parameter values are dependent on the solution used to swell the loaded hydrogels.

Permeation protocol

Permeation studies may be conducted using human skin samples. The naltrexone loaded hydrogels are placed into respective swelling solutions for 5 minutes. The swelled hydrogels are then cut and applied to the human skin membranes. Each hydrogel is rubbed onto the skin surface using a glass rod to ensure good contact. Scotchpak™ 9732 Polyester Film Laminate Backing is used to occlude the hydrogels.

Absorption may be assessed by collecting receptor fluid samples at 0 (pre-dose), 1, 2, 4, 8, 30, 48, 56 and 72 h post dose. The removed receptor fluid volume is replenished with fresh receptor fluid solution after each withdrawal (except for the 72 h time point). After the 72 h sample is collected, cells are dismantled, the backing liner removed and discarded. The hydrogel films are removed and retained in a vial. The bulk receptor fluid is collected and the skin retained. No further samples are collected. The receptor fluid, hydrogels and skin samples are stored in a freezer maintained at -20 °C. All receptor fluid samples, hydrogel films and skin samples are then subject to HPLC analysis to determine the content of naltrexone in all these samples; and also the content of its metabolite in the receptor fluid and skin samples.

HPLC protocol

HPLC analysis is performed using a suitable C18 stationary phase chromatography system. An exemplary method may be performed as follows. The HPLC analysis was carried out using an Agilent 1290 Infinity liquid chromatography system consisting of a binary pump, Infinity autosampler, Infinity TCC and a variable wavelength detector (Agilent Technologies, Santa Clara, CA, USA). Separation was achieved by using HiChrom reverse-phase column packed with 5 $\mu$m diameter C18 particles (HiChrom, Reading, UK). Column length was 250 mm with an internal diameter of 4.6 mm. The column was maintained at 30 °C for all chromatographic separations. The detection wavelength was set to 282 nm. The separations used an isocratic method over a 20 minute run time. Mobile phase was mixed in an 80:20 ratio of aqueous to organic. The organic phase was HPLC grade pure acetonitrile. The aqueous phase was 0.1% trifluoroacetic acid containing 0.1% w/v sodium 1-octane sulfonate monohydrate, adjusted to pH 3 using triethylamine. The pH was measured using a calibrated Hanna pH 20 pH meter (Hanna Instruments, Leighton Buzzard, UK). The injection volume for all separations was set to 10 $\mu$L. The flow rate was maintained at 1.0 mL/min. Samples were filtered using 0.2 $\mu$m Whatman syringeless filters vials with nylon filter media and polypropylene housing (GE Healthcare UK Ltd., Little Chalfont, Buckinghamshire, UK) and injected in triplicate to the HPLC).

Analysis of naltrexone remaining in film after permeation

Each post-release film formulation was dissolved in 5 ml 80% v/v acetonitrile for 16 hours at 30°C in an Elmasonic S 30 H sonicating water bath (Elma Schmidbauer, Singen, Germany). After 16 hours, the samples were filtered using 0.2 $\mu$m Whatman syringeless filters vials with nylon filter media and polypropylene housing (GE Healthcare UK Ltd., Little Chalfont, Buckinghamshire, UK) and injected in triplicate to the HPLC in order to determine the residual drug remaining in the film.

**(D) Stability assay**

Stability testing provides an indication of the shelf-life of the transdermal patches of the present invention.

The stability of the transdermal patch of the invention may be investigated over the course of 6 months under ambient (20.9 ± 0.6 °C / 43 ± 2.5 %RH) and accelerated (40 °C / 75 %RH) conditions. The patches are tested at time intervals of 0, 1, 2, 3 and 6 months to determine the extent of any physical, chemical and microbial changes that may have occurred.

A number of specific tests may be conducted to determine the stability of the patches of the present invention. These tests may involve pH, FTIR, polarised microscopy, differential scanning calorimetry, tensile strength, peel adhesion, drug content analysis, and microbiology. The methods for performing these tests are disclosed in Banerjee *et al.* (2014), Zhang *et al.* (2018), Parhi and Padilam (2018), Dodou *et al.* (2015), Chenevas-Paule *et al.* (2017), Suksaeree *et al.* (2017), Thakur *et al.* (2016), Patel *et al.* (2009), European Medicines Agency (2014), Schulke (2019), and Koch *et al.* (2015). pH

Three samples of xerogel are partially swollen in distilled water and the surface pH is measured with a calibrated pH meter, such as a calibrated Hanna pH 20 pH meter. The surface pH was measured in triplicate. The percentage ionisation of naltrexone within the formulation can be calculated using the equation:

$$\text{Percentage ionisation (\%)} = \frac{100}{1 + \text{anti}\log(\text{pH} - \text{pKa})}$$

Where pKa of naltrexone free base for the tertiary aliphatic nitrogen is 8.3. <u>FTIR</u>

Fourier Transform Infrared Spectroscopy can be conducted to confirm the presence of naltrexone within the hydrogel network. Xerogel films can be observed on PerkinElmer Spectrum BX Fourier Transform Infrared Spectrometer (PerkinElmer Inc., Seer Green, UK). The spectral range was 4000-550 $cm^{-1}$ and the resolution was 2 $cm^{-1}$. Spectra were obtained using Spectrum™ software and evaluated based on literature absorbance values (Lambert, 1987).

Differential Scanning Calorimetry

Differential scanning calorimetry (DSC) of NTX.base, NTX.HCl, PEO and loaded hydrogel films (irradiated and non-irradiated) may be performed with suitable DSC instruments, such as a DSC Q1000 (TA Instruments, New Castle, DE, USA). The DSC was conditioned for two hours prior to use. Samples (5-10 mg) were accurately weighed using a microbalance (Mettler-Toledo, Greifensee, Switzerland), placed in an aluminium hermetic sample pan and sealed with a hermetic lid (TA Instruments, New Castle, DE, USA). The samples were subjected to standard heat/cool/heat at a rate of 10 °C/min, under a constant flow of nitrogen at a rate of 50 mL/min. Suitable settings include the following:

- For NTX.base API and NTX.base loaded films, the heat/cool/heat cycle was from - 80 °C to 200 °C to -80 °C to 200 °C.

- NTX.HCl API was heated from -80 °C to 285 °C, then cooled to -80 °C, and then heated for a second time to 285 °C. All naltrexone hydrochloride film samples (medicated and unmedicated) were heated from -80 °C to 265 °C, then cooled to - 80 °C, and then heated for a second time to 265 °C.

The first heating cycle is represented as cycle 1, cooling as cycle 2, and second heating cycle as cycle 3. The second heating cycles of these runs were used to determine the true melting point of the polymer and drug.

Hot Stage Microscopy

Hot stage microscopy may be performed according to a method adapted from Lofty and Dodou, 2014. In the adapted method, NTX.base, NTX.HCl, unmedicated and medicated hydrogel films were observed under the hot stage microscope (HSM) equipped with a Leica DM750 microscope (Leica, Milton Keynes, UK), Infinity 2 camera (Lumenera, Ottawa, Canada), Mettler Toledo FP82HT hot stage and Mettler Toledo FP90 central processor (Mettler Toledo, Leicester, UK). Video footage was recorded using Studio Capture software. For naltrexone base samples were heated and cooled from 25 °C to 200 °C to 25 °C at a heating and cooling rate of 2 °C/min and 5 °C/min, respectively. For naltrexone hydrochloride, samples were heated and cooled from 25 °C to 285 °C to 25 °C at a heating and cooling rate of 2 °C/min and 5 °C/min, respectively.

Rheology

The rheological properties of unmedicated and medicated xerogels were established using a Malvern Kinexus rotational rheometer (Malvern Instruments Ltd, Malvern, UK) fitted with a 20 mm diameter stainless steel plate. Five drops of distilled water was added the surface of each xerogel disc, and left to swell for approximately 1 minute. The swollen film was then fixed between the lower plate and upper parallel plate. The hydrogel was trimmed to size. The swollen thickness of the film (290 µm - 330 µm) was measured using a micrometer, and recorded as the gap size. An amplitude sweep test and a frequency sweep test were conducted to assess the films' function of shear strain and frequency, respectively. All tests were carried out in triplicate at 32°C.

*Amplitude Sweep*

The linear viscoelastic region (LVR) of all samples was determined with an amplitude sweep at incremental shear strains (1 to $10^6$ Pa) and a fixed frequency of 1 Hz. The mean elastic moduli (G') and viscous moduli (G") were plotted against percentage complex shear strain. The resulting plot displayed the LVR. The LVR was then used to identify stress and strain values suitable for frequency sweep test.

*Frequency Sweep*

Frequency sweep measurements of film samples were established from 100 to 0.1 rad/s. The mean elastic moduli (G') and viscous moduli (G") were plotted against frequency.

Mechanical testing

Tensile strength, Young's modulus and percentage elongation on breakage may be determined using a mechanical testing protocol. A suitable protocol is as follows. Xerogel strips were cut to a width of 10 mm and gauge length of 50 mm. Fifteen drops of distilled water was added to the surface of each xerogel strip. The strips were allowed to swell for approximately 1 minute. The thickness of each tape strip was measured using a micrometer (Duratool, Taichung, Taiwan) and recorded as 120 $\mu$m. The samples were securely attached vertically between two clamps on a Lloyd LS1 Material Tester (AMETEK Test and Calibration Instruments, Largo, FL, USA). Parameters were set to use a 0.7 N load cell, extension rate of 10 mmmin$^{-1}$ and 21 mmmin$^{-1}$ pre-load stress speed. Samples were subjected to tension until breakage. The mechanical test was performed in triplicate. Tensile strength, Young's modulus and percentage elongation on breakage data were acquired from Nexygen Plus software (Lloyd Instruments, Bognor Regis, UK).

**EXAMPLES**

Example 1: Manufacture of naltrexone base loaded xerogels

Naltrexone base (0.9223 g) was transferred to a glass jar containing 100mL of 25% v/v ethanol solution in water and sonicated until fully dissolved. Poly(ethylene oxide) (8 g) was added to the solution under rigorous stirring for 24 hours until a homogenous gel mixture was formed. 5% w/w pentaerythritol tetraacrylate (0.4 g) was added to the mixture and stirred for a further 8 hours and was then sealed and allowed to stand for approximately 16 hours. The mixture was then cast onto a clean glass tile and dried at room temperature for 48 hours to form a xerogel film containing 9.89% w/w naltrexone free base. The xerogel film was cut into 3 × 5 cm rectangles and placed into the vacuum oven at 40 °C for 24 hours to dry completely. A micrometer was used to measure the thickness of the xerogel film which was determined to be 220 $\mu$m. Each xerogel film was irradiated under nitrogen for 7.5 minutes on each side (15 minutes total) using a 150W medium pressure mercury lamp (UV emission = 248-579 nm, $\lambda_{max}$ = 366 nm).
The pH value was measured as 6.78 ($\pm$ 0.01, n=9), providing drug ionisation in the film of97.1%.

Example 2: Manufacture of naltrexone hydrochloride loaded xerogels

Naltrexone hydrochloride (0.6678 g) was transferred to a glass jar containing 100 mL deionised water and sonicated until fully dissolved. Poly(ethylene oxide) (8 g) was added to the solution under rigorous stirring for 24 hours until a homogenous gel mixture formed. 5% w/w pentaerythritol tetraacrylate (0.4 g) was added and stirred for a further 8 hours. The mixture was sealed and allowed to stand for approximately 16. The mixture was then cast onto a clean glass tile and dried at room temperature for 48 hours to form a xerogel film containing 7.36% w/w naltrexone hydrochloride. The xerogel film was cut into 3 × 5 cm rectangles and placed into the vacuum oven at 40 °C for 24 hours to dry completely. A micrometer was used to measure the thickness of the xerogel film and was determined to be 230 $\mu$m. Each xerogel film was irradiated under nitrogen for 7.5 minutes on each side (15 minutes total) using a 150W medium pressure mercury lamp (UV emission = 248-579 nm, $\lambda_{max}$ = 366 nm).
The pH value was measured as 5.64 ($\pm$ 0.00, n=9), providing drug ionisation in the film of 99.8%.

Example 3: Manufacture of transdermal patches

The loaded xerogels films were cut to 1 cm$^2$ squares and swollen in either water or 25% v/v propylene glycol solution. Once swollen, the loaded hydrogels were placed onto the centre of the adhesive side of Blenderm surgical tape (25 cm2) (3M™ Healthcare Ltd., Loughborough, UK) and secured using CoTran 9702 rate-controlling membrane (6.25 cm2) (3M™ Healthcare Ltd., Loughborough, UK). Scotchpak 9755 backing membrane (25 cm2) was used as the release liner. Patches were placed in a P616 white high barrier laminate pouch with CXB™ sealant film (35694 G) (Bemis Healthcare Packaging Ltd., Derry, Northern Ireland). Pouches were individually heat sealed using Fermant 400 heat sealer (JoKe Reno Tec, 2017) for 2.5 seconds pulse time and 7 seconds press time.

Example 4: Analysis of drug loading

Fourier Transform Infrared Spectroscopy was conducted to confirm the presence of naltrexone within the hydrogel network. The method used is as described in the "Assays" part of the present disclosure.
Naltrexone base loaded patches were examined by FTIR and can be observed in Figure 2 A). When compared to the wavenumber of the carbonyl peak for naltrexone base API, the peak shifted from 1729 cm$^{-1}$ to 1728 cm$^{-1}$ for all loaded films. This shift was negligible and therefore unlikely it could be attributed to drug-polymer interactions. The carbonyl peak increased in intensity as concentration of *in-situ* loaded drug increased. This was expected as peak intensity is relative to concentration. There was no shift observed for the $CH_2$ stretch for poly(ethylene) oxide, this remained constant in all samples at a wavenumber of 2882 cm$^{-1}$. Therefore it could be likely that for naltrexone base-loaded films there were no drug-polymer interactions.

Naltrexone HCl loaded films were examined by FTIR and can be observed in Figure 2 B). When compared to the wavenumber of the carbonyl peak for naltrexone hydrochloride API, the peak had shifted from 1717 cm$^{-1}$ to 1728 cm$^{-1}$. The $CH_2$ stretch of polyethylene oxide shifted to a lower wavenumber from 2882 cm$^{-1}$ to 2874 cm$^{-1}$. This is indicative of increase in intermolecular stabilisation, indicative of strong drug-polymer interactions (Kothari et al., 2014). The shape of the $CH_2$ stretch changed post-irradiation, therefore suggesting that crosslinking had an effect on the drug-polymer interactions. Even though the FTIR study indicated that there was some drug-polymer interaction, it is important that the hydrogel films are sufficiently crosslinked in order to improve rheological and mechanical properties for the hydrogels to be applied to the skin. In addition, the results for peak flux for Naltrexone HCl (see Table 3, below) indicated that this drug-polymer interaction did not prevent Naltrexone HCl having sufficiently high permeation values.

The drug content in the patches was determined using the HPLC method described in the "Assays" part of the present disclosure. The HPLC analysis was carried out using an Agilent 1290 Infinity liquid chromatograph consisting of a binary pump, Infinity autosampler, Infinity TCC and a variable wavelength detector (Agilent Technologies, Santa Clara, CA, USA). Separation was achieved by using HiChrom reverse-phase column packed with 5 μm diameter C18 particles (HiChrom, Reading, UK). Column length was 250 mm and an internal diameter of 4.6 mm, resulting in a column volume of 4.15 mL. The column was maintained at 30 °C for all chromatographic separations. The detection wavelength was set to 282 nm, which was determined to be the lambda max of NTX.base and NTX.HCl from using a full scan on a UV spectrophotometer. The separation used an isocratic method over a 10 minute run time. Mobile phase was mixed in a 65:35 ratio of aqueous to organic. The organic phase was HPLC grade pure acetonitrile. The aqueous phase was 0.1% trifluoroacetic acid (TFA) containing 0.065% w/v sodium 1-octane sulfonate monohydrate, adjusted to pH 3 using trimethylamine (TEA). The pH was measured using a calibrated Hanna pH 20 pH meter (Hanna Instruments, Leighton Buzzard, UK). The injection volume for all separations was set to 10 μL. The flow rate was maintained at 0.7 mL/min.

Standard deviation values of loaded hydrogels were low, suggesting homogeneous drug loading.

**Table 1:** Levels of drug loading for the naltrexone base and naltrexone hydrochloride loaded cross-linked poly(ethylene oxide) hydrogels of the invention.

| Hydrogel type | Target drug loading (% w/w) | Actual drug loading (% w/w) (±SD) (n = 6) |
|---|---|---|
| *In-situ NTX base* | 9.89 | 10.89 (± 0.86) |
| *In-situ* NTX.HCl | 7.36 | 7.65 (± 0.11) |

Example 5: DSC analysis of hydrogels

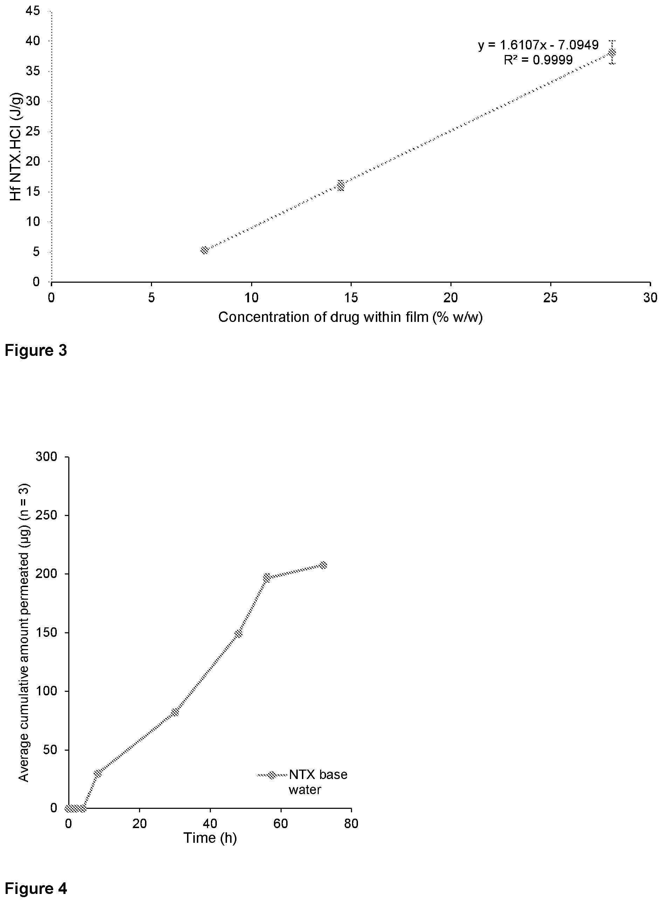

DSC analysis was performed, in accordance with the method described in the "Assays" part of the present disclosure. DSC results are summarised in Table 2. The DSC results indicated that Crystalline drug was only apparent by DSC for the naltrexone hydrochloride loaded films. This was a result of naltrexone hydrochloride being saturated within the film. From the enthalpy of fusion values for NTX.HCl within the films, the saturation solubility of naltrexone hydrochloride within PEO was calculated by plotting enthalpy against drug concentration. The resulting graph can be observed in Figure 3. From this graph, the *y* value can be substituted with 0 to find that the maximum drug loading before crystals are loaded to the film is 4.40% w/w. A similar observation was presented graphically in a study by Bansal et al., where they correlated enthalpy to drug concentration (Bansal et al., 2008).

Polymer crystallinity was decreased with the addition of drug. This was apparent by DSC studies whereby there was a significant reduction is $T_m$ and $H_f$ of poly(ethylene oxide) after irradiation for the naltrexone hydrochloride loaded films. There was an insignificant change in the $T_m$ and $H_f$ of poly(ethylene oxide) after irradiation for the naltrexone base loaded films. This suggested that it was caused by presence of crystalline drug which was interacting with the polymer (evidence from FTIR). Also, the thermal behaviour was affected when the films were exposed to different thermal conditions. The films that were loaded with naltrexone hydrochloride were heated to a maximum of 265 °C, which differed to the naltrexone base films which were heated to 200 °C. This was because the melting point of the pure active pharmaceutical ingredients differed. Naltrexone hydrochloride was found to decompose immediately after melting and therefore the films could only be heated to a maximum of 265 °C. There was a significant decrease in the $T_g$ of poly(ethylene oxide) when the loading of naltrexone hydrochloride was increased. This was because of the plasticising effect of the drug i.e. there was an increase in molecular mobility due to the formation of fewer polymer-polymer crosslinks as a result of drug loading (Stelescu et al., 2018).

Drug-polymer interactions were also evaluated by DSC. The melting temperature of NTX.HCl within the samples was lower than the melting temperature recorded for the API (∼ 250 °C compared to 280 °C). This was likely due to miscibility of naltrexone hydrochloride with the polyethylene oxide) hydrogel (Alvarez-Fuentes et al., 1997). This observation was comparable to that in a study by Dodou and Saddique whereby the melting point of ibuprofen was depressed when present in a drug-polymer blend compared to the melting of ibuprofen API analysed on its own (Dodou and Saddique, 2012). A similar observation was seen in a recent study by Rask *et al* where the researchers used a melting point depression method to predict drug-polymer solubility. (Rask et al., 2018).

There was a greater difference between melting of the polymer for unmedicated samples and medicated samples observed during the second heat, strengthening the evidence that as the drug concentration was increased, the drug-polymer miscibility decreased. In the study by Dodou and Saddique, the mixture with the highest percentage of polymer displayed the endotherm closest to the reference endotherm for polymer on its own (Dodou and Saddique, 2012; Park et al., 2001)).

The DSC results were correlated with the results for hot stage microscopy, as a visual technique whereby melting and phase transitions were observed using a camera.

Table 2: Effect of drug loading on enthalpy and melting of hydrogel films, measured by differential scanning calorimetry.

| Sample | $H_f$ NTX base (J/g) $X_c$ (n = 3) (± SD) | NTX base (%) (n = 3) (± SD) | $T_m$ 1st heat (NTX base) (°C) (n = 3) (± SD) | $T_m$ 1st heat (PEO) (°C) (n = 3) (± SD) | $T_m$ 2nd heat (PEO) (°C) (n = 3) (± SD) | $T_g$ PEO (°C) (n = 3) (± SD) | $T_g$ NTX (°C) (n = 3) (± SD) |
|---|---|---|---|---|---|---|---|
| NTX base API | 147.17 (± 0.55) | | 170.95 (± 0.68) | | | | 80.74 (± 0.54) |
| PEO | | | | 69.91 (±0.19) | 65.44 (±0.28) | -52.17 (±0.14) | |
| Unmedicated film | | | | 74.19 (± 1.64) | 69.15 (± 0.45) | -52.15 (± 0.79) | |
| Film *In-situ* loaded with NTX base | N/A | N/A | N/A | 69.47 (± 1.31) | 65.84 (± 0.34) | -45.51 (± 0.35) | N/A |
| NTX.HCl API | 193.40 (± 5.66) | | 279.39 (± 0.32) | | | | 97.58 (± 0.64) |
| Unmedicated film | | | | 171.02 (± 0.50) | 69.41 (± 1.20) | -52.25 (± 1.02) | |
| Film *In-situ* loaded with NTX.HCl | 5.33 (± 0.43) | 2.75 (± 0.22) | N/A | 70.33 (± 0.39) | 67.18 (± 1.72) | -52.22 (± 0.27) | N/A |

Example 6: Rheological analysis of hydrogels

The hydrogels were subjected to rheology analysis in accordance with the method set out in the "Assays" part of the present disclosure. The results obtained for the rheology analysis of the hydrogels are indicated in table 3.

Table 3: Effect of drug loading on rheological properties of hydrogel films

| Sample | Elastic modulus G' $10^4$ (Pa) (± SD) (n = 3) | Viscous modulus G" $10^4$ (Pa) (± SD) (n = 3) | Critical strain $\gamma_0$ (%) (± SD) (n = 3) | Complex viscosity at frequency = 10 Hz $\eta^* 10^3$ (Pa.s) (±SD) (n = 3) |
|---|---|---|---|---|
| Unmedicated (prepared with 100% water) | 10.03 (± 0.12) | 1.31 (± 0.98) | 1.51 (± 0.03) | 1.32 (± 0.49) |
| Unmedicated (prepared with 25% v/v EtOH) | 10.37 (± 0.51) | 1.35 (± 0.05) | 1.89 (± 0.15) | 1.51 (± 0.02) |

(continued)

| Sample | Elastic modulus G' $10^4$ (Pa) (± SD) (n = 3) | Viscous modulus G" $10^4$ (Pa) (± SD) (n = 3) | Critical strain $\gamma_0$ (%) (± SD) (n = 3) | Complex viscosity at frequency = 10 Hz $\eta^* 10^3$ (Pa.s) (±SD) (n = 3) |
|---|---|---|---|---|
| *in-situ* loaded with NTX base | 23.25 (± 5.72) | 2.48 (± 0.49) | 1.07 (± 0.01) | 4.32 (± 0.87) |
| *in-situ* loaded with NTX.HCl | 16.62 (± 1.73) | 2.47 (± 0.58) | 1.34 (± 0.07) | 2.32 (± 0.48) |

The amplitude sweep test was conducted for all samples using the rheometer in order to determine elastic modulus, viscous modulus and critical strain. The moduli were measured as a function of strain. Therefore, the resulting graphs yielded a linear viscoelastic region (LVR). A linear viscoelastic region is indicative of successful crosslinking of the hydrogel films. All swollen film samples displayed an LVR which was a result of sufficient crosslinking. Based on the results, the solvent used to prepare the hydrogels, such as 25% v/v ethanol solution in order to facilitate the dissolution of naltrexone base, did not affect the rheological properties of the resulting hydrogel.

All films showed solid-like behaviour as confirmed by the elastic modulus values being consistently greater than viscous modulus values (Yan and Pochan, 2011). Rheological properties also have an effect on whether a hydrogel is suitable for use on the skin. Rheological tests were conducted at 32 °C to simulate the temperature of the skin's surface (Redisch et al., 1952). The films tested in this study were all independent of frequency as the moduli did not change over a range of 0.1 to 100 Hz. Frequency independent behaviour is a characteristic behaviour of gels. Whereas frequency dependence is indicative of adhesive capability as identified in a study by Ho and Dodou (Ho and Dodou, 2007). All of these properties are ideal for skin application. Therefore, it could be inferred that since the present hydrogels are frequency independent, it would be appropriate to incorporate an adhesive as part of the patch assembly of the present disclosure e.g. through the use of an adhesive and occlusive backing such as Blenderm™ surgical tape. Complex viscosity is indicative of how solid a sample is. Relating this to drug release, the more likely that drug release would be sustained, as diffusion coefficient of drug molecules is inversely proportional to the viscosity of the diffusion medium. Thus the cross-linked naltrexone hydrogels of the present invention have suitable rheological properties for dermatological application.

Example 7: Mechanical testing of hydrogels

Mechanical testing of hydrogels was performed in accordance with the method set out in the "Assays" part of the present disclosure. The mechanical testing results are provided in Table 4.

**Table 4:** Effect of drug loading method on the mechanical properties of hydrogels determined using the tensile tester

| Hydrogel type | Tensile strength (MPa) (± SD) (n = 3) | Young's modulus (MPa) (± SD) (n = 3) | Percentage total elongation at break (%) (± SD) (n = 3) |
|---|---|---|---|
| Unmedicated (using 100% water) | 1.116 (± 0.06) | 6.799 (± 1.04) | 20.427 (± 2.42) |
| Unmedicated (using 25% v/v EtOH solution) | 1.104 (± 0.01) | 6.383 (± 0.41) | 21.237 (± 0.52) |
| *In-situ* loaded NTX base | 0.712 (± 0.08) | 6.880 (± 0.49) | 11.790 (± 1.14) |
| *In-situ* loaded NTX.HCl | 0.912 (± 0.10) | 7.021 (± 0.56) | 17.914 (± 1.54) |

Unmedicated films were subject to mechanical testing in order to directly compare the effect of drug loading on the tensile strength, Young's modulus and percentage total elongation at break. Firstly, unmedicated films that were made with 100% water were examined. The tensile strength was measured to be 1.116 ± 0.06 MPa, Young's modulus was 6.799 ± 1.04 MPa and percentage total elongation at break was 20.427 ± 2.42 %. The *in-situ* naltrexone base films were prepared using 25% v/v ethanol solution in order to facilitate the dissolution of naltrexone base during manufacture. Therefore, unmedicated films that had been made with 25% v/v ethanol solution were examined. The tensile strength was measured to be 1.104 ± 0.01 MPa, Young's modulus was 6.383 ± 0.41 MPa and percentage total elongation at break was 21.237 ± 0.52 %. The differences were statistically insignificant (p = 0.563, p = 0.841 and p = 0.534 for tensile strength, Young's modulus and percentage total elongation at break, respectively).

All films were dried for 24 hours in a vacuum oven before conducting the tensile test. This was to remove any trapped water from within the hydrogel network. This was of particular importance for the *in-situ* films as bound water would likely alter elasticity. Swelling capacity of a hydrogel is inversely proportional to the mechanical strength of the hydrogel (Anseth et al., 1996). This is believed to be due to the presence of fewer crosslinks resulting in decreased mechanical strength. The results obtained in the present study were consistent with this: for the tested hydrogels, higher drug loading broadly correlates with greater equilibrium swelling and weaker films, as confirmed by the reduction in tensile strength and critical strain data.

The mechanical properties do, however, establish that the naltrexone containing hydrogels of the present disclosure are suitable for dermatological application. All films had good tensile and Young's modulus properties and therefore were flexible enough to conform to movement of skin and strong enough to avoid brittleness (breakage).

Example 8: Swelling properties and degree of crosslinking in naltrexone loaded hydrogels

Swelling properties of the hydrogels were assessed in accordance with the methods set out in the "Assays" part of the present disclosure. There were significant differences between the swelling properties of all the drug-loaded hydrogels compared with the unmedicated hydrogels ($p < 0.05$). As drug concentration increased, equilibrium swelling increased.

Without wishing to be bound by theory, it is believed that increased levels of drug loading result in the formation of the crosslinked polymer network around any undissolved drug, increasing the size of the pores.

Equilibrium swelling was significantly higher for the naltrexone hydrochloride loaded hydrogels whereby drug exceeded saturation and therefore was undissolved. This theory was confirmed by the increase in average molecular weight between crosslinks and mesh size, which was also found to increase when drug concentration increased.

Table 5: Effect of drug type on the swelling properties and degree of crosslinking in naltrexone base (NTX base) and naltrexone hydrochloride (NTX.HCl) loaded poly(ethylene oxide) hydrogels

| Hydrogel type | Equilibrium swelling (%) ($\pm$SD) (n = 4) | Gel fraction (%) ($\pm$SD) (n = 4) | Average molecular weight between crosslinks $\overline{M}_c$ (g.mol$^{-1}$) ($\pm$SD) (n = 4) | Crosslink density $\rho_c$ 10$^{-4}$ (mol.cm$^{-3}$) ($\pm$SD) (n = 4) | Mesh size $\xi$ (Å) ($\pm$SD) (n = 4) |
|---|---|---|---|---|---|
| Unmedicated | 202.82 ($\pm$ 4.54) | 91.08 ($\pm$ 0.80) | 756.30 ($\pm$ 33.31) | 15.90 ($\pm$ 0.70) | 27.57 ($\pm$ 0.75) |
| NTX base | 342.98 ($\pm$ 23.45) | 78.68 ($\pm$ 2.88) | 2160.34 ($\pm$ 264.21) | 5.63 ($\pm$ 0.70) | 53.19 ($\pm$ 4.66) |
| NTX.HCl | 345.45 ($\pm$ 10.15) | 84.67 ($\pm$ 0.50) | 2185.37 ($\pm$ 129.29) | 5.51 ($\pm$ 0.32) | 53.64 ($\pm$ 2.01) |

Gel fraction decreased as the drug loading increased, indicating that the degree of crosslinking is greater at lower drug saturation levels. At higher drug saturations, the degree of cross-linking is reduced.

The drug-loaded hydrogels all displayed mesh size less than 100 Å. Mesh classification indicates the only the space between the polymer crosslinks is susceptible to diffusion.

Example 9: Permeation Analysis of naltrexone loaded hydrogels

Three samples of full thickness human skin (abdomen) were obtained from three donors aged 39, 47 and 61 years old. Split-thickness skin was prepared by cutting the skin to a depth of 200 - 400 $\mu$m with an electric dermatome and the skin integrity was confirmed. The permeation of naltrexone from the hydrogels into the skin was then assessed in accordance with the methods set out in the "Assays" part of the present disclosure.

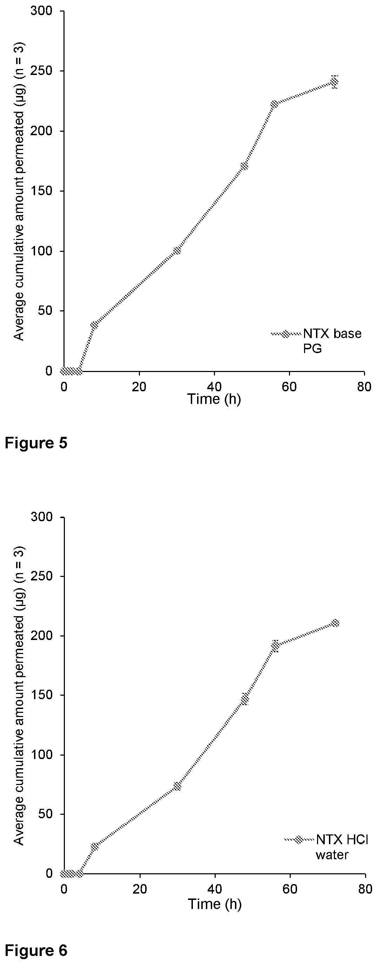

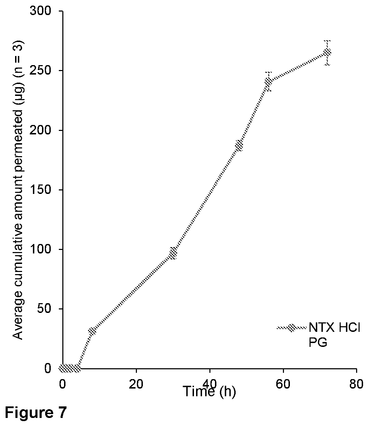

The permeation parameters of naltrexone base and naltrexone hydrochloride from hydrogels of the invention swollen in either water or 25% v/v propylene glycol are provided in Table 6. Peak flux was calculated from the gradient of the linear portion of the permeation profiles (Figures 4 to 7). Daily flux may be calculated simply by multiplying the peak flux by 24 hours. Lag time was calculated using Equation 7:

$$L = \frac{h^2}{6D} \qquad (7)$$

where:

h = thickness of the membrane (cm)

D = Diffusion coefficient (cm$^2$/h).

**Table 6:** Permeation parameters of naltrexone base and naltrexone hydrochloride from hydrogels of the invention.

| Permeation parameter | Mean $\pm$ SD (n = 3) | | | |
| --- | --- | --- | --- | --- |
| | NTX base water | NTX base PG | NTX HCL water | NTX HCL PG |
| Peak flux ($\mu$g/cm$^2$/h) | 6.74 $\pm$ 0.04 | 6.75 $\pm$ 0.10 | 6.31 $\pm$ 0.06 | 7.83 $\pm$ 0.31 |
| Daily flux ($\mu$g/cm$^2$/day) | 161.74 $\pm$ 0.84 | 161.99 $\pm$ 2.51 | 151.45 $\pm$ 1.53 | 181.87 $\pm$ 7.41 |
| Lag time (h) | 4.52 $\pm$ 0.49 | 4.35 $\pm$ 0.29 | 5.58 $\pm$ 0.38 | 4.51 $\pm$ 0.42 |
| Percentage of dose permeated in 72h (%) | 61.77 $\pm$ 0.17 | 71.69 $\pm$ 1.54 | 52.31 $\pm$ 0.59 | 65.73 $\pm$ 2.51 |
| Required surface area in order to deliver 0.075 mg per day (cm$^2$) | 0.46 $\pm$ 0.00 | 0.46 $\pm$ 0.01 | 0.50 $\pm$ 0.01 | 0.40 $\pm$ 0.02 |
| Required surface area in order to deliver 2.7 mg per day (cm$^2$) | 16.69 $\pm$ 0.09 | 16.67 $\pm$ 0.26 | 17.83 $\pm$ 0.18 | 14.39 $\pm$0.58 |

Hydrogels of the invention swelled in 25% v/v propylene glycol aqueous solution achieved a peak flux value of 7.83 $\pm$ 0.31 $\mu$g/cm$^2$. This value is better than that obtained previously for other means of transdermal delivery of naltrexone using prior art methods and formulations. For example, a naltrexone formulation of propylene glycol and buffer in a flow-through solution demonstrated a peak flux value of 1.6046 $\mu$g/cm$^2$/h *via* human skin (Paudel *et al.,* 2005). Additionally, while the prior art demonstrated a cumulative permeation of 56.6 $\mu$g/cm$^2$ over 48 hours, the hydrogels of the invention achieved a permeation of three times that value in 24 hours (181.87 $\pm$ 7.41 $\mu$g/cm$^2$).

Typical LDN doses are of the order of 0.075 mg - 2.7 mg per day. The LDN patches of the invention are able to achieve this with a surface area of at least about 0.4 cm$^2$ to not more than 18 cm$^2$. This is comparable to the size of commercial transdermal patches.

At 72 hours, between 52.31 - 71.69 % of the naltrexone applied was found to permeate the skin i.e. was found in the receptor fluids. After the study had been completed, the residual drug content within the hydrogel films and the skin samples was extracted and quantified. Extraction of residual drug and metabolite from the skin samples was carried out using the method from Dodou *et al* 2015. Each skin sample was added to 2 mL 1M potassium hydroxide and dissolved using stirring and sonication. Undissolved skin was filtered out, and the filtrate was neutralised with glacial acetic acid before quantifying. Metabolite amounts in the skin and receptor fluid samples were converted to drug amounts as in previous work (Dodou *et al* 2015). It was found that at 72 hours between 11.54 - 15.97 % naltrexone base/naltrexone hydrochloride was retained within the hydrogel films. Between 15.77 - 21.94 % of the total naltrexone content was found residual in the skin samples. Therefore, between 92.51 - 99.00 % of the total naltrexone loaded in the films was accounted for across the compartments of the diffusion cells at the end of the diffusion study.

Example 10: Stability of transdermal patches

**Stability Protocol**

Packaged pouches were either placed under ambient conditions (20.9 $\pm$ 0.7 °C / 46.0 $\pm$ 3.1% RH) or under accelerated conditions (40 °C / 75 %RH), for 6 months with intermediate testing at 0, 1, 2, 3 and 6 months according to the protocol in Table 7.

**Table :7** Stability testing protocol

| Test | Reference | Testing time points(months) |
|---|---|---|
| Appearance (macroscopic and microscopic) | (Banerjee et al., 2014) (Zhang et al., 2018) (Parhi and Padilam, 2018) (Dodou et al., 2015) (Chenevas-Paule et al., 2017) | 0, 1, 2, 3, 6 |
| pH | (Banerjee et al., 2014) | 0, 1, 2, 3, 6 |
| FTIR | (Parhi and Padilam, 2018) | 0, 1, 2, 3, 6 |
| Differential scanning calorimetry | (Banerjee et al., 2014) (Parhi and Padilam, 2018) (Chenevas-Paule et al., 2017) | 0, 3, 6 |
| Tensile strength | (Suksaeree et al., 2017) (Thakur et al., 2016) (Patel et al., 2009) | 0, 6 |
| Peel adhesion | (European Medicines Agency, 2014) | 0, 6 |
| Drug content analysis | (Banerjee et al., 2014 | )0, 1, 3, 6 |
| Microbiology | (Schulke, 2019) (Koch et al., 2015) | 0, 2, 6 |

*Appearance*

Patches were removed from their stability conditions and packaging was inspected for seal integrity and development of perforations or holes on the surface of the pouch. The patches were then taken apart and each component (Blendern, hydrogel, release membrane and removable liner) was examined by microscopy under polarised and non-polarised light at 10x magnification using an Olympus BH-2 Microscope (Optivision, Japan) equipped with Carl Zeiss AxioCam MRc Camera (AG, Germany). Micrographs were acquired using AxioVision v4.4 software (AG, Germany).

*pH*

Three patches from each batch had their permeable membrane removed and the surface pH of the hydrogel film was measured in triplicate using a calibrated pH meter (Hanna Instruments, Leighton Buzzard, UK).

*FTIR*

All components of patches were tested on PerkinElmer Spectrum BX Fourier Transform Infrared Spectrometer (PerkinElmer Inc., Seer Green, UK) as explained herein in the "Stability assay" section. The hydrogel films were allowed to turn to xerogels by storage at ambient conditions for 24 hours, before FTIR testing. The spectral range was 4000-550 $cm^{-1}$ and the resolution was 2 $cm^{-1}$. Spectra were obtained using Spectrum™ software and evaluated based on literature absorbance values, and spectra were compared to previous time points (Lambert, 1987).

*Differential scanning calorimetry*

The hydrogel films were allowed to turn to xerogels by storage at ambient conditions for 24 hours before DSC testing. DSC method explained herein in the "Stability assay" section.

*Tensile strength*

Rectangular strips, with a width of 10 mm and gauge length of 50 mm were cut from the centre of the hydrogel patches (n = 3). The thickness of each patch strip was measured using a micrometer (Duratool, Taichung, Taiwan) and recorded as 480-550 $\mu$m (depending upon the patch type). The samples were securely attached vertically between two clamps on a Lloyd LS1 Material Tester (AMETEK Test and Calibration Instruments, Largo, FL). Parameters were set to use a 5 N load cell, extension rate of 10 mmmin$^{-1}$ and 21 mmmin$^{-1}$ pre-load stress speed. Samples were subjected to tension until breakage. The mechanical test was performed in triplicate. Tensile strength, Young's modulus and percentage elongation on breakage data were acquired from Nexygen Plus software (Lloyd Instruments, Bognor Regis, UK).

*90° peel test*

This method was based upon EMA guidelines (European Medicines Agency, 2014). The width and length of each patch was measured, with a width of 50 mm and length of 50 mm. The patches were adhered to a 90 degree sliding peel table (228 mm × 123 mm) which was fixed to the Lloyd LS1 Material Tester. One end of the patch was not adhered, and was attached to the upper clamp at a 90 degree angle. A 90/180 degree test was performed, subjecting force until the patch was detached from the sliding peel table. The crosshead speed was set to 30 mm/min. The calculation limits were set so that limit 1 was 10 mm from the start of the test and limit 2 was 40 mm from the start of the test. The 90 degree peel test was repeated in triplicate. After the test, it was observed if there was any residue remaining on the peel table.

*Drug content analysis*

To quantify the amount of drug present at each time point, hydrogel films were removed from each patch (n = 3) at 0, 1, 3 and 6 months and dissolved in 5 mL 80% v/v acetonitrile for 16 hours at 30 °C in an Elmasonic S 30 H sonicating water bath (Elma Schmidbauer, Singen, Germany). After 16 hours, the samples were filtered using 0.2 $\mu$m Whatman syringeless filters vials with nylon filter media and polypropylene housing (GE Healthcare UK Ltd., Little Chalfont, Buckinghamshire, UK) and injected in triplicate to the HPLC. Results were then compared with the stated percentage drug content (10.89 % w/w for NTX base and 7.65% w/w for NTX.HCl) in order to determine the drug content uniformity using the HPLC method.

In order to assess the migration of drug from the hydrogel to other patch components, at t = 6 months, the drug content in all patch components (hydrogel, Blenderm™ medical tape, CoTran™ 9702 rate-controlling membrane and Scotchpak™ 9755 release liner) was quantified by HPLC. The same method as above was followed whereby each component was dissolved in 5 mL 80% v/v acetonitrile for 16 hours, filtered and injected in triplicate to the HPLC.

*Microbiology*

Mikrocount® duo dipslides (Schülke & Mayr GmbH, Norderstedt, Germany) were used to determine total plate count and to detect presence of moulds and yeasts. The surface of each side of the dipslide was placed in contact with the hydrogel surface of the patch for 5 seconds. The dipslides were then placed in their sealed container and incubated for 72 hours at 27 $\pm$ 1 °C. Once incubated, the dipslides were compared to images within the mikrocount® brochure to evaluate the colony forming units per cm$^2$.

*Statistical analysis*

One-way ANOVA was used to determine statistically significant differences between initial and subsequent time points, taking into account the different storage conditions. Statistically analysis was conducted using SPSS version 24 (SPSS UK Ltd, IBM, Woking, UK). Post-hoc analysis was achieved using either Scheffe's or Games-Howell test, depending on whether criteria of normality and homogeneity were met or compromised.

**Results**

*Uniformity of weight of hydrogels*

The xerogel films (n = 30) were weighed before swelling in order to determine the stated drug content based on the drug loading being 10.89 % w/w for naltrexone base films and 7.65% w/w for naltrexone hydrochloride films. The average weight of the films had a low standard deviation value for all films, meaning that each film was of a uniform weight. Results may be observed in Table 8.

**Table 8:** Uniformity of weight of xerogel films before making into patches, in order to determine the amount of drug in each film (n = 30)

| Sample | Unmedicated | NTX base | NTX.HCl |
|---|---|---|---|
| Average weight of film (mg) ($\pm$ SD) | 12.328 ($\pm$ 0.226) | 14.458 ($\pm$ 0.232) | 20.283 ($\pm$ 0.465) |
| Amount of drug in each film (mg) ($\pm$ SD) | N/A | 1.574 ($\pm$ 0.025) | 1.552 ($\pm$ 0.036) |

*Macroscopic appearance*

Pouches at all time points were sealed well and no holes or perforations were observed on the pouch surface. The pouches were then cut open with scissors and the patches removed. The unmedicated (control) patches consisted of a white, translucent hydrogel sealed between CoTran™ membrane and Blenderm™ medical tape backing. Both naltrexone base and naltrexone hydrochloride patches consisted of a yellow, translucent hydrogel sealed between the membrane and backing. All patches appeared to be homogenous at all time points, no inconsistencies were observed macroscopically.

*pH*

The surface pH of all hydrogel patches was found to be within the range of 5.46-6.78. Changes in pH were not significant until t = 6 month time point, however the actual variation was a maximum of 0.04 pH units and therefore still indicated good stability with regards to pH.

*FTIR*

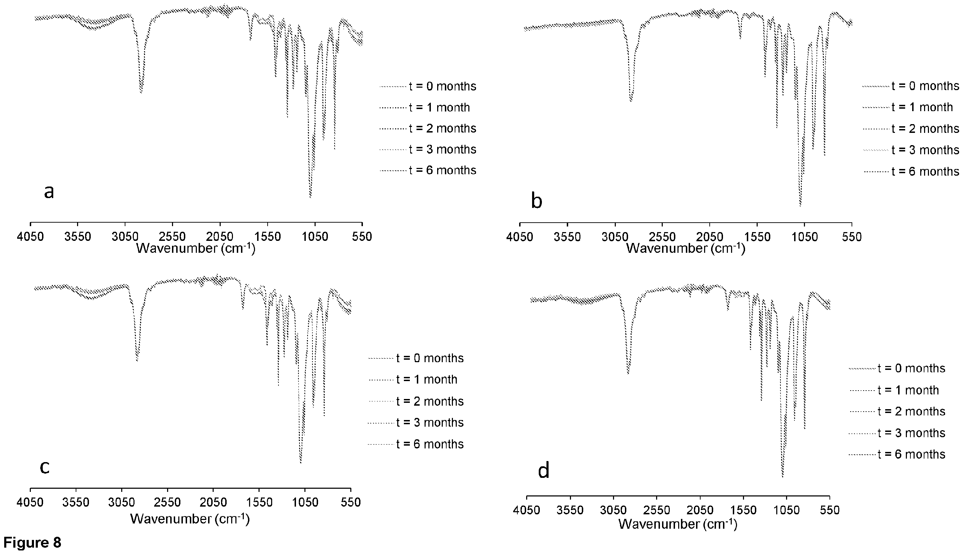

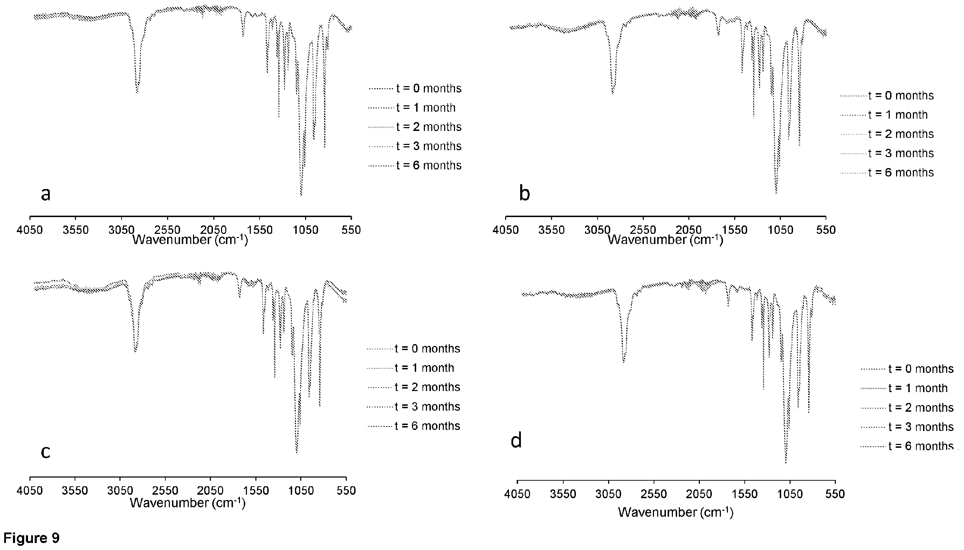

FTIR results are provided in Figures 8 to 15. The FTIR spectra of the hydrogels in Figures 8 and 9 showed no difference in the characteristic peaks of naltrexone. For all samples, the ketone carbonyl stretch remained at 1729 cm$^{-1}$ and the CH$_2$ stretch for poly(ethylene) oxide remained constant in all samples at a wavenumber of 2882 cm$^{-1}$, which suggested no change in the degree of drug-polymer interactions.

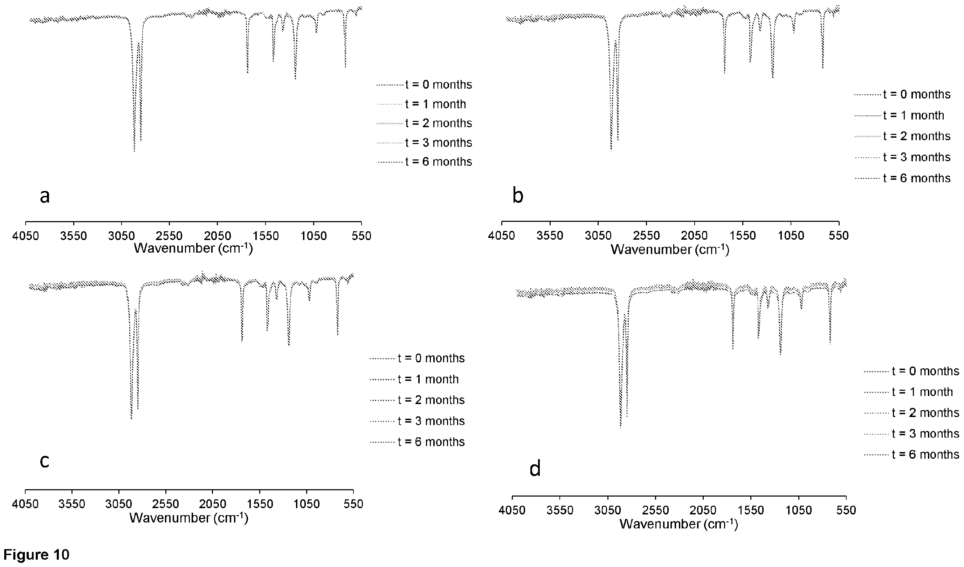

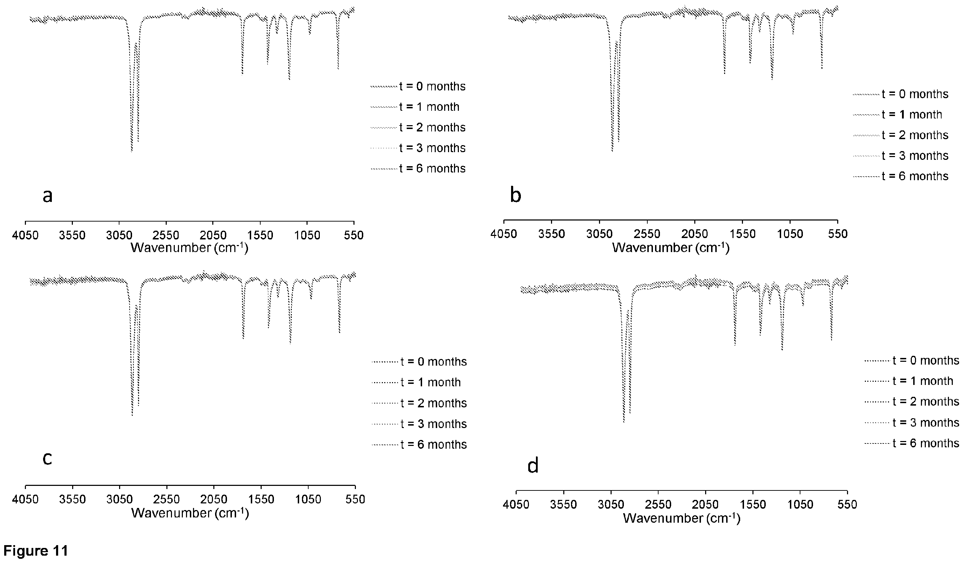

Figures 10 and 11 show the FTIR spectra for the CoTran membrane under ambient and accelerated conditions, respectively. For the samples swollen in 25% v/v propylene glycol (c and d), the C=O peak at 1740 cm$^{-1}$ were more intense as the storage time period increased suggesting the presence of some naltrexone by the 6 month time point, as confirmed by the mass balance study where between 4.83 - 8.69 % naltrexone was quantified in the CoTran membrane.

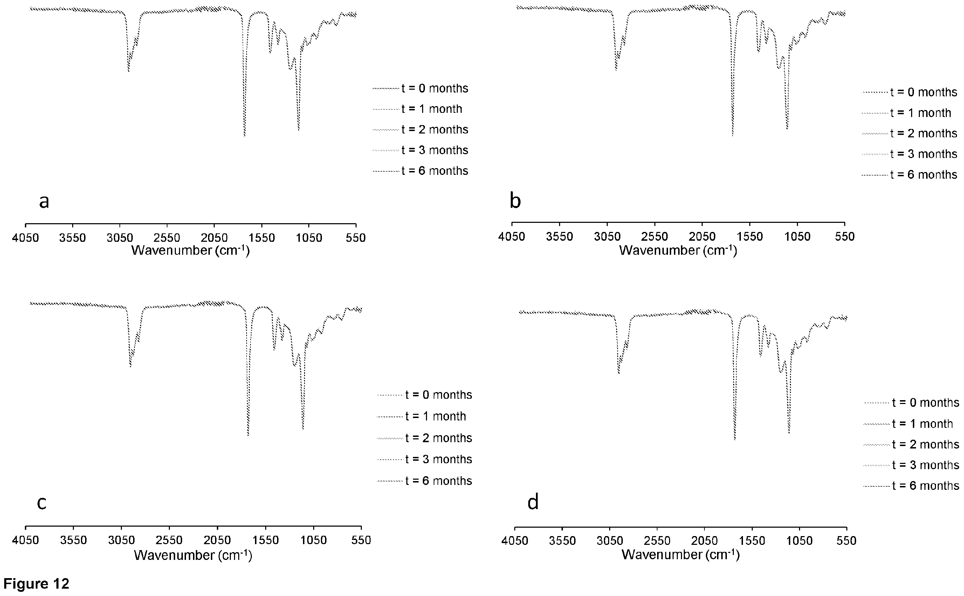

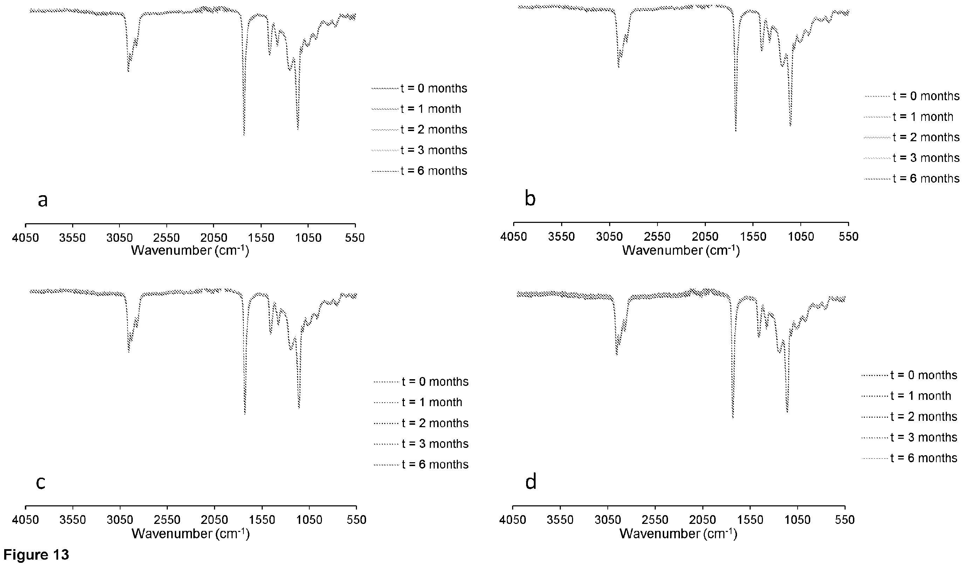

Figures 12 and 13 show the FTIR spectra for the adhesive side of the Blenderm medical tape, which had been in direct contact with the drug-loaded hydrogel through the storage period. The wavenumber for the carbonyl group of the Blenderm was identical for all samples at 1726 cm$^{-1}$. However, it was noticed that on Figure 13b and d (naltrexone base film and naltrexone hydrochloride film swollen in 25% v/v propylene glycol under accelerated conditions) that the intensity of the C=O stretch increased slightly suggesting the presence of some naltrexone molecules on the surface of the film. This was likely as observed in the mass balance study as part of drug content analysis where between 0.64 - 0.66 % naltrexone was quantified in the Blenderm medical tape at t = 6 months.

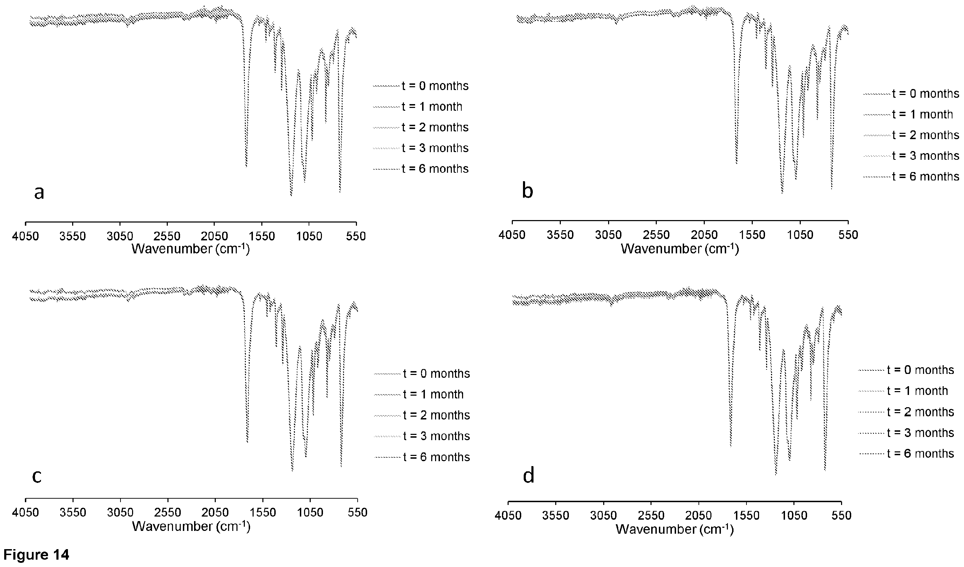

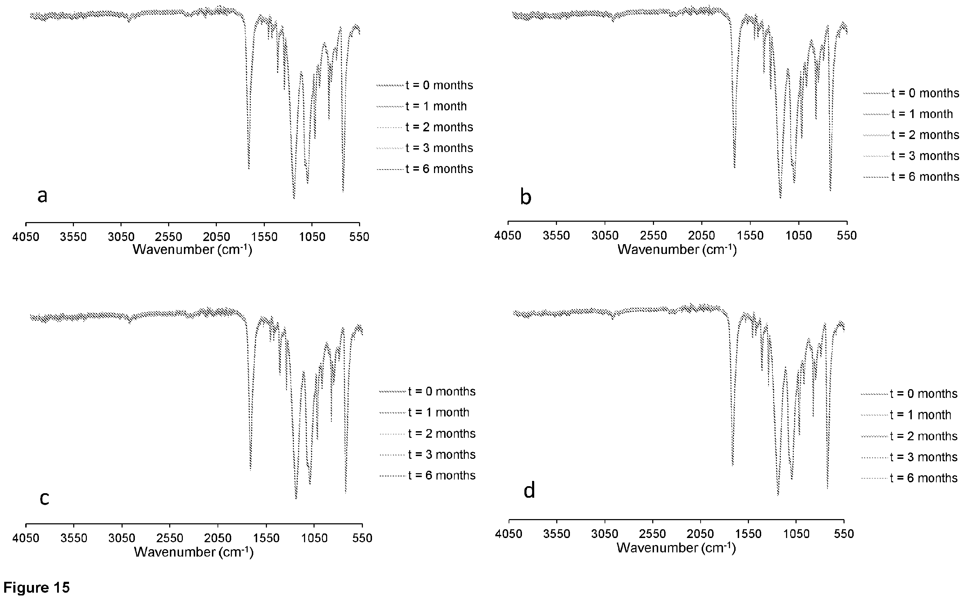

Figures 14 and 15 show the FTIR spectra for the Scotchpak release liner. The C=O stretch remained at 1728 cm-1 for all samples at all time points, and remained the same intensity. This suggested no naltrexone had migrated to the release liner, which was confirmed by the mass balance study.

The FTIR data also confirms that there was no noticeable drug decomposition over the 6 month study period, there was just modest migration from the hydrogel to into the CoTran membrane, which would not undermine drug release from the patch.

*Differential scanning calorimetry*

DSC data is provided in Table 9. At no time point were crystals present for the naltrexone base films. The opposite was true for naltrexone hydrochloride whereby there were crystals present from t = 0 months. This was found to increase after 3 and 6 months. The crystallisation of naltrexone hydrochloride in films swollen in water increased from 2.80 - 6.44 % and 2.80 - 6.87 % for ambient and accelerated conditions, respectively. There was an insignificant increase in percentage crystallisation between ambient and accelerated conditions (p > 0.05). This suggested that the packaging was sufficient to protect the hydrogels from temperature and humidity. The crystallisation in films swollen in 25% v/v propylene glycol increased insignificantly from 2.77 - 4.21 % and 2.77 -5.77 % for ambient and accelerated conditions, respectively. Comparing the swelling solutions, it was therefore shown that propylene glycol has crystallisation inhibition properties and acts to slow down the growth of crystals over time compared to pure water.

**Table 9:** Differential scanning calorimetry parameters for all NTX base and NTX.HCl patches after storage under ambient and accelerated conditions (t = 0, 3 and 6 months)

| Hydrogel type | Storage condition | Swelling solution | Timepoint (months) (months) | Average DSC parameters (n = 3) (± SD) | | | | | |
|---|---|---|---|---|---|---|---|---|---|
| | | | | $H_f$ drug (J/g) | (%) $X_D$ drug (%) | 1st $T_m$ 1st heat NTX (°C) | 1st $T_m$ 1st heat PEO (°C) | 2nd heat PEO $T_m$ (°C) | (°C) $T_g$ (°C) |
| NTX base | Ambient | Water | 0 | N/A | N/A | N/A | 68.93 (± 0.73) | 65.34 (± 0.59) | -45.60 (± 0.55) |
| | | | 3 | N/A | N/A | N/A | 68.88 (± 0.40) | 65.10 (± 0.66) | -45.55 (± 0.17) |
| | | | 6 | N/A | N/A | N/A | 68.61 (± 2.21) | 65.00 (± 2.96) | -45.59 (± 1.69) |
| | | PG | 0 | N/A | N/A | N/A | 68.52 (± 0.46) | 65.13 (± 0.57) | -45.16 (± 0.06) |
| | | | 3 | N/A | N/A | N/A | 68.46 (± 0.38) | 64.57 (± 0.63) | -45.17 (± 0.42) |
| | | | 6 | N/A | N/A | N/A | 67.76 (± 0.95) | 63.64 (± 1.19) | -44.85 (± 0.59) |
| | Accelerated | Water | 0 | N/A | N/A | N/A | 68.93 (± 0.73) | 65.34 (± 0.59) | -45.60 (± 0.55) |
| | | | 3 | N/A | N/A | N/A | 68.61 (± 0.85) | 64.41 (± 0.27) | -45.50 (± 0.44) |
| | | | 6 | N/A | N/A | N/A | 66.55 (± 1.61) | 64.36 (± 1.01) | -44.81 (± 1.45) |
| | | PG | 0 | N/A | N/A | N/A | 68.52 (± 0.46) | 65.13 (± 0.57) | -45.16 (± 0.06) |
| | | | 3 | N/A | N/A | N/A | 68.01 (± 1.88) | 63.72 (± 0.43) * | -44.92 (± 0.43) |
| | | | 6 | N/A | N/A | N/A | 67.68 (± 2.27) | 63.30 (± 0.49) * | -44.43 (± 1.56) |

(continued)

| | | | | Average DSC parameters (n = 3) (± SD) | | | | | |
|---|---|---|---|---|---|---|---|---|---|
| Hydrogel type | Storage condition | Swelling solution | Timepoint (months) | $H_f$ drug (J/g) | $X_D$ drug (%) | 1st $T_m$ 1st heat NTX (°C) | 1st $T_m$ 1st heat PEO (°C) | 2nd heat PEO $T_m$ (°C) | $T_g$ (°C) |
| NTX HCl | Ambient | Water | 0 | 5.422 (± 1.27) | 2.80 (± 0.66) | N/D | 69.76 (± 0.38) | 65.74 (± 0.62) | -52.81 ± (2.50) |
| | | | 3 | 12.120 (± 0.74) * | 6.27 (± 0.38) * | N/D | 68.53 (± 1.00) | 65.37 (± 0.50) | -52.07 (± 0.74) |
| | | | 6 | 12.463 (± 0.82) * | 6.44 (± 0.43) * | N/D | 68.25 (± 1.81) | 65.07 (± 0.17) | -50.52 (± 1.26) |
| | | PG | 0 | 5.348 (± 0.25) | 2.77 (± 0.13) | N/D | 69.65 (± 0.43) | 65.74 (± 0.21) | -52.19 (± 0.75) |
| | | | 3 | 7.375 (± 2.08) | 3.81 (± 1.08) | N/D | 69.24 (± 1.13) | 65.64 (± 1.03) | -51.09 (± 0.36) |
| | | | 6 | 8.139 (± 0.98) | 4.21 (± 0.51) | N/D | 69.08 (± 0.60) | 65.33 (± 0.17) | -49.14 (± 1.28) * |
| | Accelerated | Water | 0 | 5.422 (± 1.27) | 2.80 (± 0.66) | N/D | 69.76 (± 0.38) | 65.74 (± 0.62) | -52.81 (± 2.50) |
| | | | 3 | 12.337 (± 0.90) * | 6.38 (± 0.46) * | N/D | 69.56 (± 0.79) | 67.39 (± 0.81) | -51.67 (± 0.46) |
| | | | 6 | 13.283 (± 1.07) * | 6.87 (± 0.56) * | N/D | 66.82 (± 0.86) * | 65.36 (± 0.21) | -49.77 (± 0.30) |
| | | PG | 0 | 5.348 (± 0.25) | 2.77 (± 0.13) | N/D | 70.37 (± 0.58) | 66.58 (± 1.70) | -52.19 (± 0.75) |
| | | | 3 | 9.748 (± 0.23) * | 5.04 (± 0.12) * | N/D | 69.65 (± 0.43) | 65.74 (± 0.21) | -50.84 (± 0.60) |
| | | | 6 | 11.160 (± 0.04) * | 5.77 (± 0.02) * | N/D | 67.81 (± 1.63) | 65.41 (± 0.37) | -48.28 (± 1.85) * |

*Mechanical testing*

The mechanical testing data can be observed in Table 10. Over a storage period of 6 months, the tensile strength and Young's modulus of the hydrogel patches were found to decrease. Significant changes were noticed in the tensile strength, Young's modulus and percentage elongation on breakage for the patches made with 25% v/v propylene glycol. This was likely due to the weakening effect propylene glycol has on materials by increasing the permeability due to diffusing faster than the drug. The accelerated conditions (increased temperature and stability) were also found to have a reduction effect on the mechanical properties of the patches compared to the ambient conditions. This was likely due to the propylene glycol causing increase in permeability to be more pronounced at higher temperatures. Although a lot of the data are significantly different to the initial time point, the patches retain good tensile strength and good Young's modulus and therefore are relatively physically stable during storage. Consequently, the patches remain flexible enough to conform to movement of skin and remain strong enough even after storage.

**Table 10:** Mechanical properties for all NTX base and NTX.HCl patches after storage under ambient and accelerated conditions (t = 0 and 6 months) *statistically significantly different from t = 0 months data (p < 0.05)

| Storage condition | Hydrogel type | Swelling solution | Time point (months) | Mechanical property (n=3) ($\pm$ SD) | | |
| --- | --- | --- | --- | --- | --- | --- |
| | | | | Tensile strength (MPa) | Young's modulus (MPa) | Percentage total elongation at break (%) |
| Ambient | Unmedicated | Water | 0 | 9.464 ($\pm$ 0.49) | 12.684 ($\pm$ 0.53) | 129.09 ($\pm$ 3.13) |
| | | | 6 | 9.374 ($\pm$ 0.16) | 12.515 ($\pm$ 0.03) | 126.28 ($\pm$ 2.62) |
| | | PG | 0 | 9.445 ($\pm$ 0.26) | 12.614 ($\pm$ 0.26) | 126.39 ($\pm$ 2.71) |
| | | | 6 | 9.183 ($\pm$ 0.78) | 11.596 ($\pm$0.41) * | 121.30 ($\pm$ 2.05) |
| | NTX base | Water | 0 | 9.029 ($\pm$ 0.12) | 12.147 ($\pm$ 0.30) | 124.10 ($\pm$ 4.01) |
| | | | 6 | 8.916 ($\pm$ 0.10) | 12.089 ($\pm$ 0.06) | 121.99 ($\pm$ 0.98) |
| | | PG | 0 | 8.995 ($\pm$ 0.13) | 12.012 ($\pm$ 0.05) | 123.90 ($\pm$ 2.65) |
| | | | 6 | 8.718 ($\pm$ 0.24) | 11.595 ($\pm$ 0.50) * | 120.37 ($\pm$ 1.86) |
| | NTX.HCl | Water | 0 | 8.917 ($\pm$ 0.12) | 11.941 ($\pm$ 0.09) | 123.45 ($\pm$ 2.84) |
| | | | 6 | 8.744 ($\pm$ 0.12) | 11.710 ($\pm$ 0.14) | 120.72 ($\pm$ 0.89 |
| | | PG | 0 | 8.900 ($\pm$ 0.11) | 11.912 ($\pm$ 0.05) | 123.25 ($\pm$ 0.88) |
| | | | 6 | 8.509 ($\pm$ 0.04) * | 10.876 ($\pm$ 0.26) * | 120.14 ($\pm$ 0.75) * |

(continued)

| Storage condition | Hydrogel type | Swelling solution | Time point (months) | Mechanical property (n=3) (± SD) | | |
|---|---|---|---|---|---|---|
| | | | | Tensile strength (MPa) | Young's modulus (MPa) | Percentage total elongation at break (%) |
| Accelerated | Unmedicated | Water | 0 | 9.464 (± 0.49) | 12.684 (± 0.53) | 129.09 (± 3.13) |
| | | | 6 | 9.155 (± 0.11) | 12.378 (± 0.03) | 125.29 (± 2.45) |
| | | PG | 0 | 9.445 (± 0.26) | 12.614 (± 0.26) | 126.39 (± 2.71) |
| | | | 6 | 8.974 (± 0.47) | 11.377 (± 0.25) * | 121.21 (± 1.95) |
| | NTX base | Water | 0 | 9.029 (± 0.12) | 12.147 (± 0.30) | 124.10 (± 4.01) |
| | | | 6 | 8.775 (± 0.23) | 11.888 (± 0.21) | 120.75 (± 0.53) |
| | | PG | 0 | 8.995 (± 0.13) | 12.012 (± 0.05) | 123.90 (± 2.65) |
| | | | 6 | 8.190 (± 0.21) * | 11.203 (± 0.21) * | 118.69 (± 1.02) * |
| | NTX.HCl | Water | 0 | 8.917 (± 0.12) | 11.941 (± 0.09) | 123.45 (± 2.84) |
| | | | 6 | 8.535 (± 0.11) * | 11.473 (± 0.21) * | 119.74 (± 0.87) |
| | | PG | 0 | 8.900 (± 0.11) | 11.912 (± 0.05) | 123.25 (± 0.88) |
| | | | 6 | 7.807 (± 0.25) * | 10.488 (± 0.21) * | 118.53 (± 0.91) * |

*90° peel test*

The adhesion data transdermal patches before and after storage can be found in Table 11. Ambient storage conditions were found to cause an insignificant decrease in the peel force. Conversely, accelerated storage conditions were found to cause a significant decrease in the peel force for all patches, irrespective of their swelling solution or drug type. This means that it requires less force to remove the adhesive tape from the stainless steel testing plate. There was no sign of cohesive failure whereby a residue would be present on the peel table.

**Table 11:** Adhesion properties for all NTX base and NTX.HCl patches after storage under ambient and accelerated conditions (t = 0 and 6 months) *statistically significantly different from t = 0 months data ($p < 0.05$)

| Storage condition | Hydrogel type | Swelling solution | Peel force (n = 3) (± SD) | |
|---|---|---|---|---|
| | | | 0 | 6 |
| Ambient | Unmedicated | Water | 5.0657 (± 0.15) | 4.8577 (± 0.37) |
| | | PG | 5.0581 (± 0.07) | 4.8983 (± 0.25) |
| | NTX base | Water | 5.0661 (± 0.05) | 4.7284 (± 0.35) |
| | | PG | 5.0677 (± 0.01) | 4.8871 (± 0.64) |
| | NTX HCl | Water | 5.0767 (± 0.07) | 4.8983 (± 0.25) |
| | | PG | 5.0782 (± 0.10) | 4.7029 (± 0.15) |

(continued)

| Storage condition | Hydrogel type | Swelling solution | Peel force (n = 3) ($\pm$ SD) | |
| --- | --- | --- | --- | --- |
| | | | 0 | 6 |
| Accelerated | Unmedicated | Water | 5.0657 ($\pm$ 0.15) | 3.6043 ($\pm$ 0.27) * |
| | | PG | 5.0581 ($\pm$ 0.07) | 6.5107 ($\pm$ 0.08) * |
| | NTX base | Water | 5.0661($\pm$ 0.05) | 3.4590 ($\pm$ 0.30) * |
| | | PG | 5.0677 ($\pm$ 0.01) | 3.4339 ($\pm$ 0.21) * |
| | NTX HCl | Water | 5.0767 ($\pm$ 0.07) | 3.4399 ($\pm$ 0.22) * |
| | | PG | 5.0782 ($\pm$ 0.10) | 3.3390 ($\pm$ 0.07) * |

*Drug content analysis*

Drug content analysis of hydrogel results obtained after 0, 1, 3 and 6 months storage under either ambient or accelerated conditions may be observed in Tables 12 and 13 for naltrexone base and naltrexone hydrochloride loaded films respectively. There were significant changes in drug content of naltrexone base and naltrexone hydrochloride for all 6 month time points. The drug content of the optimised transdermal patches was found to decrease over time, irrespective of the storage conditions and swelling conditions. However, it was clear from the data that the percentage stated content decreased at a faster rate when the films had been swollen in 25% v/v propylene glycol and had been stored under accelerated conditions. A single peak for naltrexone was observed on all chromatograms showing that naltrexone did not degrade under storage into any other compounds. Therefore, it was hypothesised that the drug was diffusing out of the hydrogel into other components of the patch. This hypothesis led to a different approach for the t = 6 month data time point whereby all patch components were dissolved and drug content was quantified in order to confirm any migration of drug molecules.

Table 14 shows the mass balance data from all patch components at t = 6 months. It can be observed that close to 100% of the loaded drug is accounted for across all patch components. As expected, the hydrogel films contained the largest proportion of drug, followed by the CoTran membrane. It is beneficial to have drug diffuse towards the CoTran membrane so that when the patch is applied to a patient, drug release can begin instantaneously, reducing the lag time. The percentage of drug in the CoTran was highest for the patches that had been swollen in 25% v/v propylene glycol. This was because propylene glycol is a permeation enhancer which has a lower molecular weight than naltrexone (76.09 gmol$^{-1}$ compared to 341.40 gmol$^{-1}$) and therefore diffused out of the hydrogel faster than the drug, disrupting the structure of the CoTran membrane making it more permeable than it was previously. This resulted in the permeation of some naltrexone molecules to the CoTran membrane.

A very low percentage (0.21 - 0.67 %) of naltrexone was found in the Blenderm medical tape. Although it is not ideal for any drug to be present in an area of the patch that won't be in contact with the patient's skin, the percentage of drug present in the Blenderm was negligible after 6 months storage and therefore unlikely to affect the performance of the patch.

Based on the drug content of the hydrogels only, a shelf life may be predicted by extrapolating the data and calculating how long it would take to reach 90% of the stated dose. This data may be observed in Table 15. The shelf life is likely to be longer as the drug content in the CoTran membrane should be taken into account as this is useful as it is available for drug release, however the drug in the Blenderm is not available for drug release.

**Table 12:** Average percentage of stated content (10.89% w/w) NTX base films after storage under ambient and accelerated conditions (t = 0, 1, 2 and 6 months) *statistically significantly different from t = 0 months data (p < 0.05).

| Storage condition | Swelling solution | Average percentage of stated content (%) (n = 3) ($\pm$ SD) | | | |
| --- | --- | --- | --- | --- | --- |
| | | 0 | 1 | 2 | 6 |
| Ambient | Water | 99.54 ($\pm$ 0.83) | 99.00 ($\pm$ 0.38) | 98.75 ($\pm$ 0.88) | 93.91 ($\pm$ 0.15)* |
| | 25% v/v PG | 99.64 ($\pm$ 0.45) | 99.41 ($\pm$ 0.50) | 98.89 ($\pm$ 0.33) | 92.94 ($\pm$ 0.56)* |
| Accelerated | Water | 99.54 ($\pm$ 0.83) | 99.50 ($\pm$ 0.25) | 97.89 ($\pm$ 1.06) | 92.85 ($\pm$ 0.87)* |
| | 25% v/v PG | 99.64 ($\pm$ 0.45) | 99.55 ($\pm$ 1.00) | 97.62 ($\pm$ 0.59)* | 89.95 ($\pm$ 0.42)* |

**Table 13:** Average percentage of stated content (7.65% w/w) NTX.HCl films after storage under ambient and accelerated conditions (t = 0, 1, 2 and 6 months) *statistically significantly different from t = 0 months data (p < 0.05)

| Storage condition | Swelling solution | Average percentage of stated content (%) (n = 3) ($\pm$ SD) | | | |
|---|---|---|---|---|---|
| | | 0 | 1 | 2 | 6 |
| Ambient | Water | 100.20 ($\pm$ 0.84) | 99.30 ($\pm$ 0.45) | 98.28 ($\pm$ 1.00) | 94.59 ($\pm$ 1.08) * |
| | 25% v/v PG | 99.72 ($\pm$ 0.08) | 99.23 ($\pm$ 1.23) | 98.72 ($\pm$ 0.72) | 91.33 ($\pm$ 0.54) * |
| Accelerate d | Water | 100.20 ($\pm$ 0.84) | 98.70 ($\pm$ 0.26) | 98.13 ($\pm$ 0.98) | 94.30 ($\pm$ 0.64) * |
| | 25% v/v PG | 99.72 ($\pm$ 0.08) | 98.47 ($\pm$ 0.29) * | 98.15 ($\pm$ 0.27) * | 90.04 ($\pm$ 0.52) * |

**Table 14:** Mass balance to determine migration of drug from hydrogel film to other patch components (adhesive backing, release membrane and temporary release liner)

| Sample | | Drug in film (%) | Drug in adhesive backing (%) | Drug in release membrane (%) | Drug in temporary release liner (%) | Mass balance (%) | Average mass balance (%) | Std dev |
|---|---|---|---|---|---|---|---|---|
| NTX base, Water, Amb | 1 | 93.83 | 0.00 | 5.14 | 0.00 | 98.98 | | |
| | 2 | 94.08 | 0.00 | 4.93 | 0.00 | 99.01 | 99.13 | 0.24 |
| | 3 | 93.81 | 0.00 | 5.61 | 0.00 | 99.42 | | |
| NTX base, Water, Acc | 1 | 93.19 | 0.00 | 4.83 | 0.00 | 98.02 | | |
| | 2 | 91.86 | 0.00 | 5.48 | 0.00 | 97.35 | 98.04 | 0.71 |
| | 3 | 93.50 | 0.00 | 5.27 | 0.00 | 98.77 | | |
| NTX base, PG, Amb | 1 | 92.41 | 0.26 | 7.07 | 0.00 | 99.74 | | |
| | 2 | 92.87 | 0.22 | 6.35 | 0.00 | 99.44 | 99.95 | 0.64 |
| | 3 | 93.53 | 0.22 | 6.91 | 0.00 | 100.66 | | |
| NTX base, PG, Acc | 1 | 89.78 | 0.66 | 7.07 | 0.00 | 97.51 | | |
| | 2 | 89.66 | 0.66 | 6.99 | 0.00 | 97.31 | 97.72 | 0.55 |
| | 3 | 90.43 | 0.67 | 7.24 | 0.00 | 98.34 | | |
| NTX HCL, Water, Amb | 1 | 95.77 | 0.00 | 5.13 | 0.00 | 100.90 | | |
| | 2 | 93.65 | 0.00 | 5.77 | 0.00 | 99.42 | 99.86 | 0.90 |
| | 3 | 94.35 | 0.00 | 4.92 | 0.00 | 99.27 | | |
| NTX HCL, Water, Acc | 1 | 94.43 | 0.00 | 4.85 | 0.00 | 99.27 | | |
| | 2 | 94.87 | 0.00 | 5.00 | 0.00 | 99.87 | 99.45 | 0.36 |
| | 3 | 93.61 | 0.00 | 5.61 | 0.00 | 99.21 | | |
| NTX HCL, PG, Amb | 1 | 90.90 | 0.22 | 7.99 | 0.00 | 99.11 | | |
| | 2 | 91.94 | 0.22 | 8.69 | 0.00 | 100.85 | 99.99 | 0.87 |
| | 3 | 91.17 | 0.21 | 8.64 | 0.00 | 100.02 | | |
| NTX HCL, PG, Acc | 1 | 90.40 | 0.64 | 7.57 | 0.00 | 98.60 | | |
| | 2 | 89.44 | 0.64 | 7.49 | 0.00 | 97.57 | 98.23 | 0.58 |
| | 3 | 90.27 | 0.65 | 7.62 | 0.00 | 98.53 | | |

**Table 15:** Estimated shelf life of patches based on drug content over 6 months of the hydrogel

| Sample | Estimated shelf life (months) |
|---|---|
| NTX base water ambient | 10.61 |
| NTX base water accelerated | 8.40 |
| NTX base PG ambient | 8.77 |

(continued)

| Sample | Estimated shelf life (months) |
|---|---|
| NTX base PG accelerated | 5.81 |
| NTX.HCL water ambient | 11.42 |
| NTX.HCL water accelerated | 11.30 |
| NTX.HCL PG ambient | 7.07 |
| NTX.HCL PG accelerated | 6.22 |

*Microbiology*

Two interesting observations were noticed during the study. Firstly, the unmedicated films that had been swollen in water had grown colonies of bacteria after 2 months under ambient and accelerated conditions, whereas the medicated films did not grow any microbes. This may be attributed to the irradiation time used to crosslink the films before formulating to patches. The unmedicated films only required 10 minutes to optimise the crosslinking, whereas the medicated films required 15 minutes. It could be possible that UV irradiation acts as a steriliser for the films and was more successful after irradiating for a longer period of time due to increased UV exposure. On the yeast and mould side of the mikrocount duo dipslide, the only sample which showed growth was the 6 month unmedicated swollen in water and stored under accelerated conditions patch. This was likely due to the increased presence of non-sterile water in the patch and storage chamber.

The second observation to note was how the presence of propylene glycol prevented bacteria, yeast and mould from growing at all throughout the study even under accelerated conditions.

The above results suggest that naltrexone slows down the growth of microbes. This was also evidenced in a small scale study in which the microbial growth in solutions of naltrexone base and naltrexone hydrochloride were compared to a control after being stored for 25 days at 27°C. After 24 hours, the control sample displayed some growth of bacterial colonies, while no bacterial growth was observed in either of the naltrexone slides. After 48 hours, the bacterial colony count increased to approximately $10^4$ CFU/mL in the control sample and to approximately $10^2$ CFU/mL in the NTX base sample. After 72 hours, further bacterial growth was observed on the NTX base dipslide. Negligible bacterial growth was detected on the NTX.HCl dipslide after 72 hours. After 3 weeks incubation time, the bacterial colony count increased to ~$10^5$ CFU/mL on the control sample, ~$10^3$ CFU/mL for naltrexone base and ~$10^2$ CFU/mL for naltrexone hydrochloride. Thus, a longer incubation time was required for growth of microbial colonies in the medicated samples. No yeasts or moulds were detected at any incubation time points, suggesting naltrexone, particularly at high aqueous solubility, slowed down the growth of bacteria.

Accordingly, microbial growth was abundant for the control (water) (approximately $10^5$ CFU/mL), while microbes did not grow to such an extent in the solutions containing naltrexone base (approximately $10^3$ CFU/mL) and naltrexone hydrochloride (approximately $10^2$ CFU/mL). Thus, the presence of naltrexone appeared to delay the growth of bacteria, particularly for naltrexone hydrochloride in which the aqueous solubility is higher (90 mg/mL compared to 0.51 mg/mL for naltrexone base).

Example 11: Effect of different molecular weights of poly(ethylene oxide) on the hydrogel network

The mechanical and swelling properties of unmedicated PEO hydrogels comprising either PEO 1,000,000 or PEO 5,000,000 and different concentrations of PETRA were assessed in accordance with the methods set out in the "Assays" part of the present disclosure.

**Table 16:** Effect of PEO molecular weight and concentration of PETRA on the properties of unmedicated PEO hydrogels.

| PEO Mw (g/mol) | 1,000,000 | | | | 5,000,000 | | |
|---|---|---|---|---|---|---|---|
| PETRA Conc (%w/w) | 10 | 5 | 2.5 | 1 | 1 | 5 | 10 |
| Solid Content (%w/v) | 8 | 8 | 8 | 8 | 8 | 8 | 8 |
| Irradiation Time (mins) | 15 | 15 | 15 | 15 | 15 | 15 | 15 |
| Thickness of casted film (μm) | 250 | 200 | 200 | 230 | 250 | 320 | 250 |

(continued)

| PEO Mw (g/mol) | 1,000,000 | | | | 5,000,000 | | |
|---|---|---|---|---|---|---|---|
| Thickness of swollen film ($\mu$m) | 320 | 320 | 350 | 470 | 430 | 430 | 320 |
| Total thickness Increased ($\mu$m) | 70 | 120 | 150 | 240 | 220 | 110 | 70 |
| Equilibrium Swelling Time | 5h | 5h | 24 h | 48 h | 90 min | 5 h | 5h |
| Degree of Swelling (%) | 130.21 ($\pm$12.78) | 199.82 ($\pm$9.24) | 336.02 ($\pm$16.93) | 598.60 ($\pm$23.29) | 523.98 ($\pm$18.11) | 244.44 ($\pm$9.87) | 128.91 ($\pm$3.91) |
| Water Content (%) | 56.56 ($\pm$0.53) | 66.84 ($\pm$1.30) | 77.04 ($\pm$0.87) | 85.67 ($\pm$0.48) | 83.963 ($\pm$0.47) | 75.51 ($\pm$6.77) | 56.30 ($\pm$0.74) |
| Gel Fraction (%) | 89.47 ($\pm$0.36) | 85.84 ($\pm$1.65) | 79.69 ($\pm$2.40) | 67.02 ($\pm$1.381) | 75.46 ($\pm$1.87) | 82.77 ($\pm$2.84) | 84.66 ($\pm$6.56) |
| Tensile Strength (N/mm$^2$) | 0.77 | 1.11 | 0.20 | 0.11 | 0.10 | 0.13 | 0.25 |
| Youngs Modulus, E (KPa) | 84.55 | 41.06 | 11.22 | 2.33 | 3.06 | 16.80 | 48.43 |
| Percentage Elongation, $\varepsilon$ (%) | 9.13 | 27.00 | 18.28 | 47.28 | 33.07 | 7.52 | 5.10 |
| Average Molecular Weight Between Crosslinks, $\overline{M}_{c,s}$ (g/mol) | 323.30 ($\pm$11.87) | 734.48 ($\pm$62.36) | 2073.79 ($\pm$214.80) | 6643.65 ($\pm$516.21) | 5081.53 ($\pm$351.40) | 1092.87 ($\pm$87.69) | 316.18 ($\pm$17.86) |
| Average Molecular Weight Between Crosslinks, $\overline{M}_{c,T}$ (g/mol) | 143.27 | 326.03 | 1363.90 | 7790.30 | 5630.34 | 851.69 | 250.08 |
| Crosslink Density, $\rho_{c,S}$ (x 10$^{-4}$) | 38.39 ($\pm$2.71) | 16.45 ($\pm$1.56) | 5.84 ($\pm$0.57) | 1.79 ($\pm$0.15) | 2.37 ($\pm$0.17) | 11.04 ($\pm$0.89) | 38.05 ($\pm$2.10) |
| Crosslink Density, $\rho_{c,T}$ (x 10$^{-4}$) | 83.76 | 36.80 | 8.79 | 1.54 | 2.13 | 14.09 | 47.99 |
| Mesh Size From Swelling Studies, $\xi s$ (Å) | 1.50 ($\pm$0.04) | 2.57 ($\pm$0.14) | 4.92 ($\pm$0.32) | 10.57 ($\pm$0.72) | 8.70 ($\pm$0.89) | 3.28 ($\pm$0.17) | 1.45 ($\pm$0.15) |
| Mesh Size From Tensile Testing, $\xi_T$ (Å) | 1.03 | 1.71 | 4.01 | 11.49 | 9.16 | 2.95 | 1.35 |

Hydrogels having a molecular weight of PEO of 5,000,000 g/mol gave higher crosslink density, leading to higher gel fraction and lower degree of swelling. The high crosslinking made them more brittle and rigid as compared to the more elastic lower molecular weight (PEO 1,000,000 g/mol) hydrogels. This demonstrated by the lower values obtained for tensile strength, elastic modulus, and percentage of elongation. For hydrogels containing 5%w/w and 10%w/w PETRA, a slightly lower gel fraction was observed in the higher molecular weight hydrogel films due to the presence of cracking. 10%w/w of PETRA seems to be maximum plateau concentration in the formulation of PEO hydrogels as it gave similar values in the degree of swelling, water content, average molecular weight between crosslinks, crosslink density, and mesh size from the swelling studies for both PEO hydrogels of 1,000,000 g/mol and 5,000,000 g/mol.

However, the values for average molecular weight between crosslinks, crosslink density, and mesh size obtained from the tensile testing of higher molecular weight hydrogels are lower than those of lower molecular weight due to their lower tensile strength, elastic modulus, and percentage of elongation.

When the concentration of PETRA falls below 5%w/w, the 1,000,000 g/mol PEO hydrogels took longer than the usual 5 hours to reach equilibrium swelling. This is likely due to the lower crosslinking of the hydrogels, which is proven by the low gel fractions (67.02% for hydrogels containing 1%w/w PETRA, and 77.04% for hydrogels containing 2.5%w/w PETRA). Furthermore, hydrogels containing 1%w/w PETRA exhibited a total of 12.22% drop in weight from 90 minutes of swelling, while a total of 4.71% weight drop was observed from 5 hours of swelling for hydrogels containing 2.5%w/w PETRA. The swelling studies of all hydrogel batches were, however, carried out for 72 hours. This means that all hydrogels had reached the equilibrium of swelling.

References

ABD, E., YOUSEF, S. A., PASTORE, M. N., TELAPROLU, K., MOHAMMED, Y. H., NAMJOSHI, S., GRICE, J. E. & ROBERTS, M. S. 2016. Skin models for the testing of transdermal drugs. Clin Pharmacol, 8, 163-176.

AKHLAQ, M., ARSHAD, M. S., MUDDASSIR, A. M. & HUSSAIN, A. 2015. Formulation and evaluation of anti-rheumatic dexibuprofen transdermal patches: a quality-by-design approach. J Drug Target, 24, 1-27.

ALKILANI, A. Z., MCCRUDDEN, M. T. C. & DONNELLY, R. F. 2015. Transdermal Drug Delivery: Innovative Pharmaceutical Developments Based on Disruption of the Barrier Properties of the stratum corneum. Pharmaceutics, 7, 438-470.

ALVAREZ-FUENTES, J., CARABALLO, I., BOZA, A., LLERA, J. M., HOLGADO, M. A. & FERNANDEZ-AREVALO, M. 1997. Study of a complexation process between naltrexone and Eudragit® L as an oral controlled release system. Int J Pharm, 148, 219-230.

AM ENDE, M. T. & PEPPAS, N. A. 1997. Transport of ionizable drugs and proteins in crosslinked poly(acrylic acid) and poly(acrylic acid-co-2-hydroxyethyl methacrylate) hydrogels. II. Diffusion and release studies. J Control Release, 48, 47-56.

ANSETH, K. S., BOWMAN, C. N. & BRANNON-PEPPAS, L. 1996. Mechanical properties of hydrogels and their experimental determination. Biomaterials, 17, 1647-1657.

BANERJEE, S., CHATTOPADHYAY, P., GHOSH, A., BHATTACHARYA, S. S., KUNDU, A. & VEER, V. 2014. Accelerated stability testing of a transdermal patch composed of eserine and pralidoxime chloride for prophylaxis against ($\pm$)-anatoxin A poisoning. J Food Drug Anal, 22, 264-270.

BANKS, S. L., PINNINTI, R. R., GILLB, H. S., CROOKS, P. A., M.R., P. & STINCHCOMB, A. L. 2008. Flux across microneedle-treated skin is increased by increasing charge of naltrexone and naltrexol in vitro. Pharm Res, 25, 1677-1685.

BANSAL, S. S., KAUSHAL, A. M. & BANSAL, A. K. 2008. Co-relationship of physical stability of amorphous dispesions with enthalpy relaxation. Pharmazie, 63, 812-814.

BARIYA, S. H., GOHEL, M. C., MEHTA, T. A. & SHARMA, O. P. 2011. Microneedles: an emerging transdermal drug delivery system. J Pharm Pharmacol, 64, 11-29.

BEMIS HEALTHCARE LTD 2017. P616 white high barrier laminate with CXB sealant film data specification.

BIGLIARDI, P. L., STAMMER, H., JOST, G., RUFLI, T., BUCHNER, S. & BIGLIARDI-QI, M. 2007. Treatment of pruritus with topically applied opiate receptor antagonist. J Am Acad Dermatol, 56, 979-988.

BLU-EMU 2016. Blu-Emu to include first OTC Lidocaine Pain Relief Patch - Lidocare.

BUTSCHLI, J. 2017. Bemis Wins FPA Award for Lidocare Pouch Development. Healthcare Packaging.

CAMPBELL, V., MCGRATH, C. & CORRY, A. 2017. Low-dose naltrexone: a novel treatment for Hailey-Hailey disease. Br J Dermatol, 178, 1196-1198.

CAMPISI, G., GIANNOLA, L. I., FLORENA, A. M., DE CARO, V., SCHUMACHER, A., GOTTSCHE, T., PADERNI, C. & WOLFF, A. 2010. Bioavailability in vivo of naltrexone following transbuccal administration by an electronically controlled intraoral device: A trial on pigs. J Cont Rel, 145, 214-220.

CAYKARA, T. & INAM, R. 2004. Determination of average molecular weight between crosslinks and polymer-solvent interaction parameters of poly(acrylamide-g-ethylene diamine tetraacetic acid) polyelectrolyte hydrogels. J Appl Polym Sci, 91, 2168-2175.

CHENEVAS-PAULE, C., WOLFF, H.-M., ASHTON, M., SCHUBERT, M. & DODOU, K. 2017. Development of a Predictive Model for the Long-Term Stability Assessment of Drug-In-Adhesive Transdermal Films Using Polar Pressure-Sensitive Adhesives as Carrier/Matrix. J Pharm Sci, 106, 1293-1301.

CLEMENTS, R. 1963. Modern Chemical Discoveries, pp 22

CORDERY, S. F., HUSBANDS, S. M., BAILEY, C. P., GUY, R. H. & DELGADO-CHARRO, M. B. 2019. Simultaneous Transdermal Delivery of Buprenorphine Hydrochloride and Naltrexone Hydrochloride by Iontophoresis. Mol Pharm, 16, 2808-2816.

DEY, S. & MALGOPE, A. 2010. Preparation of carvedilol transdermal patch and the effect of propylene glycol on permeation. Int J Pharm Pharm Sci, 2, 137-143.

DODOU, K., ARMSTRONG, A., KELLY, I., WILKINSON, S., CARR, K., SHATTOCK, P. & WHITELEY, P. 2015. Ex vivo studied for the passive transdermal delivery of low-dose naltrexone from a cream; detection of naltrexone and its active metabolite, 6$\beta$-naltrexol, using a novel LC Q-ToF MS assay. Pharm Dev Technol, 20, 694-701.

DODOU, K. & SADDIQUE, W. 2012. Effect of manufacturing method on the in vitro drug release and adhesive performance of drug-in-adhesive films containing binary mixtures of ibuprofen with polozamer 188. Pharm Dev Technol, 17, 552-561.

DOYTCHEVA, M., DOTCHEVA, D., STAMENOVA, R. & TSVETANOV, C. 2001. UV-initiated crosslinking of poly(ethylene oxide) with pentaerythritol triacrylate in solid state. Macromol Mater Eng, 286, 30-33.

EUROPEAN MEDICINES AGENCY. 2014. Guideline on quality of transdermal patches [Online]. Available: http://www.ema.europa.eu/docs/en GB/document library/Scientific guideline/201 4/12/WC500179071.pdf [Accessed 31st October 2017].

GAZELIUS, B. 2018. Iontophoresis - theory. Periflux Systems, 1-8.

GHOSH, K., SHU, X. Z., MOU, R., LOMBARDI, J., PRESTWICH, G. D., RAFAILOVICH, M. H. & CLARK, R. A. F. 2005. Rheological Characterization of in Situ Cross-Linkable Hyaluronan Hydrogels. Biomacromol, 6, 2857-2865.

GHOSH, P., BROGDEN, N. K. & STINCHCOMB, A. L. 2013. Effect of formulation pH on transport of naltrexone species and pore closure in microneedle-enhanced transdermal drug delivery. Mol Pharm, 10, 2331-2339.

GLAXOSMITHKLINE. 2014. Voltarol Gel Patch 140 mg medicated plaster [Online]. Available: https://www.voltarol.co.uk/content/dam/cf-consumer-healthcare/voltaren/en GB/PDF%20Leaflets/VOLTAROL%20140MG%20PLASTE R%200010%20GB 924294Z PIL.PDF [Accessed 17 May 2018].

GRUNENTHAL. 2017. Ralvo® 700 mg medicated plaster [Online]. Available: https://www.medicines.org.uk/emc/files/pil.2469.pdf [Accessed 17 May 2018].

HAMMELL, D. C., HAMAD, M., VADDI, H. K., CROOKS, P. A. & STINCHCOMB, A. L. 2004. A duplex "Gemini" prodrug of naltrexone for transdermal delivery. J Control Release, 97, 283-290.

HO, K. Y. & DODOU, K. 2007. Rheological studies on pressure-sensitve silicone adhesives and drug-in-adhesive layers as a means to characterise adhesive performance. Int J Pharm, 333, 24-33.

JOKE RENO TEC 2017. Film sealing device - Fermant 22N/40N/60N/80N/120N.

KOCH, T., PASSMAN, F. & RABENSTEIN, A. 2015. Comparative study of microbiological monitoring of water-miscible metalworking fluids. Int Biodeterior Biodegradation, 98, 19-25.

KOTHARI, K., RAGOONANAN, V. & SURYANARAYANAN, R. 2014. The Role of Drug-Polymer Hydrogen Bonding Interactions on the Molecular Mobility and Physical Stability of Nifedipine Solid Dispersions. Mol Pharm, 12, 162-170.

LAMBERS, H., PIESSENS, S., BLOEM, A., PRONK, H. & FINKEL, P. 2006. Natural skin surface pH is on average below 5, which is beneficial for its resident flora. Int J Cosmet Sci, 28, 359-370.

LAMBERT, J. B. 1987. Introduction to Organic Spectroscopy, Macmillan.

LESSMANN, H., SCHNUCH, A., GEIER, J. & UTER, W. 2005. Skin-sensitizing and irritant properties of propyleneglycol. Contact Dermatitis, 53, 247-259.

LI, J. L. & MOONEY, D. J. 2016. Designing hydrogels for controlled drug delivery. Nat Rev Mater, 1, 1-38.

LIRA, L. M., MARTINS, K. A. & CORDOBA DE TORRESI, S. I. 2009. Structural parameters of polyacrylamide hydrogels obtained by the Equilibrium Swelling Theory. Eur Polym J, 45, 1232-1238.

LOFTY, S. & DODOU, K. 2014. The use of hot stage microscopy for the study of ibuprofen crystallisation in acrylic and silicone adhesives. APS PharmSci: The Science of Medicines. University of Hertfordshire, Hatfield.

MCCANDLESS, J. 2006. Low-Dose Naltrexone (LDN) for Mood Regulation and Immunomodulation in ASD [Online]. Available: https://www.lowdosenaltrexone.org/ conf2006/J McCandless2.pdf [Accessed 12 December 2017].

MCLAUGHLIN, P. J., CAIN, J. D., TITUNICK, M. B., SASSINI, J. W. & ZAGON, I. S. 2017. Topical Naltrexone Is a Safe and Effective Alternative to Standard Treatment of Diabetic Wounds. Adv Wound Care, 6, 279-288.

MEDICINES AND HEALTHCARE REGULATORY AGENCY. 2019. Nurofen Joint & Muscular Pain Relief 200 mg Medicated Plaster Ibuprofen [Online]. Available: http://www.mhra.gov.uk/home/groups/spcpil/documents/spcpil/con1531718788499 .pdf.

METZ, T. O., ZHANG, Q., PAGE, J. S., SHEN, Y., CALLISTER, S. J., JACOBS, J. M. & SMITH, R. D. 2007. The future of liquid chromatography-mass spectrometry (LC-MS) in metabolic profiling and metabolomic studies for biomarker discovery. Biomark Med, 1, 159-185.

MILEWSKI, M., PINNINTI, R. R. & STINCHCOMB, A. L. 2012. Naltrexone salt selection for enhanced transdermal permeation through microneedle-treated skin. J Pharm Sci, 101, 2777-2786.

MILEWSKI, M., YERRAMREDDY, T. R., GHOSH, P., CROOKS, P. A. & STINCHCOMB, A. L. 2010. In vitro permeation of a pegylated naltrexone prodrug across microneedle-treated skin. J Control Release, 146, 37-44.

MONTI, K. M., FOLTZ, R. L. & CHINN, D. M. 1991. Analysis of Naltrexone and 6-β-Naltrexol in Plasma and Urine by Gas Chromatography/Negative Ion Chemical Ionization Mass Spectrometry. J Anal Toxicol, 15, 136-140.

NALLURI, B. N., MILLIGAN, C., CHEN, J., CROOKS, P. A. & STINCHCOMB, A. L. 2005. In Vitro Release Studies on Matrix Type Transdermal Drug Delivery Systems of Naltrexone and Its Acetyl Prodrug. Drug Dev Ind Pharm, 31, 871-877.

OMIDIAN, H., HASHEMI, S.-A., ASKARI, F. & NAFIAI, S. 1994. Swelling and Crosslink Density Measurements for Hydrogels. Iran J Polym Sci Technol, 3, 115-119.

ORION. 2018. Sandrena 0.5 mg gel & 1 mg gel [Online]. Available: https://www.medicines.org.uk/emc/files/pil.2219.pdf [Accessed 16 Apr 2019].

PADMAVATHI, N. C. & CHATTERJI, P. R. 1996. Structural Characteristics and Swelling Behavior of Poly(ethylene glycol) Diacrylate Hydrogels. Macromolecules, 29, 1976-1979.

PANCHAGNULA, R., SALVE, P. S., THOMAS, N. S., JAIN, A. K. & RAMARAO, P. 2001. 5Transdermal delivery of naloxone: effect of water, propyleneglycol, ethanol and their binary combinations on permeation through rat skin. Int J Pharm, 219, 95-105.

PARHI, R. & PADILAM, S. 2018. In vitro permeation and stability studies on developed drug-in-adhesive transdermal patch of simvastatin. B-FOPCU, 56, 26-33.

PARK, J.-S., PARK, J.-W. & RUCKENSTEIN, E. 2001. Thermal and dynamic mechanical analysis of PVA/MC blend hydrogels. Polymer, 42, 4271-4280.

PATEL, R. P., PATEL, G. & BARIA, A. 2009. Formulation and evaluation of transdermal patch of Aceclofenac. Int J Drug Deliv, 1, 41-51.

PAUDEL, K. S., NALLURI, B. N., HAMMELL, D. C., VALIVETI, S., KIPTOO, P., HAMAD, M. O., CROOKS, P. A. & STINCHCOMB, A. L. 2005. Transdermal Delivery of Naltrexone and its Active Metabolite 6-B-naltrexol in Human Skin In Vitro and Guinea Pigs In Vivo. J Pharm Sci, 94, 1965-1975.

PEPPAS, N. A. & MEADOWS, D. L. 1983. Macromolecular Structure and Solute Diffusion in Membranes: An Overview of Recent Theories. J Memb Sci, 16, 361-377.

PILLAI, P., HAMAD, M. O., CROOKS, P. A. & STINCHCOMB, A. L. 2004. Physicochemical Evaluation, in Vitro Human Skin Diffusion, and Concurrent Biotransformation of 3-O-Alkyl Carbonate Prodrugs of Naltrexone. Pharm Res, 21, 1146-1152.

RAMMENSEE, S., HUEMMERICH, D., HERMANSON, K. D., SCHEIBEL, T. & BAUSCH, A. R. 2005. Rheological characterization of hydrogels formed by recombinantly produced spider silk. Appl Phys A, 1-4.

RASK, M. B., KNOPP, M. M., OLESEN, N. E., HOLM, R. & RADES, T. 2018. Comparison of two DSC-based methods to predict drug-polymer solubility. Int J Pharm, 540, 98-105.

RAWAT, S., VENGURLEKAR, S., RAKESH, B., JAIN, S. & SRIKARTI, G. 2008. Transdermal Delivery by Iontophoresis. Indian J Pharm Sci, 70, 5-10.

REDISCH, W., SHECKMAN, E. & STEELE, J. M. 1952. Skin Temperature Response of Normal Human Subjects to Various Conditions. Circulation, 6, 862-867.

ROY, N., SAHA, N., KITANO, T. & SAHA, P. 2009. Development and characterization of novel medicated hydrogels for wound dressing. Soft Mater, 8, 130-148.

SABRI, A. H., OGILVIE, J., ABDULHAMIS, K., SHPADARUK, V., MCKENNA, J., DEGAL, J., SCURR, D. J. & MARLOW, M. 2019. Expanding the applications of microneedles in dermatology. Eur J Pharm Biopharm, 140, 121-140.

SCHMID-WENDTNER, M.-H. & KORTING, H. C. 2006. The pH of the Skin Surface and Its Impact on the Barrier Function. Skin Pharmacol Physiol, 19, 296-302.

SCHULKE. 2019. Mikrocount Duo [Online]. Available: https://www.schuelke.com/intl-en/products/mikrocount-duo.php [Accessed 12 Feb 2019].

SERRANO-CASTANEDA, P., ESCOBAR-CHAVEZ, J. J., RODRIGUEQ-CRUZ, I. M., MELGOZA-CONTRERAS, L. M. & MARTINEZ-HERNANDEZ, J. 2018. Microneedles as Enhancer of Drug Absorption Through the Skin and Applications in Medicine and Cosmetology J Pharm Sci, 21, 73-93.

SHARMA, S., PARVEZ, N. & SHARMA, P. K. 2015. Iontophoresis-Models and Applications: A Review. AJBAS, 7, 1-7.

SIGMA ALDRICH. 2019. Propylene glycol [Online]. Available: https://www.sigmaaldrich.com/catalog/substance/propyleneglycol76095755611?la ng=en&region=GB [Accessed 12 July 2019].

SLAWSON, M. H., CHEN, M., MOODY, D. E., COMER, S. D., NUWAYSER, E. S., FANG, W. B. & FOLTZ, R. L. 2007. Quantitative Analysis of Naltrexone and 6B-Naltrexol in Human, Rat, and Rabbit Plasma by Liquid Chromatography-Electrospray Ionization Tandem Mass Spectrometry with Application to the Pharmacokinetics of Depotrex in Rabbits. J Anal Toxicol, 31, 453-461.

STELESCU, M. D., AIRINEI, A., MANAILA, E., CRACIUN, G., FIFERE, N., VARGANICI, C., PAMFIL, D. & DOROFTEI, F. 2018. Effects of Electron Beam Irradiation on the Mechanical, Thermal, and Surface Properties of Some EPDM/Butyl Rubber Composites. Polymers, 10, 1-21.

SUKSAEREE, J., SIRIPORNPINYO, P. & CHAIPRASIT, S. 2017. Formulation, Characterization, and In Vitro Evaluation of Transdermal Patches for Inhibiting Crystallization of Mefenamic Acid. Journal of Drug Delivery, 2017, 1-7.

THAKUR, G., SINGH, A. & SINGH, I. 2016. Formulation and evaluation of transdermal composite films of chitosan-montmorillonite for the delivery of curcumin. Int J Pharm Investig, 6, 23-31.

TOENNES, S. W., KAUERT, G. F., GRUSSER, S. M., JAKEL, W. & PARTECKE, G. 2004. Determination of naltrexone and 6-beta-naltrexol in human plasma following implantation of naltrexone pellets using gas chromatography-mass spectrometry. J Pharm Biomed Anal, 35, 169-176.

VADDI, H. K., BANKS, S. L., CHEN, J., HAMMELL, D. C., CROOKS, P. A. & STINCHCOMB, A. L. 2009. Human Skin Permeation of 3-O-Alkyl Carbamate Prodrugs of Naltrexone. J Pharm Sci, 98, 2611-2625.

VANDERHOOFT, J. L., ALCOUTLABI, M., MAGDA, J. J. & PRESTWICH, G. D. 2009. Rheological Properties of Cross-Linked Hyaluronan-Gelatin Hydrogels for Tissue Engineering. Macromol Biosci, 9, 20-28.

WARSHAW, E. M., BOTTO, N. C., MAIBACH, H. I., FOWLER, J. F., RIETSCHEL, R. L., ZUG, K. A., BELSITO, D. V., TAYLOR, J. S., DELEO, V. A., PRATT, M. D., SASSEVILLE, D., STORRS, F. J., MARKS, J. G. & MATHIAS, C. G. 2009. Positive patch-test reactions to propylene glycol: a retrospective cross-sectional analysis from the North American Contact Dermatitis Group, 1996 to 2006. Dermatitis, 20, 14-20.

WILLIAMS, A. C. & BARRY, B. W. 2004. Penetration enhancers. Ad Drug Deliv Rev, 56, 603-618.

WONG, R. S. H., ASHTON, M. & DODOU, K. 2015. Effect of Crosslinking Agent Concentration on the Properties of Unmedicated Hydrogels. Pharmaceutics, 7, 305-319.

WONG, R. S. H. & DODOU, K. 2017. Effect of Drug Loading Method and Drug Physicochemical Properties on the Material and Drug Release Properties of Poly (Ethylene Oxide) Hydrogels for Transdermal Delivery. Polymers, 9, 1-29.

WONG, R. S. H., ASHTON, M. & DODOU, K. 2016. Analysis of residual crosslinking agent content in UV cross-linked poly(ethylene oxide) hydrogels for dermatological application by gas chromatography. J. Pharm. Anal., 6, 307-312.

XIA, Z., PATCHAN, M., MARANCHI, J., ELISSEEFF, J. & TREXLER, M. 2013. Determination of crosslinking density of hydrogels prepared from microcrystalline cellulose. J Appl Polym Sci, 127, 4537-4541.

YACOB, N. & HASHIM, K. 2014. Morphological effect on swelling behaviour of hydrogel. AIP Conference Proceedings, 1584, 153-159.

YAN, C. & POCHAN, D. J. 2011. Rheological properties of peptide-based hydrogels for biomedical and other applications. Chem Soc Rev, 39, 3528-3540.

YUN, H. Y., BANDG, S. C., LEE, K. C., BAEK, I. H., LEE, S. P., KANG, W. & KWON, K. I. 2007. Simultaneous analysis of naltrexone and its major metabolite, 6-beta-naltrexol, in human plasma using liquid chromatography-tandem mass spectrometry: Application to a parent-metabolite kinetic model in humans. Talanta, 71, 1553-1559.

ZHANG, Y., NG, W., HU, J., MUSSA, S. S., GE, Y. & XU, H. 2018. Formulation and in vitro stability evaluation of ethosomal carbomer hydrogel for transdermal vaccine delivery. Colloids Surf B Biointerfaces, 163, 184-191.

ZUSTIAK, S. P. & LEACH, J. B. 2011. Hydrolytically degradable poly(ethylene glycol) hydrogel scaffolds with tunable degradation and mechanical properties. Biomacromolecules, 11, 1348-1357.

## Claims

1. A transdermal patch comprising:

   a cross-linked poly(ethylene oxide) hydrogel; and
   an occlusive adhesive tape;

   wherein the hydrogel further comprises naltrexone, or a pharmaceutically acceptable salt or solvate thereof, and wherein the poly(ethylene oxide) hydrogel has a crosslink density of at least $4.5 \times 10^{-4}$ mol cm$^{-3}$ and not more than $16 \times 10^{-4}$ mol cm$^{-3}$.

2. The transdermal patch of claim 1, wherein the crosslink density is at least $5 \times 10^{-4}$ mol cm$^{-3}$; and/or wherein the crosslink density is not more than $10 \times 10^{-4}$ mol cm$^{-3}$, optionally not more than $7 \times 10^{-4}$ mol cm$^{-3}$.

3. The transdermal patch of any preceding claim, wherein the hydrogel comprises propylene glycol in an amount of about 13% to about 23% w/w of the hydrogel, optionally about 15% to about 20% w/w of the hydrogel, further optionally about 17% to about 19% w/w of the hydrogel.

4. The transdermal patch of any preceding claim, wherein the crosslinked poly(ethylene oxide) is formed by reacting poly(ethylene oxide) with a cross-linking agent.
   optionally wherein the ratio of poly(ethylene oxide) to cross-linking agent in the hydrogel film is from about 15:1 to 25:1 w/w, further optionally wherein the ratio of poly(ethylene oxide) to cross-linking agent in the hydrogel film is about 20:1 w/w.

5. The transdermal patch of claim 4, wherein the crosslinking agent is an acrylate monomer, optionally wherein the cross-linking agent is selected from: pentaerythritol tetraacrylate, pentaerythritol triacrylate, ethylene glycol diacrylate, di(ethylene glycol) diacrylate, tetra(ethylene glycol) diacrylate, ethylene glycol dimethacrylate, di(ethylene glycol) dimethacrylate, and tri(ethylene glycol) dimethacrylate,

> optionally wherein the crosslinking agent is pentaerythritol tetraacrylate; and/or
> wherein the poly(ethylene oxide) has a viscosity average molecular weight (Mv) of at least about 600,000 g/mol and not more than about 8,000,000 g/mol;
> optionally wherein the poly(ethylene oxide) has a viscosity average molecular weight (Mv) of at least about 800,000 g/mol and not more than about 5,000,000 g/mol;
> further optionally wherein the poly(ethylene oxide) has a viscosity average molecular weight (Mv) of at least about 900,000 g/mol and not more than about 1,500,000 g/mol.

6. The transdermal patch of any preceding claim, wherein the naltrexone, or pharmaceutically acceptable salt or solvate thereof, is present in an amount of at least about 3% w/w of the hydrogel,

> optionally wherein the naltrexone, or pharmaceutically acceptable salt or solvate thereof, is present in an amount of at least about 7% w/w of the hydrogel; and/or
> optionally wherein either

> > (i) the naltrexone is in the form of naltrexone base, further optionally wherein the naltrexone base is present in an amount of from about 7% to about 15% w/w of the hydrogel, optionally wherein the naltrexone base is present in an amount of from about 7% to about 12% w/w of the hydrogel; or
> > (ii) wherein the naltrexone is in the form of naltrexone salt, optionally wherein the salt is naltrexone hydrochloride, further optionally wherein the naltrexone salt is present in an amount of from about 7% to about 30% w/w of the hydrogel, optionally wherein the naltrexone salt is present in an amount of from about 7% to about 12% w/w of the hydrogel.

7. The transdermal patch of any preceding claim, wherein the hydrogel film has a tensile strength of at least about 0.50 MPa; and/or
wherein the occlusive adhesive tape is impermeable.

8. The transdermal patch of any preceding claim, wherein the patch further comprises a permeable membrane, such that the cross-linked poly(ethylene oxide) hydrogel is sandwiched between the occlusive adhesive tape and the permeable membrane, optionally wherein the permeable membrane is an ethylene vinyl acetate membrane.

9. The transdermal patch of any preceding claim, wherein the patch further comprises a release liner,

> optionally wherein the release liner is coated with fluoropolymer; and/or
> optionally wherein the transdermal patch is packaged in a sealed pouch.

10. A method for preparing a cross-linked poly(ethylene oxide) hydrogel, the method comprising:

> (a) mixing an aqueous solution comprising poly(ethylene oxide) and naltrexone or a pharmaceutically acceptable salt or solvate thereof;
> (b) adding a cross-linking agent to the mixture;
> (c) drying the mixture to form a xerogel;
> (d) irradiating the xerogel under UV radiation to create cross-linking; and
> (e) swelling of the irradiated crosslinked xerogel to provide the cross-linked hydrogel.

11. The method of claim 10, further comprising one or more of features (i) to (viii):

> (i) wherein step (e) is performed in aqueous solution comprising about 5% to about 30% v/v propylene glycol, optionally wherein said aqueous solution comprises about 15 to about 25% v/v propylene glycol;
> (ii) wherein step (d) is performed for between at least about 10 minutes and 20 minutes, optionally wherein step d) is performed for between at least about 13 minutes and 16 minutes;
> (iii) wherein the ratio of poly(ethylene oxide) to cross-linking agent in step (b) is from about 15:1 w/w to about 25:1 w/w;
> (iv) wherein the cross-linking agent of step (b) is an acrylate monomer, optionally wherein the cross-linking agent of step (b) is selected from: pentaerythritol tetraacrylate, pentaerythritol triacrylate, ethylene glycol diacrylate, di(ethylene glycol) diacrylate, tetra(ethylene glycol) diacrylate, ethylene glycol dimethacrylate, di(ethylene glycol) dimethacrylate, and tri(ethylene glycol) dimethacrylate;

(v) wherein the cross-linking agent of step (b) is pentaerythritol tetraacrylate;

(vi) wherein the poly(ethylene oxide) has a viscosity average molecular weight (Mv) of at least about 600,000 g/mol and not more than about 8,000,000 g/mol; optionally wherein the poly(ethylene oxide) has a viscosity average molecular weight (Mv) of at least about 800,000 g/mol and not more than about 5,000,000 g/mol; further optionally wherein the poly(ethylene oxide) has a viscosity average molecular weight (Mv) of at least about 900,000 g/mol and not more than about 1,500,000 g/mol;

(vii) wherein the drying is performed at a temperature of not more than 40 °C, optionally between 20 °C and 30 °C; and

(viii) wherein the method further comprises forming a transdermal patch by:

(f) affixing the cross-linked hydrogel to an occlusive adhesive tape, or sandwiching the cross-linked hydrogel between an occlusive adhesive tape and a permeable membrane; optionally further comprising attaching a release liner to an adhesive surface of the adhesive tape; optionally wherein the method further comprises:
(g) sealing the transdermal patch in a pouch.

12. The method of claim 10 or claim 11, wherein the naltrexone is naltrexone base or naltrexone hydrochloride, optionally wherein the naltrexone is naltrexone base, further optionally wherein the aqueous solution of step (a) further comprises ethanol.

13. A transdermal patch obtainable or obtained by the method of any of claims 10 to 12.

14. The transdermal patch of any of claims 1 to 9 or claim 13 for use as a medicament.

15. A transdermal patch of any of claims 1 to 9 or claim 13 for use in the treatment of a condition selected from: opioid dependency, alcohol dependency, Crohn's disease/ulcerative colitis, chronic fatigue syndrome/myalgic encephalomyelitis, autism, pruritus, diabetic wounds, HIV/AIDS, fibromyalgia, multiple sclerosis, inflammatory bowel disease, Crohn's disease/ulcerative colitis, complex regional pain syndrome, Hailey-Hailey disease, psoriasis, Ehlers-Danlos syndrome, cancer, Gulf War illness, depression, chronic arthritis, autoimmune disorders, and respiratory inflammatory disorders.

**Patentansprüche**

1. Transdermales Pflaster, umfassend:

ein vernetztes Poly(ethylenoxid)Hydrogel; und
ein okklusives Klebeband;
wobei das Hydrogel ferner Naltrexon oder ein pharmazeutisch verträgliches Salz oder Solvat davon umfasst und wobei das Poly(ethylenoxid)Hydrogel eine Vernetzungsdichte von zumindest $4{,}5 \times 10^{-4}$ mol cm$^{-3}$ und nicht mehr als $16 \times 10^{-4}$ mol cm$^{-3}$ aufweist.

2. Transdermales Pflaster nach Anspruch 1, wobei die Vernetzungsdichte zumindest $5 \times 10^{-4}$ mol cm$^{-3}$ ist; und/oder wobei die Vernetzungsdichte nicht mehr als $10 \times 10^{-4}$ mol cm$^{-3}$, optional nicht mehr als $7 \times 10^{-4}$ mol cm$^{-3}$ ist.

3. Transdermales Pflaster nach einem vorhergehenden Anspruch, wobei das Hydrogel Propylenglykol in einer Menge von etwa 13 Gew.-% bis etwa 23 Gew.-% des Hydrogels, optional etwa 15 Gew.-% bis etwa 20 Gew.-% des Hydrogels, ferner optional etwa 17 Gew.-% bis etwa 19 Gew.-% des Hydrogels umfasst.

4. Transdermales Pflaster nach einem vorhergehenden Anspruch, wobei das vernetzte Poly(ethylenoxid) gebildet wird, indem Poly(ethylenoxid) mit einem Vernetzungsmittel reagiert wird, wobei optional das Verhältnis von Poly(ethylenoxid) zu Vernetzungsmittel in dem Hydrogelfilm etwa 15:1 bis 25:1 Gew./Gew. ist, wobei ferner optional das Verhältnis von Poly(ethylenoxid) zu Vernetzungsmittel in dem Hydrogelfilm etwa 20:1 Gew./Gew. ist.

5. Transdermales Pflaster nach Anspruch 4, wobei das Vernetzungsmittel ein Acrylatmonomer ist, wobei optional das Vernetzungsmittel ausgewählt ist aus: Pentaerythritoltetraacrylat, Pentaerythritoltriacrylat, Ethylenglykoldiacrylat, Di(ethylenglykol)diacrylat, Tetra(ethylenglykol)diacrylat, Ethylenglykoldimethacrylat, Di(ethylenglykol)dimethacrylat und Tri(ethylenglykol)dimethacrylat,

wobei optional das Vernetzungsmittel Pentaerythritoltetraacrylat ist; und/oder
wobei das Poly(ethylenoxid) ein viskositätsmittleres Molekulargewicht (Mv) von zumindest etwa 600.000 g/mol und nicht mehr als etwa 8.000.000 g/mol aufweist;
wobei optional das Poly(ethylenoxid) ein viskositätsmittleres Molekulargewicht (Mv) von zumindest etwa 800.000 g/mol und nicht mehr als etwa 5.000.000 g/mol aufweist;
wobei ferner optional das Poly(ethylenoxid) ein viskositätsmittleres Molekulargewicht (Mv) von zumindest etwa 900.000 g/mol und nicht mehr als etwa 1.500.000 g/mol aufweist.

6. Transdermales Pflaster nach einem vorhergehenden Anspruch, wobei das Naltrexon oder das pharmazeutisch verträgliche Salz oder Solvat davon in einer Menge von zumindest etwa 3 Gew.-% des Hydrogels vorhanden ist,

wobei optional das Naltrexon oder das pharmazeutisch verträgliche Salz oder Solvat davon in einer Menge von zumindest etwa 7 Gew.-% des Hydrogels vorhanden ist; und/oder
wobei optional entweder

(i) das Naltrexon in der Form von Naltrexonbase ist, wobei ferner optional die Naltrexonbase in einer Menge von etwa 7 Gew.-% bis etwa 15 Gew.-% des Hydrogels vorhanden ist, wobei optional die Naltrexonbase in einer Menge von etwa 7 Gew.-% bis etwa 12 Gew.-% des Hydrogels vorhanden ist; oder
(ii) wobei das Naltrexon in der Form von Naltrexonsalz ist, wobei optional das Salz Naltrexonhydrochlorid ist, wobei ferner optional das Naltrexonsalz in einer Menge von etwa 7 Gew.-% bis etwa 30 Gew.-% des Hydrogels vorhanden ist, wobei optional das Naltrexonsalz in einer Menge von etwa 7 Gew.-% bis etwa 12 Gew.-% des Hydrogels vorhanden ist.

7. Transdermales Pflaster nach einem vorhergehenden Anspruch, wobei der Hydrogelfilm eine Zugfestigkeit von zumindest etwa 0,50 MPa aufweist; und/oder
wobei das okklusive Klebeband undurchlässig ist.

8. Transdermales Pflaster nach einem vorhergehenden Anspruch, wobei das Pflaster ferner eine durchlässige Membran umfasst, sodass das vernetzte Poly(ethylenoxid)Hydrogel zwischen dem okklusiven Klebeband und der durchlässigen Membran eingefügt ist,
wobei optional die durchlässige Membran eine Ethylenvinylacetatmembran ist.

9. Transdermales Pflaster nach einem vorhergehenden Anspruch, wobei das Pflaster ferner eine Abziehfolie umfasst,

wobei optional die Abziehfolie mit Fluorpolymer beschichtet ist; und/oder
wobei optional das transdermale Pflaster in einem versiegelten Beutel verpackt ist.

10. Verfahren zur Herstellung eines vernetzten Poly(ethylenoxid)Hydrogels, wobei das Verfahren Folgendes umfasst:

(a) Mischen einer wässrigen Lösung, die Poly(ethylenoxid) und Naltrexon oder ein pharmazeutisch verträgliches Salz oder Solvat davon umfasst;
(b) Hinzufügen eines Vernetzungsmittels zu der Mischung;
(c) Trocknen der Mischung, um ein Xerogel zu bilden;
(d) Bestrahlen des Xerogels unter UV-Strahlung, um Vernetzung zu erzeugen; und
(e) Quellen des bestrahlten vernetzten Xerogels, um das vernetzte Hydrogel bereitzustellen.

11. Verfahren nach Anspruch 10, ferner umfassend eines oder mehrere der Merkmale (i) bis (viii):

(i) wobei Schritt (c) in einer wässrigen Lösung durchgeführt wird, die etwa 5 Vol.-% bis etwa 30 Vol.-% Propylenglykol umfasst, wobei optional die wässrige Lösung etwa 15 bis etwa 25 Vol.-% Propylenglykol umfasst;
(ii) wobei Schritt (d) zwischen zumindest etwa 10 Minuten und 20 Minuten durchgeführt wird, wobei optional Schritt d) zwischen zumindest etwa 13 Minuten und 16 Minuten durchgeführt wird;
(iii) wobei das Verhältnis von Poly(ethylenoxid) zu Vernetzungsmittel in Schritt (b) etwa 15:1 Gew./Gew. bis etwa 25:1 Gew./Gew. ist;
(iv) wobei das Vernetzungsmittel von Schritt (b) ein Acrylatmonomer ist, wobei optional das Vernetzungsmittel von Schritt (b) ausgewählt ist aus: Pentaerythritoltetraacrylat, Pentaerythritoltriacrylat, Ethylenglykoldiacrylat, Di(ethylenglykol)diacrylat, Tetra(ethylenglykol)diacrylat, Ethylenglykoldimethacrylat, Di(ethylenglykol)dimethacrylat und Tri(ethylenglykol)dimethacrylat;

(v) wobei das Vernetzungsmittel von Schritt (b) Pentaerythritoltetraacrylat ist;

(vi) wobei das Poly(ethylenoxid) ein viskositätsmittleres Molekulargewicht (Mv) von zumindest etwa 600.000 g/mol und nicht mehr als etwa 8.000.000 g/mol aufweist; wobei optional das Poly(ethylenoxid) ein viskositätsmittleres Molekulargewicht (Mv) von zumindest etwa 800.000 g/mol und nicht mehr als etwa 5.000.000 g/mol aufweist; wobei ferner optional das Poly(ethylenoxid) ein viskositätsmittleres Molekulargewicht (Mv) von zumindest etwa 900.000 g/mol und nicht mehr als etwa 1.500.000 g/mol aufweist;

(vii) wobei das Trocknen bei einer Temperatur von nicht mehr als 40 °C, optional zwischen 20 °C und 30 °C durchgeführt wird; und

(viii) wobei das Verfahren ferner Bilden eines transdermalen Pflasters durch Folgendes umfasst:

(f) Befestigen des vernetzten Hydrogels an einem okklusiven Klebeband oder Einfügen des vernetzten Hydrogels zwischen einem okklusiven Klebeband und einer durchlässigen Membran; optional ferner umfassend Anbringen einer Abziehfolie an einer Klebefläche des Klebebands; wobei optional das Verfahren ferner Folgendes umfasst:

(g) Versiegeln des transdermalen Pflasters in einem Beutel.

12. Verfahren nach Anspruch 10 oder Anspruch 11, wobei das Naltrexon Naltrexonbase oder Naltrexonhydrochlorid ist, wobei optional das Naltrexon Naltrexonbase ist, wobei ferner optional die wässrige Lösung von Schritt (a) ferner Ethanol umfasst.

13. Transdermales Pflaster, erhältlich oder erhalten durch das Verfahren nach einem der Ansprüche 10 bis 12.

14. Transdermales Pflaster nach einem der Ansprüche 1 bis 9 oder Anspruch 13 zur Verwendung als Medikament.

15. Transdermales Pflaster nach einem der Ansprüche 1 bis 9 oder Anspruch 13 zur Verwendung bei der Behandlung eines Zustands ausgewählt aus: Opioidabhängigkeit, Alkoholabhängigkeit, Morbus Crohn/Colitis ulcerosa, chronischem Müdigkeitssyndrom/myalgischer Enzephalomyelitis, Autismus, Pruritus, diabetischen Wunden, HIV/AIDS, Fibromyalgie, multipler Sklerose, entzündlicher Darmerkrankung, Morbus Crohn/Colitis ulcerosa, komplexem regionalen Schmerzsyndrom, Hailey-Hailey-Krankheit, Psoriasis, Ehlers-Danlos-Syndrom, Krebs, Golfkriegskrankheit, Depression, chronischer Arthritis, Autoimmunstörungen und entzündlichen Störungen der Atemwege.

**Revendications**

1. Timbre transdermique comprenant :

un hydrogel de poly(oxyde d'éthylène) réticulé ; et
un ruban adhésif occlusif ;
ledit hydrogel comprenant en outre de la naltrexone, ou un sel ou solvate pharmaceutiquement acceptable de celle-ci, et ledit hydrogel de poly(oxyde d'éthylène) présentant une densité de réticulation d'au moins $4,5 \times 10^{-4}$ mol cm$^{-3}$ et de pas plus de $16 \times 10^{-4}$ mol cm$^{-3}$.

2. Timbre transdermique selon la revendication 1, ladite densité de réticulation étant d'au moins $5 \times 10^{-4}$ mol cm$^{-3}$ ; et/ou ladite densité de réticulation étant de pas de plus de $10 \times 10^{-4}$ mol cm$^{-3}$, éventuellement de pas plus de $7 \times 10^{-4}$ mol cm$^{-3}$.

3. Timbre transdermique selon l'une quelconque des revendications précédentes, ledit hydrogel comprenant du propylène glycol en une quantité d'environ 13 % à environ 23 % en p/p de l'hydrogel, éventuellement d'environ 15 % à environ 20 % en p/p de l'hydrogel, en outre éventuellement d'environ 17 % à environ 19 % en p/p de l'hydrogel.

4. Timbre transdermique selon l'une quelconque des revendications précédentes, ledit poly(oxyde d'éthylène) réticulé étant formé en faisant réagir du poly(oxyde d'éthylène) avec un agent de réticulation, éventuellement, ledit rapport du poly(oxyde d'éthylène) à l'agent de réticulation dans le film d'hydrogel étant d'environ 15:1 à 25:1 en p/p, en outre éventuellement, ledit rapport du poly(oxyde d'éthylène) à l'agent de réticulation dans le film d'hydrogel étant d'environ 20:1 en p/p.

**5.** Timbre transdermique selon la revendication 4, ledit agent de réticulation étant un monomère d'acrylate, éventuellement ledit agent de réticulation étant choisi parmi : le tétraacrylate de pentaérythritol, le triacrylate de pentaérythritol, le diacrylate d'éthylène glycol, le diacrylate de di(éthylène glycol), le diacrylate de tétra(éthylène glycol), le diméthacrylate d'éthylène glycol, le diméthacrylate de di(éthylène glycol) et le diméthacrylate de tri(éthylène glycol),

éventuellement, ledit agent de réticulation étant le tétraacrylate de pentaérythritol ; et/ou
ledit poly(oxyde d'éthylène) présentant un poids moléculaire moyen en viscosité (Mv) d'au moins environ 600 000 g/mol et de pas plus d'environ 8 000 000 g/mol ;
éventuellement, ledit poly(oxyde d'éthylène) présentant un poids moléculaire moyen en viscosité (Mv) d'au moins environ 800 000 g/mol et de pas plus d'environ 5 000 000 g/mol ;
en outre, éventuellement, ledit poly(oxyde d'éthylène) présentant un poids moléculaire moyen en viscosité (Mv) d'au moins environ 900 000 g/mol et de pas plus d'environ 1 500 000 g/mol.

**6.** Timbre transdermique selon l'une quelconque des revendications précédentes, ladite naltrexone, ou un sel ou solvate pharmaceutiquement acceptable de celle-ci, étant présent en une quantité d'au moins environ 3 % en p/p de l'hydrogel,

éventuellement, ladite naltrexone, ou un sel ou solvate pharmaceutiquement acceptable de celle-ci, étant présent en une quantité d'au moins environ 7 % en p/p de l'hydrogel ; et/ou
éventuellement soit

(i) ladite naltrexone se présentant sous la forme d'une base de naltrexone, en outre éventuellement, ladite base de naltrexone étant présente en une quantité d'environ 7 % à environ 15 % en p/p de l'hydrogel, éventuellement, ladite base de naltrexone étant présente en une quantité d'environ 7 % à environ 12 % en p/p de l'hydrogel ; soit
(ii) ladite naltrexone se présentant sous la forme d'un sel de naltrexone, éventuellement ledit sel étant le chlorhydrate de naltrexone, en outre éventuellement ledit sel de naltrexone étant présent en une quantité d'environ 7 % à environ 30 % en p/p de l'hydrogel, éventuellement ledit sel de naltrexone étant présent en une quantité d'environ 7 % à environ 12 % en p/p de l'hydrogel.

**7.** Timbre transdermique selon l'une quelconque des revendications précédentes, ledit film d'hydrogel présentant une résistance à la traction d'au moins environ 0,50 MPa ; et/ou
ledit ruban adhésif occlusif étant imperméable.

**8.** Timbre transdermique selon l'une quelconque des revendications précédentes, ledit timbre comprenant en outre une membrane perméable, de sorte que l'hydrogel de poly(oxyde d'éthylène) réticulé soit pris en sandwich entre le ruban adhésif occlusif et la membrane perméable,
éventuellement ladite membrane perméable étant une membrane d'acétate de vinyle d'éthylène.

**9.** Timbre transdermique selon l'une quelconque des revendications précédentes, ledit timbre comprenant en outre un revêtement anti-adhésif,
éventuellement, ledit revêtement antiadhésif étant recouvert d'un fluoropolymère ; et/ou éventuellement, ledit timbre transdermique étant emballé dans un sachet scellé.

**10.** Procédé de préparation d'un hydrogel de poly(oxyde d'éthylène) réticulé, le procédé comprenant :

(a) le mélange d'une solution aqueuse comprenant du poly(oxyde d'éthylène) et de la naltrexone ou un sel ou solvate pharmaceutiquement acceptable de celle-ci ;
(b) l'ajout d'un agent de réticulation au mélange ;
(c) le séchage du mélange pour former un xérogel ;
(d) l'irradiation du xérogel sous un rayonnement UV pour créer une réticulation ; et
(e) le gonflement du xérogel réticulé irradié pour fournir l'hydrogel réticulé.

**11.** Procédé selon la revendication 10, comprenant en outre une ou plusieurs des caractéristiques (i) à (viii) :

(i) ladite étape (c) étant réalisée dans une solution aqueuse comprenant environ 5 % à environ 30 % en v/v de propylène glycol, éventuellement ladite solution aqueuse comprenant environ 15 à environ 25 % en v/v de propylène glycol ;

(ii) ladite étape (d) étant réalisée pendant entre au moins environ 10 minutes et 20 minutes, éventuellement ladite étape d) étant réalisée pendant entre au moins environ 13 minutes et 16 minutes ;

(iii) ledit rapport du poly(oxyde d'éthylène) à l'agent de réticulation à l'étape (b) étant d'environ 15:1 en p/p à environ 25:1 en p/p ;

(iv) ledit agent de réticulation de l'étape (b) étant un monomère d'acrylate, éventuellement ledit agent de réticulation de l'étape (b) étant choisi parmi : le tétraacrylate de pentaérythritol, le triacrylate de pentaérythritol, le diacrylate d'éthylène glycol, le diacrylate de di(éthylène glycol), le diacrylate de tétra(éthylène glycol), le diméthacrylate d'éthylène glycol, le diméthacrylate de di(éthylène glycol) et le diméthacrylate de tri(éthylène glycol) ;

(v) ledit agent de réticulation de l'étape (b) étant le tétraacrylate de pentaérythritol ;

(vi) ledit poly(oxyde d'éthylène) présentant un poids moléculaire moyen en viscosité (Mv) d'au moins environ 600 000 g/mol et de pas plus d'environ 8 000 000 g/mol ; éventuellement, ledit poly(oxyde d'éthylène) présentant un poids moléculaire moyen en viscosité (Mv) d'au moins environ 800 000 g/mol et de pas plus d'environ 5 000 000 g/mol ; en outre, éventuellement, ledit poly(oxyde d'éthylène) présentant un poids moléculaire moyen en viscosité (Mv) d'au moins environ 900 000 g/mol et de pas plus d'environ 1 500 000 g/mol ;

(vii) ledit séchage étant effectué à une température de pas plus de 40°C, éventuellement comprise entre 20°C et 30°C ; et

(viii) ledit procédé comprenant en outre la formation d'un timbre transdermique par :

(f) apposition de l'hydrogel réticulé sur un ruban adhésif occlusif, ou prise en sandwich de l'hydrogel réticulé entre un ruban adhésif occlusif et une membrane perméable ;
comprenant éventuellement en outre la fixation d'un revêtement anti-adhésif sur une surface adhésive du ruban adhésif ; éventuellement, ledit procédé comprenant en outre :
(g) le scellage du timbre transdermique dans un sachet.

12. Procédé selon la revendication 10 ou la revendication 11, ladite naltrexone étant la base de naltrexone ou le chlorhydrate de naltrexone,
éventuellement, ladite naltrexone étant une base de naltrexone, et éventuellement, ladite solution aqueuse de l'étape (a) comprenant en outre de l'éthanol.

13. Timbre transdermique pouvant être obtenu ou obtenu par le procédé selon l'une quelconque des revendications 10 à 12.

14. Timbre transdermique selon l'une quelconque des revendications 1 à 9 ou la revendication 13, destiné à être utilisé comme médicament.

15. Timbre transdermique selon l'une quelconque des revendications 1 à 9 ou la revendication 13, destiné à être utilisé dans le traitement d'un état choisi parmi : la dépendance aux opioïdes, la dépendance à l'alcool, la maladie de Crohn/la colite ulcéreuse, le syndrome de fatigue chronique/l'encéphalomyélite myalgique, l'autisme, un prurit, les plaies diabétiques, le VIH/SIDA, la fibromyalgie, la sclérose en plaques, les maladies intestinales inflammatoires, la maladie de Crohn/la colite ulcéreuse, le syndrome douloureux régional complexe, la maladie de Hailey-Hailey, le psoriasis, le syndrome d'Ehlers-Danlos, le cancer, la syndrome de la guerre du Golfe, la dépression, l'arthrite chronique, les maladies auto-immunes et les troubles respiratoires inflammatoires.

**Figure 1**

A)

B)

**Figure 2**

Figure 3

Figure 4

**Figure 5**

**Figure 6**

Figure 7

Figure 8

Figure 9

a

b

c

d

Figure 10

a

b

c

d

Figure 11

EP 4 051 247 B1

a

b

c

d

Figure 12

a

b

c

d

**Figure 13**

Figure 14

Figure 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TOLJAN ; VROOMAN.** Low-Dose Naltrexone (LDN) - Review of Therapeutic Utilization. *Med. Sci.,* 2018, vol. 6, 82
- **BERGE et al.** Pharmaceutical Salts. *Journal of Pharmaceutical Science,* 1977, vol. 66, 1-19
- **PETRA IN WONG et al.** *Journal of Pharmaceutical Analysis,* 2016, vol. 6 (5), 307-312
- **ABD, E ; YOUSEF, S. A. ; PASTORE, M. N. ; TELAPROLU, K. ; MOHAMMED, Y. H. ; NAMJOSHI, S. ; GRICE, J. E. ; ROBERTS, M. S.** Skin models for the testing of transdermal drugs. *Clin Pharmacol,* 2016, vol. 8, 163-176
- **AKHLAQ, M ; ARSHAD, M. S. ; MUDDASSIR, A. M. ; HUSSAIN, A.** Formulation and evaluation of anti-rheumatic dexibuprofen transdermal patches: a quality-by-design approach. *J Drug Target,* 2015, vol. 24, 1-27
- **ALKILANI, A. Z. ; MCCRUDDEN, M. T. C. ; DONNELLY, R. F.** Transdermal Drug Delivery: Innovative Pharmaceutical Developments Based on Disruption of the Barrier Properties of the stratum corneum. *Pharmaceutics,* 2015, vol. 7, 438-470
- **ALVAREZ-FUENTES ; J., CARABALLO, I. ; BOZA, A. ; LLERA, J. M. ; HOLGADO, M. A. ; FERNANDEZ-AREVALO, M.** Study of a complexation process between naltrexone and Eudragit® L as an oral controlled release system. *Int J Pharm,* 1997, vol. 148, 219-230
- **ENDE, M. T. ; PEPPAS, N. A.** Transport of ionizable drugs and proteins in crosslinked poly(acrylic acid) and poly(acrylic acid-co-2-hydroxyethyl methacrylate) hydrogels. II. Diffusion and release studies. *J Control Release,* 1997, vol. 48, 47-56
- **ANSETH, K. S. ; BOWMAN, C. N. ; BRANNON-PEPPAS, L.** Mechanical properties of hydrogels and their experimental determination. *Biomaterials,* 1996, vol. 17, 1647-1657
- **BANERJEE, S. ; CHATTOPADHYAY, P. ; GHOSH, A. ; BHATTACHARYA, S. S. ; KUNDU, A. ; VEER, V.** Accelerated stability testing of a transdermal patch composed of eserine and pralidoxime chloride for prophylaxis against (±)-anatoxin A poisoning. *J Food Drug Anal,* 2014, vol. 22, 264-270
- **BANKS, S. L. ; PINNINTI, R. R. ; GILLB, H. S ; CROOKS, P. A. ; M.R., P. ; STINCHCOMB, A. L.** Flux across microneedle-treated skin is increased by increasing charge of naltrexone and naltrexol in vitro. *Pharm Res,* 2008, vol. 25, 1677-1685
- **BANSAL, S. S. ; KAUSHAL, A. M. ; BANSAL, A. K.** Co-relationship of physical stability of amorphous dispesions with enthalpy relaxation. *Pharmazie,* 2008, vol. 63, 812-814
- **BARIYA, S. H. ; GOHEL, M. C ; MEHTA, T. A. ; SHARMA, O. P.** Microneedles: an emerging transdermal drug delivery system. *J Pharm Pharmacol,* 2011, vol. 64, 11-29
- **BIGLIARDI, P. L. ; STAMMER, H. ; JOST, G. ; RUFLI, T. ; BUCHNER, S. ; BIGLIARDI-QI, M.** Treatment of pruritus with topically applied opiate receptor antagonist. *J Am Acad Dermatol,* 2007, vol. 56, 979-988
- **BUTSCHLI, J.** Bemis Wins FPA Award for Lidocare Pouch Development. *Healthcare Packaging.,* 2017
- **CAMPBELL, V. ; MCGRATH, C. ; CORRY, A.** Low-dose naltrexone: a novel treatment for Hailey-Hailey disease. *Br J Dermatol,* 2017, vol. 178, 1196-1198
- **CAMPISI, G. ; GIANNOLA, L. I. ; FLORENA, A. M. ; DE CARO, V. ; SCHUMACHER, A. ; GOTTSCHE, T. ; PADERNI, C. ; WOLFF, A.** Bioavailability in vivo of naltrexone following transbuccal administration by an electronically controlled intraoral device: A trial on pigs. *J Cont Rel,* 2010, vol. 145, 214-220
- **CAYKARA, T. ; INAM, R.** Determination of average molecular weight between crosslinks and polymer-solvent interaction parameters of poly(acrylamide-g-ethylene diamine tetraacetic acid) polyelectrolyte hydrogels. *J Appl Polym Sci,* 2004, vol. 91, 2168-2175
- **CHENEVAS-PAULE, C. ; WOLFF, H.-M. ; ASHTON, M. ; SCHUBERT, M. ; DODOU, K.** Development of a Predictive Model for the Long-Term Stability Assessment of Drug-In-Adhesive Transdermal Films Using Polar Pressure-Sensitive Adhesives as Carrier/Matrix. *J Pharm Sci,* 2017, vol. 106, 1293-1301
- **CLEMENTS, R.** *Modern Chemical Discoveries,* 1963, 22
- **CORDERY, S. F. ; HUSBANDS, S. M. ; BAILEY, C. P. ; GUY, R. H. ; DELGADO-CHARRO, M. B.** Simultaneous Transdermal Delivery of Buprenorphine Hydrochloride and Naltrexone Hydrochloride by Iontophoresis. *Mol Pharm,* 2019, vol. 16, 2808-2816

- **DEY, S ; MALGOPE, A.** Preparation of carvedilol transdermal patch and the effect of propylene glycol on permeation. *Int J Pharm Pharm Sci,* 2010, vol. 2, 137-143
- **DODOU, K ; ARMSTRONG, A. ; KELLY, I. ; WILKINSON, S. ; CARR, K. ; SHATTOCK, P. ; WHITELEY, P.** Ex vivo studied for the passive transdermal delivery of low-dose naltrexone from a cream; detection of naltrexone and its active metabolite, 6β-naltrexol, using a novel LC Q-ToF MS assay. *Pharm Dev Technol,* 2015, vol. 20, 694-701
- **DODOU, K. ; SADDIQUE, W.** Effect of manufacturing method on the in vitro drug release and adhesive performance of drug-in-adhesive films containing binary mixtures of ibuprofen with polozamer 188. *Pharm Dev Technol,* 2012, vol. 17, 552-561
- **DOYTCHEVA, M. ; DOTCHEVA, D. ; STAMENOVA, R. ; TSVETANOV, C.** UV-initiated crosslinking of poly(ethylene oxide) with pentaerythritol triacrylate in solid state. *Macromol Mater Eng,* 2001, vol. 286, 30-33
- Guideline on quality of transdermal patches. *Guideline on quality of transdermal patches,* 2014, http://www.ema.europa.eu/docs/en GB/document library/Scientific guideline/201 4/12/WC500179071.pdf
- **GAZELIUS, B.** Iontophoresis - theory. *Periflux Systems,* 2018, vol. 1-8
- **GHOSH, K. ; SHU, X. Z. ; MOU, R. ; LOMBARDI, J. ; PRESTWICH, G. D. ; RAFAILOVICH, M. H. ; CLARK, R. A. F.** Rheological Characterization of in Situ Cross-Linkable Hyaluronan Hydrogels. *Biomacromol,* 2005, vol. 6, 2857-2865
- **GHOSH, P. ; BROGDEN, N. K. ; STINCHCOMB, A. L.** Effect of formulation pH on transport of naltrexone species and pore closure in microneedle-enhanced transdermal drug delivery. *Mol Pharm,* 2013, vol. 10, 2331-2339
- **GLAXOSMITHKLINE.** *Voltarol Gel Patch 140 mg medicated plaster,* 2014, https://www.voltarol.co.uk/content/dam/cf-consumer-healthcare/voltaren/en GB/PDF%20Leaflets/VOLTAROL%20140MG%20PLASTE R%200010%20GB 924294Z PIL.PDF
- **GRUNENTHAL.** *Ralvo® 700 mg medicated plaster,* 2017, https://www.medicines.org.uk/emc/files/pil.2469.pdf
- **HAMMELL, D. C. ; HAMAD, M. ; VADDI, H. K. ; CROOKS, P. A. ; STINCHCOMB, A. L.** A duplex "Gemini" prodrug of naltrexone for transdermal delivery. *J Control Release,* 2004, vol. 97, 283-290
- **HO, K. Y. ; DODOU, K.** Rheological studies on pressure-sensitve silicone adhesives and drug-in-adhesive layers as a means to characterise adhesive performance. *Int J Pharm,* 2007, vol. 333, 24-33
- *Film sealing device - Fermant 22N/40N/60N/80N/120N,* 2017
- **KOCH, T. ; PASSMAN, F. ; RABENSTEIN, A.** Comparative study of microbiological monitoring of water-miscible metalworking fluids. *Int Biodeterior Biodegradation,* 2015, vol. 98, 19-25
- **KOTHARI, K. ; RAGOONANAN, V. ; SURYANARAYANAN, R.** The Role of Drug-Polymer Hydrogen Bonding Interactions on the Molecular Mobility and Physical Stability of Nifedipine Solid Dispersions. *Mol Pharm,* 2014, vol. 12, 162-170
- **LAMBERS, H. ; PIESSENS, S. ; BLOEM, A. ; PRONK, H. ; FINKEL, P.** Natural skin surface pH is on average below 5, which is beneficial for its resident flora. *Int J Cosmet Sci,* 2006, vol. 28, 359-370
- **LAMBERT, J. B.** *Introduction to Organic Spectroscopy,* 1987
- **LESSMANN, H. ; SCHNUCH, A. ; GEIER, J. ; UTER, W.** Skin-sensitizing and irritant properties of propyleneglycol. *Contact Dermatitis,* 2005, vol. 53, 247-259
- **LI, J. L. ; MOONEY, D. J.** Designing hydrogels for controlled drug delivery. *Nat Rev Mater,* 2016, vol. 1, 1-38
- **LIRA, L. M. ; MARTINS, K. A. ; CORDOBA DE TORRESI, S. I.** Structural parameters of polyacrylamide hydrogels obtained by the Equilibrium Swelling Theory. *Eur Polym J,* 2009, vol. 45, 1232-1238
- The use of hot stage microscopy for the study of ibuprofen crystallisation in acrylic and silicone adhesives. **LOFTY, S. ; DODOU, K.** APS PharmSci: The Science of Medicines. University of Hertfordshire, 2014
- **MCCANDLESS, J.** *Low-Dose Naltrexone (LDN) for Mood Regulation and Immunomodulation in ASD,* 2006, https://www.lowdosenaltrexone.org/conf2006/J McCandless2.pdf
- **MCLAUGHLIN, P. J. ; CAIN, J. D. ; TITUNICK, M. B ; SASSINI, J. W. ; ZAGON, I. S.** Topical Naltrexone Is a Safe and Effective Alternative to Standard Treatment of Diabetic Wounds. *Adv Wound Care,* 2017, vol. 6, 279-288
- **MEDICINES AND HEALTHCARE REGULATORY AGENCY.** *Nurofen Joint & Muscular Pain Relief 200 mg Medicated Plaster Ibuprofen,* 2019, http://www.mhra.gov.uk/home/groups/spcpil/documents/spcpil/con1531718788499 .pdf.
- **METZ, T. O. ; ZHANG, Q. ; PAGE, J. S. ; SHEN, Y. ; CALLISTER, S. J. ; JACOBS, J. M. ; SMITH, R. D.** The future of liquid chromatography-mass spectrometry (LC-MS) in metabolic profiling and metabolomic studies for biomarker discovery. *Biomark Med,* 2007, vol. 1, 159-185
- **MILEWSKI, M. ; PINNINTI, R. R. ; STINCHCOMB, A. L.** Naltrexone salt selection for enhanced transdermal permeation through microneedle-treated skin. *J Pharm Sci,* 2012, vol. 101, 2777-2786

- **MILEWSKI, M. ; YERRAMREDDY, T. R ; GHOSH, P. ; CROOKS, P. A. ; STINCHCOMB, A. L.** vitro permeation of a pegylated naltrexone prodrug across microneedle-treated skin. *J Control Release,* 2010, vol. 146, 37-44
- **MONTI, K. M. ; FOLTZ, R. L. ; CHINN, D. M.** Analysis of Naltrexone and 6-β-Naltrexol in Plasma and Urine by Gas Chromatography/Negative Ion Chemical Ionization Mass Spectrometry. *J Anal Toxicol,* 1991, vol. 15, 136-140
- **NALLURI, B. N. ; MILLIGAN, C. ; CHEN, J. ; CROOKS, P. A. ; STINCHCOMB, A. L.** Vitro Release Studies on Matrix Type Transdermal Drug Delivery Systems of Naltrexone and Its Acetyl Prodrug. *Drug Dev Ind Pharm,* 2005, vol. 31, 871-877
- **OMIDIAN, H ; HASHEMI, S.-A. ; ASKARI, F. ; NAFIAI, S.** Swelling and Crosslink Density Measurements for Hydrogels. *Iran J Polym Sci Technol,* 1994, vol. 3, 115-119
- **ORION.** *Sandrena 0.5 mg gel & 1 mg gel,* 2018, https://www.medicines.org.uk/emc/files/pil.2219.pdf
- **PADMAVATHI, N. C. ; CHATTERJI, P. R.** Structural Characteristics and Swelling Behavior of Poly(ethylene glycol) Diacrylate Hydrogels. *Macromolecules,* 1996, vol. 29, 1976-1979
- **PANCHAGNULA, R. ; SALVE, P. S. ; THOMAS, N. S. ; JAIN, A. K. ; RAMARAO, P.** 5Transdermal delivery of naloxone: effect of water, propyleneglycol, ethanol and their binary combinations on permeation through rat skin. *Int J Pharm,* 2001, vol. 219, 95-105
- **PARHI, R. ; PADILAM, S.** In vitro permeation and stability studies on developed drug-in-adhesive transdermal patch of simvastatin. *B-FOPCU,* 2018, vol. 56, 26-33
- **PARK, J.-S. ; PARK, J.-W. ; RUCKENSTEIN, E.** Thermal and dynamic mechanical analysis of PVA/MC blend hydrogels. *Polymer,* 2001, vol. 42, 4271-4280
- **PATEL, R. P. ; PATEL, G. ; BARIA, A.** Formulation and evaluation of transdermal patch of Aceclofenac. *Int J Drug Deliv,* 2009, vol. 1, 41-51
- **PAUDEL, K. S. ; NALLURI, B. N. ; HAMMELL, D. C. ; VALIVETI, S. ; KIPTOO, P. ; HAMAD, M. O. ; CROOKS, P. A. ; STINCHCOMB, A. L.** Transdermal Delivery of Naltrexone and its Active Metabolite 6-B-naltrexol in Human Skin In Vitro and Guinea Pigs In Vivo. *J Pharm Sci,* 2005, vol. 94, 1965-1975
- **PEPPAS, N. A. ; MEADOWS, D. L.** Macromolecular Structure and Solute Diffusion in Membranes: An Overview of Recent Theories. *J Memb Sci,* 1983, vol. 16, 361-377
- **PILLAI, P. ; HAMAD, M. O ; CROOKS, P. A. ; STINCHCOMB, A. L.** Physicochemical Evaluation, in Vitro Human Skin Diffusion, and Concurrent Biotransformation of 3-O-Alkyl Carbonate Prodrugs of Naltrexone. *Pharm Res,* 2004, vol. 21, 1146-1152
- **RAMMENSEE, S ; HUEMMERICH, D. ; HERMANSON, K. D. ; SCHEIBEL, T. ; BAUSCH, A. R.** Rheological characterization of hydrogels formed by recombinantly produced spider silk. *Appl Phys A,* 2005, (1-4
- **RASK, M. B. ; KNOPP, M. M. ; OLESEN, N. E. ; HOLM, R. ; RADES, T.** Comparison of two DSC-based methods to predict drug-polymer solubility. *Int J Pharm,* 2018, vol. 540, 98-105
- **RAWAT, S. ; VENGURLEKAR, S. ; RAKESH, B. ; JAIN, S. ; SRIKARTI, G.** Transdermal Delivery by Iontophoresis. *Indian J Pharm Sci,* 2008, vol. 70, 5-10
- **REDISCH, W. ; SHECKMAN, E. ; STEELE, J. M.** Skin Temperature Response of Normal Human Subjects to Various Conditions. *Circulation,* 1952, vol. 6, 862-867
- **ROY, N ; SAHA, N. ; KITANO, T. ; SAHA, P.** Development and characterization of novel medicated hydrogels for wound dressing. *Soft Mater,* 2009, vol. 8, 130-148
- **SABRI, A. H. ; OGILVIE, J. ; ABDULHAMIS, K. ; SHPADARUK, V ; MCKENNA, J. ; DEGAL, J. ; SCURR, D. J. ; MARLOW, M.** Expanding the applications of microneedles in dermatology. *Eur J Pharm Biopharm,* 2019, vol. 140, 121-140
- **SCHMID-WENDTNER, M.-H. ; KORTING, H. C.** The pH of the Skin Surface and Its Impact on the Barrier Function. *Skin Pharmacol Physiol,* 2006, vol. 19, 296-302
- **SCHULKE.** *Mikrocount Duo,* 12 February 2019, https://www.schuelke.com/intl-en/products/mikrocount-duo.php
- **SERRANO-CASTANEDA, P. ; ESCOBAR-CHAVEZ, J. J. ; RODRIGUEQ-CRUZ, I. M. ; MELGOZA-CONTRERAS, L. M. ; MARTINEZ-HERNANDEZ, J.** Microneedles as Enhancer of Drug Absorption Through the Skin and Applications in Medicine and Cosmetology. *J Pharm Sci,* 2018, vol. 21, 73-93
- **SHARMA, S. ; PARVEZ, N. ; SHARMA, P. K.** Iontophoresis-Models and Applications: A Review. *AJBAS,* 2015, vol. 7, 1-7
- **SIGMA ALDRICH.** *Propylene glycol,* 12 July 2019, https://www.sigmaaldrich.com/catalog/substance/propyleneglycol76095755611?la ng=en&region=GB

- **SLAWSON, M. H. ; CHEN, M ; MOODY, D. E ; COMER, S. D. ; NUWAYSER, E. S. ; FANG, W. B. ; FOLTZ, R. L.** Quantitative Analysis of Naltrexone and 6B-Naltrexol in Human, Rat, and Rabbit Plasma by Liquid Chromatography-Electrospray Ionization Tandem Mass Spectrometry with Application to the Pharmacokinetics of Depotrex in Rabbits. *J Anal Toxicol,* 2007, vol. 31, 453-461
- **STELESCU, M. D ; AIRINEI, A ; MANAILA, E. ; CRACIUN, G ; FIFERE, N. ; VARGANICI, C. ; PAMFIL, D. ; DOROFTEI, F.** Effects of Electron Beam Irradiation on the Mechanical, Thermal, and Surface Properties of Some EPDM/Butyl Rubber Composites. *Polymers,* 2018, vol. 10, 1-21
- **SUKSAEREE, J ; SIRIPORNPINYO, P. ; CHAIPRASIT, S.** Formulation, Characterization, and In Vitro Evaluation of Transdermal Patches for Inhibiting Crystallization of Mefenamic Acid. *Journal of Drug Delivery,* 2017, 1-7
- **THAKUR, G. ; SINGH, A. ; SINGH, I.** Formulation and evaluation of transdermal composite films of chitosan-montmorillonite for the delivery of curcumin. *Int J Pharm Investig,* 2016, vol. 6, 23-31
- **TOENNES, S. W ; KAUERT, G. F. ; GRUSSER, S. M. ; JAKEL, W. ; PARTECKE, G.** Determination of naltrexone and 6-beta-naltrexol in human plasma following implantation of naltrexone pellets using gas chromatography-mass spectrometry. *J Pharm Biomed Anal,* 2004, vol. 35, 169-176
- **VADDI, H. K ; BANKS, S. L. ; CHEN, J ; HAMMELL, D. C. ; CROOKS, P. A. ; STINCHCOMB, A. L.** Human Skin Permeation of 3-O-Alkyl Carbamate Prodrugs of Naltrexone. *J Pharm Sci,* 2009, vol. 98, 2611-2625
- **VANDERHOOFT, J. L. ; ALCOUTLABI, M. ; MAGDA, J. J. ; PRESTWICH, G. D.** Rheological Properties of Cross-Linked Hyaluronan-Gelatin Hydrogels for Tissue Engineering. *Macromol Biosci,* 2009, vol. 9, 20-28
- **WARSHAW, E. M. ; BOTTO, N. C. ; MAIBACH, H. I. ; FOWLER, J. F. ; RIETSCHEL, R. L. ; ZUG, K. A. ; BELSITO, D. V. ; TAYLOR, J. S. ; DELEO, V. A. ; PRATT, M. D.** Positive patch-test reactions to propylene glycol: a retrospective cross-sectional analysis from the North American Contact Dermatitis Group. *Dermatitis,* 1996, vol. 20, 14-20
- **WILLIAMS, A. C. ; BARRY, B. W.** Penetration enhancers. *Ad Drug Deliv Rev,* 2004, vol. 56, 603-618
- **WONG, R. S. H. ; ASHTON, M. ; DODOU, K.** Effect of Crosslinking Agent Concentration on the Properties of Unmedicated Hydrogels. *Pharmaceutics,* 2015, vol. 7, 305-319
- **WONG, R. S. H. ; DODOU, K.** Effect of Drug Loading Method and Drug Physicochemical Properties on the Material and Drug Release Properties of Poly (Ethylene Oxide) Hydrogels for Transdermal Delivery. *Polymers,* 2017, vol. 9, 1-29
- **WONG, R. S. H. ; ASHTON, M. ; DODOU, K.** Analysis of residual crosslinking agent content in UV cross-linked poly(ethylene oxide) hydrogels for dermatological application by gas chromatography. *J. Pharm. Anal.,* 2016, vol. 6, 307-312
- **XIA, Z. ; PATCHAN, M. ; MARANCHI, J. ; ELISSEEFF, J. ; TREXLER, M.** Determination of crosslinking density of hydrogels prepared from microcrystalline cellulose. *J Appl Polym Sci,* 2013, vol. 127, 4537-4541
- **YACOB, N. ; HASHIM, K.** Morphological effect on swelling behaviour of hydrogel. *AIP Conference Proceedings,* 2014, vol. 1584, 153-159
- **YAN, C. ; POCHAN, D. J.** Rheological properties of peptide-based hydrogels for biomedical and other applications. *Chem Soc Rev,* 2011, vol. 39, 3528-3540
- **YUN, H. Y. ; BANDG, S. C. ; LEE, K. C. ; BAEK, I. H ; LEE, S. P. ; KANG, W. ; KWON, K. I.** Simultaneous analysis of naltrexone and its major metabolite, 6-beta-naltrexol, in human plasma using liquid chromatography-tandem mass spectrometry: Application to a parent-metabolite kinetic model in humans. *Talanta,* 2007, vol. 71, 1553-1559
- **ZHANG, Y. ; NG, W. ; HU, J. ; MUSSA, S. S. ; GE, Y. ; XU, H.** Formulation and in vitro stability evaluation of ethosomal carbomer hydrogel for transdermal vaccine delivery. *Colloids Surf B Biointerfaces,* 2018, vol. 163, 184-191
- **ZUSTIAK, S. P. ; LEACH, J. B.** Hydrolytically degradable poly(ethylene glycol) hydrogel scaffolds with tunable degradation and mechanical properties. *Biomacromolecules,* 2011, vol. 11, 1348-1357